# EUROPÄISCHE PATENTANMELDUNG

(11) **EP 2 092 824 A1**
(43) Veröffentlichungstag der Anmeldung: **26.08.2009**
(21) Anmeldenummer: 08151901.9
(22) Anmeldetag: 25.02.2008
(51) Int. Cl.: A01N 43/56, C07D 403/04, A01N 43/54

(54) **Heterocyclyl-Pyrimidine**

(71) Anmelder: Bayer CropScience AG, 40789 Monheim (DE)
(72) Erfinder: Müller, Michael, 40595 Düsseldorf (DE); Schwarz, Hans-Georg, 40764 Langenfeld (DE); Wiese, Welf Burkhard, 40764 Langenfeld (DE); Wroblowsky, Heinz-Juergen, 40764 Langenfeld (DE); Arnold, Christian, 40764 Langenfeld (DE); Dahmen, Peter, 41470 Neuss (DE); Franken, Eva-Maria, 69009 Lyon (FR); Malsam, Olga, 51503 Rösrath (DE); Voerste, Arnd, 50674 Köln (DE)

(57) **Zusammenfassung**

Die vorliegende Erfindung betrifft Heterocyclyl-Pyrimidine der nachfolgenden Formel (I) 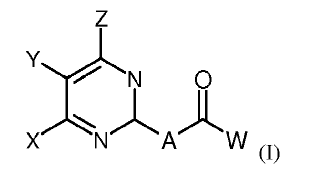 ein Verfahren zur deren Herstellung, die Verwendung der erfindungsgemäßen Heterocyclyl-Pyrimidine zum Bekämpfen von tierischen Schädlingen und/oder pflanzenpathogenen Schadpilzen, Verfahren und Mittel zur Bekämpfung von tierischen Schädlingen und/oder pflanzenpathogenen Schadpilzen in und/oder auf Pflanzen oder in und/oder auf Saatgut von Pflanzen, Verfahren zur Herstellung solcher Mittel und behandeltes Saatgut sowie deren Verwendung zur Bekämpfung von Schädlingen und/oder pflanzenpathogenen Schadpilzen in der Land-, Garten- und Forstwirtschaft, in der Tiergesundheit, im Materialschutz sowie im Bereich Haushalt und Hygiene.

## Beschreibung

Die vorliegende Erfindung betrifft Heterocyclyl-Pyrimidine der nachfolgenden Formel (I) 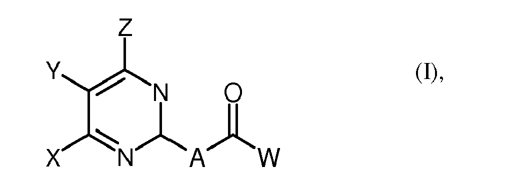 worin die Symbole die in der Beschreibung angegebenen Bedeutungen haben, oder deren agrochemisch wirksame Salze oder Mischungen dieser Verbindungen und/oder deren agrochemisch wirksamen Salzen mit anderen Wirkstoffen zur Bekämpfung von tierischen Schädlingen und/oder pflanzenpathogenen Schadpilzen, Verfahren und Mittel zur Bekämpfung von tierischen Schädlingen und/oder pflanzenpathogenen Schadpilzen in und/oder auf Pflanzen oder in und/oder auf Saatgut von Pflanzen, Verfahren zur Herstellung solcher Mittel und behandeltes Saatgut sowie deren Verwendung zur Bekämpfung von Schädlingen und/oder pflanzenpathogenen Schadpilzen in der Land-, Garten- und Forstwirtschaft, in der Tiergesundheit, im Materialschutz sowie im Bereich Haushalt und Hygiene. Die vorliegende Erfindung betrifft weiterhin ein Verfahren zur Herstellung von Heterocyclyl-Pyrimidinen der Formel (I).

Die vorliegende Erfindung betrifft weiterhin die Steigerung der Wirkung von Pflanzenschutzmitteln enthaltend insbesondere Heterocyclyl-Pyrimidine der Formel (I), durch die Zugabe von Ammonium- oder Phosphoniumsalzen und gegebenenfalls Penetrationsförderern, die entsprechenden Mittel, Verfahren zu ihrer Herstellung und ihre Anwendung im Pflanzenschutz als Insektizide und/oder Akarizide und/oder Fungizide.

Es ist bereits bekannt, dass bestimmte 2-Heterocyclyl-Pyrimidin-Derivate fungizide Eigenschaften besitzen, siehe z.B. WO 02/074753, WO 06/029867, WO 07/036477 und WO 08/015250. Weiterhin ist bereits bekannt, dass bestimmte 2-Heterocyclyl-Pyrimidin-Derivate nematizide Eigenschaften besitzen, siehe z.B. WO 06/032526.

Da sich aber die ökologischen und ökonomischen Anforderungen an moderne Schädlingsbekämpfungsmittel laufend erhöhen, beispielsweise was Wirkspektrum, Toxizität, Selektivität, Aufwandmenge, Rückstandbildung und günstige Herstellbarkeit angeht, und außerdem z.B. Probleme mit Resistenzen auftreten können, besteht die ständige Aufgabe, neue Schädlingsbekämpfungsmittel zu entwickeln, die zumindest in Teilbereichen Vorteile gegenüber den bekannten aufweisen.

Die erfindungsgemäßen Verbindungen, nämlich die Heterocyclyl-Pyrimidine, sind durch die Formel (I) allgemein definiert.

Die vorliegende Erfindung betrifft somit
(1) chemische Verbindungen, nämlich Heterocyclyl-Pyrimidine der nachfolgenden Formel (I) 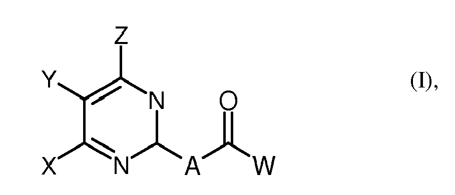 worin
   - X: für Wasserstoff, Halogen, Cyano, gegebenenfalls substituiertes Alkyl, gegebenenfalls substituiertes Phenyl steht;
   - Z: für jeweils gegebenenfalls substituiertes Alkoxy, Alkylthio, Alkylsulfonyl oder die 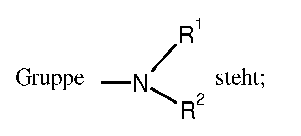 Y für gegebenenfalls substituiertes Aryl, gegebenenfalls substituiertes Heterocyclyl, gegebenenfalls substituiertes Alkyl, gegebenenfalls substituiertes Alkenyl, gegebenenfalls substituiertes Alkinyl, gegebenenfalls substituiertes Cycloalkyl, gegebenenfalls substituiertes Cycloalkenyl, gegebenenfalls substituiertes Arylalkyl, Halogen, eine gegebenenfalls substituierte Aminogruppe, gegebenenfalls substituiertes (C₁-C₈)-Alkoxy, gegebenenfalls substituiertes (C₁-C₄)-Alkoxy-(C₁-C₈)-alkoxy, gegebenenfalls substituiertes (C₁-C₄)-Dialkylamino-(C₁-C₈)-alkoxy, gegebenenfalls substituiertes (C₁-C₈)-Alkylthio, gegebenenfalls substituiertes (C₆-C₁₀)-Aryloxy, gegebenenfalls substituiertes (C₆-C₁₀)-Arylthio, gegebenenfalls substituiertes Heterocyclyloxy, gegebenenfalls substituiertes (C₆-C₁₀)-Aryl-(C₁-C₄)-alkoxy, gegebenenfalls substituiertes (C₆-C₁₀)-Aryl-(C₁-C₄)-alkylthio, gegebenenfalls substituiertes Heterocyclyl-(C₁-C₄)-alkoxy, gegebenenfalls substituiertes Heterocyclyl-(C₁-C₄)-alkylthio steht;
   A für einen drei- bis zehngliedrigen gesättigten, partiell ungesättigten oder aromatischen mono- oder bicyclischen Heterocyclus steht, enthaltend ein bis vier Heteroatome aus der Gruppe O, N oder S, wobei A durch eine bis drei gleiche oder verschiedene Gruppen R^{d} substituiert sein kann, und
   R^{d} für Halogen, Hydroxy, Cyano, Oxo, Nitro, Amino, Mercapto, C₁-C₆-Alkyl, C₁-C₆-Halogenalkyl, C₂-C₆-Alkenyl, C₂-C₆-Alkinyl, C₃-C₆-Cycloalkyl, C₁-C₆-Alkoxy, C₁-C₆- Halogenalkoxy, C₁-C₇-Alkylcarbonylamino, C₁-C₆-Alkylamino, Di-(C₁-C₆-alkyl)amino, C₁-C₆-Alkylthio, C₁-C₆-Alkylsulfinyl, C₁-C₆-Alkylsulfonyl, Aminosulfonyl, C₁-C₆-Alkylaminosulfonyl, und/oder Di-(C₁-C₆-alkyl)aminosulfonyl steht;
   - W: steht für 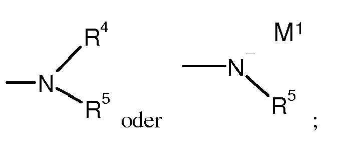
   - R¹: für Wasserstoff, Alkyl, Alkenyl, Alkinyl, Cycloalkyl, Halogenalkyl oder Heterocyclyl, Hydroxy, Alkoxy, Amin, Alkylamin, Dialkylamin, Hydroxyalkyl, Alkylcarbonyloxyalkyl oder Alkyloxycarbonyloxyalkyl steht;
   - R²: für Wasserstoff oder Alkyl steht;
   oder
   - R¹ und R²: gemeinsam mit dem Stickstoffatom, an das sie gebunden sind, für einen gegebenenfalls substituierten heterocyclischen Ring stehen;
   - R⁴: steht für Wasserstoff, jeweils gegebenenfalls substituiertes Alkyl, Alkenyl, Alkinyl und Cycloalkyl, jeweils gegebenenfalls substituiertes gesättigtes oder ungesättigtes Heterocyclyl, jeweils gegebenenfalls substituiertes Phenyl, Heteroaryl, Arylalkyl, Heteroarylalkyl;
   - M¹: steht für ein Kation;
   - R⁵: steht für die Gruppen COR⁷, S(O)₁₋₂R⁷, Cyano, COOR⁷, 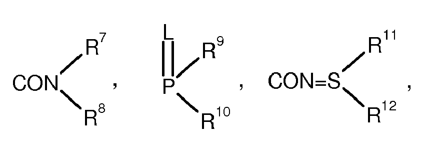 gesättigtes, teilweise oder vollständig ungesättigtes oder aromatisches, gegebenenfalls substituiertes 5- oder 6-Ring Heterocyclyl, das gegebenenfalls ein oder bis zu drei weitere Heteroatome, ausgewählt aus der Gruppe bestehend aus N, S und/oder O-Atomen enthält,
   wobei Sauerstoffatome nicht benachbart sein dürfen,
   - L: steht für Sauerstoff oder Schwefel,
   - R⁷: steht für Wasserstoff, Alkyl, Alkenyl, Alkinyl, Halogenalkyl, Cycloalkyl-alkyl, Cycloalkyl oder Cycloalkenyl, Aryl, Heteroaryl, Arylalkyl oder Heteroarylalkyl,
   - R⁸: steht für Wasserstoff, jeweils gegebenenfalls substituiertes Alkyl, Alkenyl, Alkinyl, Alkoxy oder NH-R⁴,
   - R⁷ und R⁸: stehen gemeinsam mit dem Stickstoffatom, an das sie gebunden sind, für einen gegebenenfalls substituierten gesättigten oder ungesättigten Ring, der gegebenenfalls durch weitere Heteroatome unterbrochen sein kann;
   - R⁹ und R¹⁰: stehen unabhängig voneinander für jeweils gegebenenfalls substituiertes Alkyl, Alkenyl, Alkoxy, Alkenyloxy, Alkinyloxy, Cycloalkoxy, Cycloalkenyloxy, Cycloalkylalkoxy, Alkylthio, Alkenylthio, Phenoxy, Phenylthio, Benzyloxy, Benzylthio, Heteroaryloxy, Heteroarylthio, Heteroarylalkoxy oder Heteroarylalkylthio;
   oder
   - R⁹ und R¹⁰: stehen gemeinsam mit dem Phosphoratom, an das sie gebunden sind, für einen gegebenenfalls substituierten 5- bis 7-gliedrigen Cyclus, der durch ein oder zwei Sauerstoff- und/oder Schwefelatome unterbrochen sein kann; und
   - R¹¹ und R¹²: stehen unabhängig voneinander für jeweils gegebenenfalls substituiertes Alkyl, Alkenyl, Alkinyl, Phenyl oder Phenylalkyl;
   oder von agrochemisch wirksamen Salzen davon zur Bekämpfung von tierischen Schädlingen und/oder pflanzenpathogenen Schadpilzen;
(2) ein Mittel zur Bekämpfung von tierischen Schädlingen und/oder pflanzenpathogenen Schadpilzen in und/oder auf Pflanzen oder in und/oder auf Saatgut von Pflanzen, wobei das genannte Mittel wenigstens eine wie unter (1) definierte Verbindung und/oder wenigstens eines von deren agrochemisch wirksamen Salzen oder eine wie unter (2) Mischung und agrochemisch übliche Hilfs- und/oder Zusatzstoffe umfaßt;
(3) die Steigerung der Wirkung gegen tierische Schädlinge von wie unter (1) definierten Verbindung und/oder wenigstens einem von deren agrochemisch wirksamen Salzen oder einem wie unter (2) definierten Mittel durch die Zugabe von Ammonium- oder Phosphoniumsalzen und gegebenenfalls Penetrationsförderern und die entsprechenden Mittel;
(4) ein Verfahren zur Bekämpfung von tierischen Schädlingen und/oder pflanzenpathogenen Schadpilzen in und/oder auf Pflanzen oder in und/oder auf Saatgut von Pflanzen umfassend das direkte oder indirekte Inkontaktbringen der genannten Schädlinge und/oder Schadpilze mit wenigstens einer wie unter (1) definierten Verbindung und/oder wenigstens einem von deren agrochemisch wirksamen Salzen oder einem wie unter (2) und (3) definierten Mittel;
(5) ein Verfahren zur Herstellung eines wie (2) und (3) definierten Mittels umfassend das Vermischen wenigstens einer wie unter (1) definierten Verbindung und/oder wenigstens eines von deren agrochemisch wirksamen Salzen mit agrochemisch üblichen Hilfs-und/oder Zusatzstoffen;
(6) Saatgut, welches mit wenigstens einer wie unter (1) definierten Verbindung und/oder wenigstens einem von deren agrochemisch wirksamen Salzen oder einem wie unter (2) und (3) definierten Mittel behandelt wurde;
(7) Zwischenprodukte zur Herstellung von wie unter (1) definierten Verbindungen, die ebenfalls zur Bekämpfung von tierischen Schädlingen und/oder pflanzenpathogenen Schadpilzen geeignet sind; und
(8) Verfahren zur Herstellung von wie unter (1) definierten Verbindungen sowie deren Zwischenprodukte.

Bevorzugte Substituenten, Fragmente bzw. Bereiche der in der oben und nachstehend erwähnten Formeln aufgeführten Reste werden im folgenden für eine bevorzugte Ausführungsform der vorliegenden Erfindung erläutert:
- X: steht bevorzugt für Wasserstoff, Fluor, Chlor, Brom, Cyano, C₁-C₆-Alkyl oder C₁-C₄-Halogenalkyl mit 1 bis 9 Halogenatomen;
- Z: steht bevorzugt für jeweils gegebenenfalls durch 1 bis 7 Halogenatome substituiertes geradkettiges oder verzweigtes C₁-C₆-Alkoxy, C₁-C₆-Alkylthio, C₁-C₆-Alkylsulfonyl oder die Gruppe 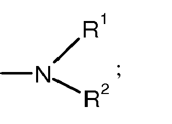
- Y: steht bevorzugt für Phenyl, das gegebenenfalls einfach bis vierfach, gleichartig oder verschieden substituiert sein kann durch
Halogen, Cyano, Nitro, Amino, Hydroxy, Formyl, Carboxy, Carboxyalkyl, Carbamoyl, Thiocarbamoyl;
jeweils geradkettiges oder verzweigtes Alkyl, Alkoxy, Alkoxyalkoxy, Dialkylaminoalkoxy, Alkylthio, Alkylsulfinyl oder Alkylsulfonyl mit jeweils 1 bis 6 Kohlenstoffatomen;
jeweils geradkettiges oder verzweigtes Alkenyl, Alkinyl, Alkinyloxy oder Alkenyloxy mit jeweils 2 bis 6 Kohlenstoffatomen;
jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen;
jeweils geradkettiges oder verzweigtes Halogenalkenyl oder Halogenalkenyloxy mit jeweils 2 bis 6 Kohlenstoffatomen und 1 bis 11 gleichen oder verschiedenen Halogenatomen;
jeweils geradkettiges oder verzweigtes Alkylamino, Dialkylamino, Alkylcarbonyl, Alkylcarbonyloxy, Alkoxycarbonyl, Alkylsulfonyloxy, Hydroximinoalkyl oder Alkoximinoalkyl mit jeweils 1 bis 6 Kohlenstoffatomen in den einzelnen Alkylteilen;
gegebenenfalls einfach bis zweifach, durch Fluor, Chlor, C₁-C₃-Alkyl oder C₁-C₃-Alkoxy substituiertes Cycloalkyl mit 3 bis 8 Kohlenstoffatomen;
in 2,3-Position oder 3,4-Position verknüpftes 1,3-Propandiyl, 1,4-Butandiyl, Methylendioxy (-O-CH₂-O-) oder 1,2-Ethylendioxy (-O-CH₂-CH₂-O-), wobei diese Reste einfach oder mehrfach, gleichartig oder verschieden substituiert sein können durch Halogen, Alkyl mit 1 bis 4 Kohlenstoffatomen und/oder Halogenalkyl mit 1 bis 4 Kohlenstoffatomen und 1 bis 9 gleichen oder verschiedenen Halogenatomen; oder
- Y: steht bevorzugt für gesättigtes oder ganz oder teilweise ungesättigtes oder aromatisches Heterocyclyl mit 3 bis 8 Ringgliedern und 1 bis 3 Heteroatomen aus der Gruppe Stickstoff, Sauerstoff und Schwefel, wobei das Heterocyclyl einfach oder zweifach substituiert sein kann durch Halogen, Alkyl mit 1 bis 4 Kohlenstoffatomen, Alkoxy mit 1 bis 4 Kohlenstoffatomen, Alkylthio mit 1 bis 4 Kohlenstoffatomen, Haloalkoxy mit 1 bis 4 Kohlenstoffatomen, Haloalkylthio mit 1 bis 4 Kohlenstoffatomen, Hydroxy, Mercapto, Cyano, Nitro und/oder Cycloalkyl mit 3 bis 6 Kohlenstoffatomen oder/und Carboxyalkyl;
- W: steht bevorzugt für 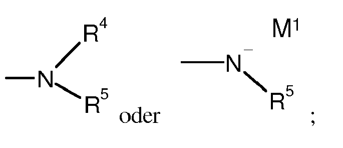
- R¹: stehtsteht bevorzugt für Wasserstoff, Alkyl mit 1 bis 10 Kohlenstoffatomen, das unsubstituiert oder einfach bis fünffach, gleichartig oder verschieden substituiert ist durch Cyano, Hydroxy, Alkoxy mit 1 bis 4 Kohlenstoffatomen, Alkylcarbonyloxy mit 1 bis 6 Kohlenstoffatomen, Alkoxycarbonyloxy mit 1 bis 6 Kohlenstoffatomen, Cycloalkyl mit 3 bis 6 Kohlenstoffatomen, Mercapto, Alkylthio mit 1 bis 4 Kohlenstoffatomen, Amino, Mono- oder Dialkylamino mit jeweils 1 bis 4 Kohlenstoffatomen; oder
- R¹: steht bevorzugt für Alkenyl mit 3 bis 10 Kohlenstoffatomen, das unsubstituiert oder einfach bis dreifach, gleichartig oder verschieden substituiert ist durch Halogen, Cyano, Hydroxy, Alkoxy mit 1 bis 4 Kohlenstoffatomen, Cycloalkyl mit 3 bis 6 Kohlenstoffatomen, Mercapto, Alkylthio mit 1 bis 4 Kohlenstoffatomen, Amino, Mono- oder Dialkylamino mit jeweils 1 bis 4 Kohlenstoffatomen; oder
- R¹: steht bevorzugt für Alkinyl mit 3 bis 10 Kohlenstoffatomen, das unsubstituiert oder einfach bis dreifach, gleichartig oder verschieden substituiert ist durch Halogen, Cyano, Hydroxy, Alkoxy mit 1 bis 4 Kohlenstoffatomen, Cycloalkyl mit 3 bis 6 Kohlenstoffatomen, Mercapto, Alkylthio mit 1 bis 4 Kohlenstoffatomen, Amino, Mono- oder Dialkylamino mit jeweils 1 bis 4 Kohlenstoffatomen; oder
- R¹: steht bevorzugt für Cycloalkyl mit 3 bis 10 Kohlenstoffatomen, das unsubstituiert oder einfach bis dreifach, gleichartig oder verschieden substituiert ist durch Halogen und/oder Alkyl, Halogenalkyl mit jeweils 1 bis 4 Kohlenstoffatomen; oder
- R1: steht bevorzugt für Halogenalkyl mit 1 bis 10 Kohlenstoff- und 1 bis 21 Halogenatomen; oder
- R¹: steht bevorzugt für gesättigtes oder ungesättigtes Heterocyclyl mit 3 bis 10 Ringgliedern und 1 bis 3 Heteroatomen, wie Stickstoff, Sauerstoff und/oder Schwefel, wobei das Heterocyclyl unsubstituiert oder einfach oder mehrfach substituiert ist durch Halogen, Alkyl mit 1 bis 4 Kohlenstoffatomen, Alkoxy mit 1 bis 4 Kohlenstoffatomen, Cyano, Nitro, Cycloalkyl mit 3 bis 6 Kohlenstoffatomen, Hydroxy, Alkoxy, Alkenyloxy, Alkinyloxy mit 1 bis 6 Kohlenstoffatomen oder Mercapto;
- R²: steht bevorzugt für Wasserstoff oder Alkyl mit 1 bis 6 Kohlenstoffatomen; oder
- R¹ und R²: stehen bevorzugt gemeinsam mit dem Stickstoffatom, an das sie gebunden sind, für einen gesättigten oder ungesättigten heterocyclischen Ring mit 3 bis 8 Ringgliedern, wobei der Heterocyclus gegebenenfalls ein weiteres Stickstoff-, Sauerstoff- oder Schwefelatom als Ringglied enthält und wobei der Heterocyclus unsubstituiert oder bis zu dreifach substituiert sein kann durch Fluor, Chlor, Brom, Alkyl mit 1 bis 4 Kohlenstoffatomen, Halogenalkyl mit 1 bis 4 Kohlenstoffatomen und 1 bis 9 Fluor- und/oder Chloratomen, Hydroxy, Alkoxy mit 1 bis 4 Kohlenstoffatomen, Hydroxyalkyl mit 1 bis 4 Kohlenstoffatomen, Alkylcarbonyloxyalkyl mit 1 bis 8 Kohlenstoffatomen, Alkyloxycarbonyloxyalkyl mit 1 bis 8 Kohlenstoffatomen, Haloalkoxy mit 1 bis 4 Kohlenstoffatomen und 1 bis 9 Fluor und/oder Chloratomen, Mercapto, Thioalkyl mit 1 bis 4 Kohlenstoffatomen und/oder Haloalkylthio mit 1 bis 4 Kohlenstoffatomen und 1 bis 9 Fluor und/oder Chloratomen;
- R⁴: steht bevorzugt für Wasserstoff,
für jeweils gegebenenfalls einfach bis mehrfach durch Fluor, Chlor, C₁-C₄-Alkoxy, C₁-C₄-Alkylthio, Cyano, CO₂-H oder CO-O-C₁-C₄-Alkyl substituiertes C₁-C₁₀-Alkyl, C₃-C₁₀-Alkenyl oder C₃-C₁₀-Alkinyl, für jeweils gegebenenfalls einfach bis mehrfach durch Fluor, Chlor, C₁-C₄-Alkyl, C₁-C₄-Halogenalkyl, C₁-C₄-Alkoxy, CN, CO₂H oder CO-O-C₁-C₄-Alkyl substituiertes C₃-C₈-Cycloalkyl oder C₅-C₈-Cyloalkenyl, welches gegebenenfalls durch ein Sauerstoff- oder Schwefelatom unterbrochen sein kann,
für jeweils gegebenenfalls einfach bis dreifach durch Fluor, Chlor, Brom C₁-C₄-Alkyl, C₁-C₄-Halogenalkyl, C₁-C₄-Alkoxy, C₁-C₄-Halogenalkoxy, Nitro oder Cyano substituiertes Phenyl, Pyridyl, Thiazolyl, Pyrimidyl, Benzyl, Phenethyl, Pyridinylmethyl oder Thiazolylmethyl;
- M¹: steht bevorzugt für Kationen aus der Reihe der Alkali- oder Erdalkalimetalle oder Ammoniumionen NH₄, mono-(C₁-C₁₀)-Alkylammonium, di-(C₁-C₁₀)-Alkylammonium, tri-(C₁-C₁₀)-Alkylammonium, tetra-(C₁-C₁₀)-Alkylammonium, wobei die Alkylreste der Ammoniumionen mit Aryl oder Hydroxy substituiert sein können, oder Cholinium,
- R⁵: steht bevorzugt für die Gruppen COR⁷, S(O)₁₋₂R⁷, Cyano, COOR⁷, 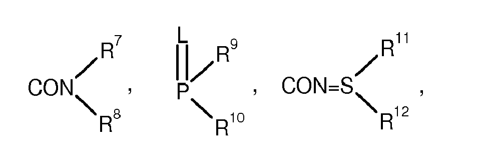 oder für jeweils gegebenenfalls einfach bis zweifach durch Fluor, Chlor, Brom, C₁-C₄Alkyl, C₁-C₄-Alkoxy, Cyano oder Nitro substituiertes Pyridin, Pyrimidin, Triazin, Thiazol oder Pyrazol,
- L: steht für Sauerstoff oder Schwefel,
- R⁷: steht bevorzugt für Wasserstoff, für jeweils gegebenenfalls einfach bis mehrfach durch C₁-C₄-Alkoxy, C₁-C₄-Alkylthio, Cyano, CO₂-H oder CO-O-C₁-C₄-Alkyl substituiertes C₁-C₁₀-Alkyl, C₃-C₁₀-Alkenyl oder C₃-C₁₀-Alkinyl, für C1-C10-Halogenalkyl mit 1 bis 21 Fluor- und/oder Chloratomen für jeweils gegebenenfalls einfach bis mehrfach durch Fluor, Chlor, C₁-C₄-Alkyl, C₁-C₄-Halogenalkyl, C₁-C₄-Alkoxy, CN, CO₂H oder CO-O-C₁-C₄-Alkyl substituiertes C₃-C₈-Cycloalkyl, C₃-C₆-Cycloalkyl-C₁-C₂-alkyl oder C₅-C₈-Cycloalkenyl, welches gegebenenfalls durch ein Sauerstoff- oder Schwefelatom unterbrochen sein kann,
für jeweils gegebenenfalls einfach bis dreifach durch Fluor, Chlor, Brom, C₁-C₄-Alkyl, C₁-C₄-Halogenalkyl, C₁-C₄-Alkoxy, C₁-C₄-Halogenalkoxy, Nitro oder Cyano substituiertes Phenyl, Pyridyl, Thiazolyl, Pyrimidyl, Benzyl, Phenethyl, Pyridinylmethyl oder Thiazolylmethyl,
- R⁸: steht bevorzugt für Wasserstoff, jeweils gegebenenfalls einfach bis mehrfach durch Fluor und/oder Chlor substituiertes C₁-C₆-Alkyl, C₃-C₆-Alkenyl, C₃-C₆-Alkinyl oder C₁-C₆-Alkoxy; oder
- R⁷ und R⁸: stehen bevorzugt gemeinsam mit dem Stickstoffatom, an das sie gebunden sind, für einen gegebenenfalls einfach bis mehrfach durch C₁-C₄-Alkyl substituierten gesättigten oder ungesättigten 5- oder 6-Ring, der gegebenenfalls durch Sauerstoff oder Schwefel unterbrochen sein kann;
- R⁹ und R¹⁰: stehen bevorzugt unabhängig voneinander für jeweils gegebenenfalls einfach bis mehrfach durch Fluor und/oder Chlor substituiertes C₁-C₈-Alkyl, C₃-C₈-Alkenyl, C₁-C₈-Alkoxy, C₃-C₈-Alkenyloxy, C₃-C₈-Alkinyloxy, C₁-C₆-Alkylthio, C₃-C₆-Alkenylthio, jeweils gegebenenfalls einfach bis zweifach durch Fluor, Chlor, C₁-C₄-Alkyl, C₁-C₄-Halogenalkyl, C₁-C₄-Alkoxy substituiertes C₃-C₈-Cycloalkoxy, C₄-C₈-Cycloalkenyloxy oder C₃-C₈-Cycloalkyl-C₁-C₂-alkoxy, für jeweils gegebenenfalls einfach bis dreifach durch Fluor, Chlor, Brom, C₁-C₆-Alkyl-C₁-C₄-Halogenalkyl, C₁-C₆-Alkoxy, C₁-C₄-Halogenalkoxy, C₁-C₆-Alkylthio, Cyano oder Nitro substituiertes Phenoxy, Phenylthio, Benzyloxy, Benzylthio, Pyridinyloxy, Pyrazolyloxy, Pyridinylthio, Pyrimidylthio, Thiazolylthio, Pyridyl-C₁- C₂-alkoxy, Pyrimidyl-C₁-C₂-alkyloxy, Thiazolyl-C₁-C₂-alkyloxy, Pyridyl-C₁-C₂-alkylthio, Thiazolyl-C₁-C₂-alkylthio oder Pyrimidyl-C₁-C₂-alkylthio; oder
- R⁹ und R¹⁰: stehen bevorzugt gemeinsam mit dem Phosphoratom an das sie gebunden sind, für einen gegebenenfalls einfach bis dreifach durch Fluor, Chlor, C₁-C₄-Alkyl, C₁-C₄-Alkoxy oder C₁-C₄-Halogenalkyl substituierten fünf- bis siebengliedrigen Cyclus der durch ein oder zwei Sauerstoff- und/oder Schwefelatome unterbrochen sein kann;
- R¹¹ und R¹²: stehen bevorzugt unabhängig voneinander für jeweils gegebenenfalls einfach bis dreifach durch Fluor oder Chlor substituiertes C₁-C₈-Alkyl, C₃-C₈-Alkenyl oder C₃-C₈-Alkinyl, für jeweils gegebenenfalls einfach bis zweifach durch Fluor, Chlor, Brom, C₁-C₄-Alkyl, C₁-C₄-Halogenalkyl, C₁-C₄-Alkoxy, C₁-C₄-Halogenalkoxy, Cyano oder Nitro substituiertes Phenyl oder Benzyl;
Die erfindungsgemäßen Verbindungen sind durch die Formel (I) allgemein definiert. Besonders bevorzugte Substituenten, Fragmente bzw. Bereiche der in der oben und nachstehend erwähnten Formeln aufgeführten Reste werden im Folgenden für eine besonders bevorzugte Ausführungsform der vorliegenden Erfindung erläutert:
- X: steht besonders bevorzugt für Wasserstoff, Fluor, Chlor, Brom, Cyano, C₁-C₄-Alkyl oder C₁-C₂-Halogenalkyl, welches mit ein bis fünf Fluor- und/oder Chloratome substituiert sein kann;
- Z: steht besonders bevorzugt für jeweils gegebenenfalls durch 1 bis 3 Halogenatome substituiertes geradkettiges oder verzweigtes C₁-C₄-Alkoxy, C₁-C₄-Alkylthio oder die Gruppe 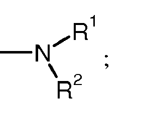
- Y: steht besonders bevorzugt für Phenyl, das gegebenenfalls einfach bis dreifach, gleichartig oder verschieden substituiert sein kann durch Fluor, Chlor, Brom, Jod, Cyano, Nitro, Formyl, Methyl, Ethyl, n- oder i-Propyl, n-, i-, s- oder t-Butyl, Vinyl, 1-Propenyl, Ethinyl, 1-Propinyl, Allyl, Propargyl, Methoxy, Ethoxy, n- oder i-Propoxy, 2-Methoxy-ethoxy, 3-Methoxypropoxy, 2-(*N,N*-Dimethylamino)-ethoxy, 3-(*N,N*-Dimethylamino)-propoxy, Methylthio, Ethylthio, n- oder i-Propylthio, Methylsulfinyl, Ethylsulfinyl, Methylsulfonyl, Ethylsulfonyl, Allyloxy, Propargyloxy, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl, Trifluormethylsulfonyl, Trichlorethinyloxy, Trifluorethinyloxy, Chlorallyloxy, Iodpropargyloxy, Methylamino, Ethylamino, n- oder i-Propylamino, Dimethylamino, Diethylamino, Acetyl, Propionyl, Acetyloxy, Methoxycarbonyl, Ethoxycarbonyl, Hydroximinomethyl, Hydroximinoethyl, Methoximinomethyl, Ethoximinomethyl, Methoximinoethyl, Ethoximinoethyl, Cyclopropyl, Cyclobutyl, Cyclopentyl oder Cyclohexyl, oder durch
in 2,3-Position oder 3,4-Position verknüpftes 1,3-Propandiyl, 1,4-Butandiyl, Methylendioxy (-O-CH₂-O-) oder 1,2-Ethylendioxy (-O-CH₂-CH₂-O-), wobei diese Reste einfach oder mehrfach, gleichartig oder verschieden substituiert sein können durch Fluor, Chlor, Methyl, Ethyl, n-Propyl, i-Propyl Trifluormethyl, Carboxy und/oder Carboxymethyl;
- W: steht besonders bevorzugt für

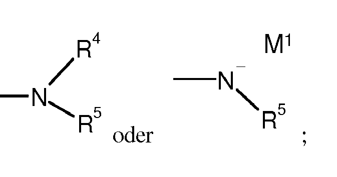

- R¹: steht besonders bevorzugt für Wasserstoff, Alkyl mit 1 bis 8 Kohlenstoffatomen, das unsubstituiert oder einfach bis dreifach, gleichartig oder verschieden substituiert ist, durch Cyano, Alkoxy mit 1 bis 3 Kohlenstoffatomen, Hydroxy, Alkylcarbonyloxy mit 1 bis 5 Kohlenstoffatomen, Alkoxycarbonyloxy mit 1 bis 5 Kohlenstoffatomen, Cycloalkyl mit 3 bis 6 Kohlenstoffatomen, Alkylthio mit 1 bis 3 Kohlenstoffatomen; oder
- R¹: steht besonders bevorzugt für Alkenyl mit 3 bis 8 Kohlenstoffatomen, das unsubstituiert oder einfach bis dreifach, gleichartig oder verschieden substituiert ist durch Fluor, Chlor, Cyano, Alkoxy mit 1 bis 3 Kohlenstoffatomen, Cycloalkyl mit 3 bis 6 Kohlenstoffatomen, Alkylthio mit 1 bis 3 Kohlenstoffatomen; oder
- R¹: steht besonders bevorzugt für Alkinyl mit 3 bis 8 Kohlenstoffatomen, das unsubstituiert oder einfach bis dreifach, gleichartig oder verschieden substituiert ist durch Fluor, Chlor, Cyano, Alkoxy mit 1 bis 3 Kohlenstoffatomen, Cycloalkyl mit 3 bis 6 Kohlenstoffatomen, Alkylthio mit 1 bis 3 Kohlenstoffatomen; oder
- R¹: steht besonders bevorzugt für Cycloalkyl mit 3 bis 8 Kohlenstoffatomen, das unsubstituiert oder einfach bis dreifach, gleichartig oder verschieden substituiert ist durch Fluor, Chlor und/oder Alkyl, Halogenalkyl, Alkoxy mit jeweils 1 bis 2 Kohlenstoffatomen; oder
- R¹: steht besonders bevorzugt für Halogenalkyl mit 1 bis 8 Kohlenstoff- und 1 bis 17 Fluor-und/oder Chloratomen; oder
- R¹: steht besonders bevorzugt für gesättigtes oder ungesättigtes Heterocyclyl mit 3 bis 8 Ringgliedern und 1 bis 2 Heteroatomen, wie Stickstoff, Sauerstoff und/oder Schwefel, steht, wobei das Heterocyclyl unsubstituiert oder einfach oder zweifach substituiert ist durch Hydroxy, Fluor, Chlor, Alkyl mit 1 bis 3 Kohlenstoffatomen, Alkoxy mit 1 bis 3 Kohlenstoffatomen, Cyano, Nitro oder Cycloalkyl mit 3 bis 6 Kohlenstoffatomen;
- R²: steht besonders bevorzugt für Wasserstoff oder Alkyl mit 1 bis 6 Kohlenstoffatomen; oder
- R¹ und R²: stehen besonders bevorzugt für gemeinsam mit dem Stickstoffatom, an das sie gebunden sind, für einen gesättigten oder ungesättigten heterocyclischen Ring mit 3 bis 6 Ringgliedern, wobei der Heterocyclus gegebenenfalls ein weiteres Stickstoff-, Sauerstoff-oder Schwefelatom als Ringglied enthält und wobei der Heterocyclus unsubstituiert oder bis zu dreifach substituiert sein kann durch Fluor, Chlor, Brom, Alkyl mit 1 bis 3 Kohlenstoffatomen, Halogenalkyl mit 1 bis 3 Kohlenstoffatomen und 1 bis 5 Fluor- und/oder Chloratomen, Alkoxy mit 1 bis 3 Kohlenstoffatomen, Hydroxyalkyl mit 1 bis 3 Kohlenstoffatomen, Alkylcarbonyloxyalkyl mit 1 bis 7 Kohlenstoffatomen, Alkyloxycarbonyloxyalkyl mit 1 bis 7 Kohlenstoffatomen, Halogenalkoxy mit 1 bis 3 Kohlenstoffatomen und 1 bis 5 Fluor und/oder Chloratomen, Thioalkyl mit 1 bis 3 Kohlenstoffatomen und/oder Halogenalkylthio mit 1 bis 3 Kohlenstoffatomen und 1 bis 5 Fluor und/oder Chloratomen;
- R⁴: steht besonders bevorzugt für Wasserstoff,
für jeweils gegebenenfalls einfach bis dreifach durch Fluor, Chlor, C₁-C₃-Alkoxy, C₁-C₃-Alkylthio, Cyano, CO₂H oder CO-O-C₁-C₃-Alkyl substituiertes C₁-C₈-Alkyl, C₃-C₈-Alkenyl oder C₃-C₈-Alkinyl, für jeweils gegebenenfalls einfach bis dreifach durch Fluor, Chlor, C₁-C₃-Alkyl, C₁-C₂-Halogenalkyl, C₁-C₃-Alkoxy, CN, CO₂H oder CO-O-C₁-C₃-Alkyl substituiertes C₃-C₆-Cycloalkyl, C₃-C₆-Cycloalkyl-methyl oder C₅-C₆-Cycloalkenyl, welches gegebenenfalls durch ein Sauerstoff-oder Schwefelatom unterbrochen sein kann,
für jeweils gegebenenfalls einfach bis zweifach durch Fluor, Chlor, Brom, C₁-C₃-Alkyl, C₁-C₃-Halogenalkyl, C₁-C₃-Alkoxy, C₁-C₃-Halogenalkoxy, Nitro oder Cyano substituiertes Phenyl, Pyridyl, Thiazolyl, Pyrimidyl, Benzyl, Phenethyl, Pyridinylmethyl oder Thiazolylmethyl;
- M¹: steht besonders bevorzugt für Na⁺, K⁺, ½ Ca²⁺, ½ Mg²⁺, NH₄⁺, NH₃CH₃⁺, NH₂(CH₃)₂⁺, NH(CH₃)₃⁺, NH(C₂H₅)₃⁺, NH₂(C₂H₅)₂⁺, NH₃C₂H₅⁺, NH₃i-C₃H₇⁺, NH₂(i-C₃H₇)₂⁺, NH₃-CH₂-C₆H₅⁺, N(CH₃)₃-H₂-C₆H₅⁺, 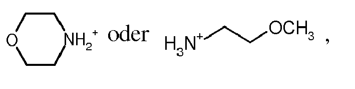
- R⁵: steht besonders bevorzugt für für die Gruppen COR⁷, S(O)₁₋₂R⁷, Cyano, COOR⁷, 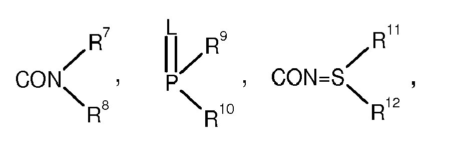 oder für jeweils gegebenenfalls einfach bis zweifach durch Fluor, Chlor, Brom, C₁-C₄Alkyl, C₁-C₄-Alkoxy, Cyano oder Nitro substituiertes Pyridin, Pyrimidin, Triazin, Thiazol oder Pyrazol,
- L: steht besonders bevorzugt für Sauerstoff oder Schwefel,
- R⁷: steht besonders bevorzugt Wasserstoff, für jeweils gegebenenfalls einfach bis fünffach durch C₁-C₃-Alkoxy, C₁-C₃-Alkylthio, Cyano, CO₂H oder CO-O-C₁-C₃-Alkyl substituiertes C₁-C₈-Alkyl, C₃-C₈-Alkenyl oder C₃-C₈-Alkinyl, für C1-C8-Halogenalkyl mit 1 bis 17 Fluor- und/oder Chloratomen, für jeweils gegebenenfalls einfach bis dreifach durch Fluor, Chlor, C₁-C₄-Alkyl, C₁-C₂-Halogenalkyl, C₁-C₂-Alkoxy, CN, CO₂H oder CO-O-C₁-C₂-Alkyl substituiertes C₃-C₆-Cycloalkyl, C₃-C₆-Cycloalkyl-methyl oder C₅-C₆-Cycloalkenyl, welches gegebenenfalls durch ein Sauerstoff- oder Schwefelatom unterbrochen sein kann,
für jeweils gegebenenfalls einfach bis zweifach durch Fluor, Chlor, Brom, C₁-C₃-Alkyl, C₁-C₃-Halogenalkyl, C₁-C₃-Alkoxy, C₁-C₃-Halogenalkoxy, Nitro oder Cyano substituiertes Phenyl, Pyridyl, Thiazolyl, Pyrimidyl, Benzyl, Phenethyl, Pyridinylmethyl oder Thiazolylmethyl,
- R⁸: steht besonders bevorzugt für Wasserstoff, jeweils gegebenenfalls einfach bis dreifach durch Fluor und/oder Chlor substituiertes C₁-C₄-Alkyl, C₃-C₄-Alkenyl, C₃-C₄-Alkinyl oder C₁-C₄-Alkoxy;
oder
- R⁷ und R⁸: stehen besonders bevorzugt gemeinsam mit dem Stickstoffatom, an das sie gebunden sind, für einen gegebenenfalls einfach bis zweifach durch C₁-C₂-Alkyl substituierten gesättigten oder ungesättigten 5- oder 6-Ring, der gegebenenfalls durch Sauerstoff oder Schwefel unterbrochen sein kann;
- R⁹ und R¹⁰: stehen besonders bevorzugt unabhängig voneinander für jeweils gegebenenfalls einfach bis dreifach durch Fluor und/oder Chlor substituiertes C₁-C₆-Alkyl, C₃-C₆-Alkenyl, C₁-C₆-Alkoxy, C₃-C₆-Alkenyloxy, C₃-C₆-Alkinyloxy, C₁-C₄-Alkylthio, C₃-C₄-Alkenylthio, jeweils gegebenenfalls einfach bis zweifach durch Fluor, Chlor, C₁-C₂-Alkyl, C₁-C₂-Halogenalkyl, C₁-C₂-Alkoxy substituiertes C₃-C₆-Cycloalkoxy, C₄-C₆-Cycloalkenyloxy oder C₃-C₆-Cycloalkyl-C₁-C₂-alkyloxy, für jeweils gegebenenfalls einfach bis zweifach durch Fluor, Chlor, Brom, C₁-C₄-Alkyl-C₁-C₂-Halogenalkyl, C₁-C₄-Alkoxy, C₁-C₂-Halogenalkoxy, C₁-C₄-Alkylthio, Cyano oder Nitro substituiertes Phenoxy, Phenylthio, Benzyloxy, Benzylthio, Pyridinyloxy, Pyrazolyloxy, Pyridinylthio, Pyrimidylthio, Thiazolylthio, Pyridyl-methyloxy, Pyrimidyl-methyloxy, Thiazolyl-methyloxy, Pyridyl-methylthio, Thiazolyl-methylthio oder Pyrimidyl-methylthio; oder
- R⁹ und R¹⁰: stehen besonders bevorzugt gemeinsam mit dem Phosphoratom an das sie gebunden sind, für einen gegebenenfalls einfach bis zweifach durch Fluor, Chlor, C₁-C₂-Alkyl, C₁-C₂-Alkoxy oder C₁-C₂-Halogenalkyl substituierten fünf- bis sechsgliedrigen Cyclus der durch ein oder zwei Sauerstoff- und/oder Schwefelatome unterbrochen sein kann; und
- R¹¹ und R¹²: stehen besonders bevorzugt unabhängig voneinander für jeweils gegebenenfalls einfach bis dreifach durch Fluor oder Chlor substituiertes C₁-C₆-Alkyl, C₃-C₆-Alkenyl oder C₃-C₆-Alkinyl;

Die erfindungsgemäßen Verbindungen sind durch die Formel (I) allgemein definiert. Ganz besonders bevorzugte Substituenten, Fragmente bzw. Bereiche der in der oben und nachstehend erwähnten Formeln aufgeführten Rest werden im Folgenden für eine ganz besonders bevorzugte Ausführungsform der vorliegenden Erfindung erläutert:
- X: steht ganz besonders bevorzugt für Wasserstoff, Chlor, Brom, Methyl oder Trifluormethyl;
- Z: steht ganz besonders bevorzugt für Methoxy, Ethoxy, Methylthio, Ethythio oder die Gruppe 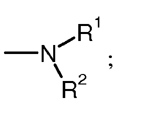
- Y: steht ganz besonders bevorzugt für einfach bis dreifach substituiertes Phenyl mit Substituenten aus der Gruppe Fluor, Chlor, Brom, Cyano, Nitro, Methyl, Ethyl, tert.-Butyl, Methoxy, Ethoxy, 2-Methoxy-ethoxy, 3-Methoxy-propoxy, 2-(*N,N*-Dimethylamino)-ethoxy, 3-(*N,N*-Dimethylamino)-propoxy, Trifluormethyl, Difluormethoxy oder Trifluormethoxy;
- W: steht ganz besonders bevorzugt für

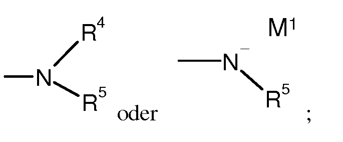

- R¹: steht ganz besonders bevorzugt für Wasserstoff, Methyl, Ethyl, Propyl, Butyl, das unsubstituiert oder einfach bis vierfach, gleichartig oder verschieden substituiert ist, durch Methyl, Ethyl, Propyl, Butyl, Hydroxy, Acetyloxy, Isobutyryloxy, Methoxy, Ethoxy, Cyclopropyl, Cyclopentyl, Cyclohexyl, Methylthio oder Ethylthio; oder
- R¹: steht ganz besonders bevorzugt für Alkenyl mit 3 bis 6 Kohlenstoffatomen, das unsubstituiert oder einfach bis dreifach, gleichartig oder verschieden substituiert ist durch Fluor, Chlor, Methoxy, Ethoxy, Methylthio oder Ethylthio; oder
- R¹: steht ganz besonders bevorzugt für Alkinyl mit 3 bis 6 Kohlenstoffatomen, das unsubstituiert oder einfach bis dreifach, gleichartig oder verschieden substituiert ist durch Fluor, Chlor, Methoxy, Ethoxy, Methylthio oder Ethylthio; oder
- R¹: steht ganz besonders bevorzugt für Cycloalkyl mit 3 bis 6 Kohlenstoffatomen, das unsubstituiert oder einfach bis dreifach, gleichartig oder verschieden substituiert ist durch Fluor, Chlor, Methyl, Ethyl, Methoxy oder Trifluormethyl;
- R1: steht ganz besonders bevorzugt für Methyl, Ethyl, Propyl oder Butyl, das mit 1 bis 9 Fluor-und /oder Chloratomen substituiert ist; oder
- R²: steht ganz besonders bevorzugt für Wasserstoff, Methyl oder Ethyl; oder
- R¹ und R²: stehen ganz besonders bevorzugt gemeinsam mit dem Stickstoffatom, an das sie gebunden sind, für einen gesättigten oder ungesättigten heterocyclischen Ring mit 3 bis 6 Ringgliedern, wobei der Heterocyclus gegebenenfalls ein weiteres Stickstoff-, Sauerstoff-oder Schwefelatom als Ringglied enthält und wobei der Heterocyclus unsubstituiert oder bis zu zweifach substituiert sein kann durch Fluor, Chlor, Methyl, Ethyl, Trifluormethyl, Methoxy, Ethoxy, Hydroxymethyl, Acetyloxymethyl oder Isobutyryloxymethyl;
- R⁴: steht ganz besonders bevorzugt für Wasserstoff,;
- M¹: steht ganz besonders bevorzugt für Na⁺, K⁺, ½ Ca²⁺, ½ Mg²⁺, NH₄⁺, NH₃CH₃⁺, NH₂(CH₃)₂⁺, NH(CH₃)₃⁺, NH(C₂H₅)₃⁺, NH₂(C₂H₅)₂⁺, NH₃C₂H₅⁺, NH₃i-C₃H₇⁺, NH₂(i-C₃H₇)₂⁺, NH₃-CH₂-C₆H₅⁺ oder N(CH₃)₃-H₂-C₆H₅⁺;
- R⁵: steht ganz besonders bevorzugt für die Gruppen COR⁷, S(O)₁₋₂R⁷, Cyano, COOR⁷, 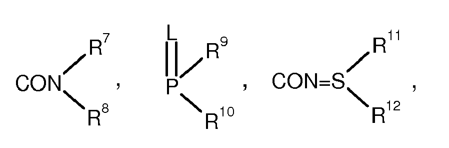
- L: steht für Sauerstoff oder Schwefel,
- R⁷: steht ganz besonders bevorzugt für Wasserstoff, für jeweils gegebenenfalls einfach bis dreifach durch Methoxy, Ethoxy, Methylthio, Ethylthio, Cyano, CO-O-Methyl oder CO-O-Ethyl substituiertes Methyl, Ethyl, n-Propyl, iso-Propyl oder Cyclopropyl, für Halogenmethyl, -propyl, -n-propyl, -i-propyl mit 1 bis 7 Fluor- und/oder Chloratomen,
- R⁸: steht ganz bevorzugt für Wasserstoff, Methyl, Ethyl, Propyl, iso-Propyl, Allyl, Propargyl, C₃-C₄-Alkenyl, Methoxy oder Ethoxy; oder
- R⁷ und R⁸: stehen ganz besonders bevorzugt gemeinsam mit dem Stickstoffatom, an das sie gebunden sind, für einen gesättigten 5- oder 6-Ring, der gegebenenfalls durch Sauerstoff oder Schwefel unterbrochen sein kann; und
- R⁹ und R¹⁰: stehen ganz besonders bevorzugt unabhängig voneinander für jeweils gegebenenfalls einfach bis dreifach durch Fluor und/oder Chlor substituiertes Methyl, Ethyl, Methoxy, Ethoxy, Propoxy, i-Propoxy, Butoxy, Alkyloxy, Methylthio, Ethylthio, Propylthio, Butylthio, i-Propylthio, sek.-Butylthio, Allylthio.

Die erfindungsgemäßen Verbindungen sind durch die Formel (I) allgemein definiert. Insbesondere ganz besonders bevorzugte Substituenten, Fragmente bzw. Bereiche der in der oben und nachstehend erwähnten Formeln aufgeführten Rest werden im Folgenden für eine ganz besonders bevorzugte Ausführungsform der vorliegenden Erfindung erläutert:
- X: steht insbesondere ganz besonders bevorzugt für Wasserstoff, Chlor, Brom, Methyl oder Trifluormethyl;
- Z: steht insbesondere ganz besonders bevorzugt für Methoxy, Ethoxy, Methylthio, Ethythio oder die Gruppe 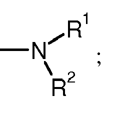
- Y: steht insbesonders ganz besonders bevorzugt für einfach bis dreifach substituiertes Phenyl mit Substituenten aus der Gruppe Fluor, Chlor, Brom, Cyano, Nitro, Methyl, Ethyl, tert.-Butyl, Methoxy, Ethoxy, 2-Methoxy-ethoxy, 3-Methoxy-propoxy, 2-(*N,N*-Dimethylamino)-ethoxy, 3-(*N,N*-Dimethylamino)-propoxy, Trifluormethyl, Difluormethoxy oder Trifluormethoxy;
- W: steht insbesonders ganz bevorzugt für 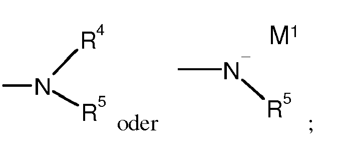
- R¹: steht insbesonders ganz besonders bevorzugt für Wasserstoff, Methyl, Ethyl, Propyl, Butyl, das unsubstituiert oder einfach bis vierfach, gleichartig oder verschieden substituiert ist, durch Methyl, Ethyl, Propyl, Butyl, Hydroxy, Acetyloxy, Isobutyryloxy, Methoxy, Ethoxy, Cyclopropyl, Cyclopentyl, Cyclohexyl, Methylthio oder Ethylthio; oder
- R¹: steht insbesonders ganz besonders bevorzugt für Alkenyl mit 3 bis 6 Kohlenstoffatomen, das unsubstituiert oder einfach bis dreifach, gleichartig oder verschieden substituiert ist durch Fluor, Chlor, Methoxy, Ethoxy, Methylthio oder Ethylthio; oder
- R¹: steht insbesonders ganz besonders bevorzugt für Alkinyl mit 3 bis 6 Kohlenstoffatomen, das unsubstituiert oder einfach bis dreifach, gleichartig oder verschieden substituiert ist durch Fluor, Chlor, Methoxy, Ethoxy, Methylthio oder Ethylthio; oder
- R¹: steht insbesonders ganz besonders bevorzugt für Cycloalkyl mit 3 bis 6 Kohlenstoffatomen, das unsubstituiert oder einfach bis dreifach, gleichartig oder verschieden substituiert ist durch Fluor, Chlor, Methyl, Ethyl, Methoxy oder Trifluormethyl;
- R1: steht ganz besonders bevorzugt für Methyl, Ethyl, Propyl oder Butyl, das mit 1 bis 9 Fluor-und /oder Chloratomen substituiert ist; oder
- R²: steht insbesondere ganz besonders bevorzugt für Wasserstoff, Methyl oder Ethyl; oder
- R¹ und R²: stehen insbesonders ganz besonders bevorzugt gemeinsam mit dem Stickstoffatom, an das sie gebunden sind, für einen gesättigten oder ungesättigten heterocyclischen Ring mit 3 bis 6 Ringgliedern, wobei der Heterocyclus gegebenenfalls ein weiteres Stickstoff-, Sauerstoff- oder Schwefelatom als Ringglied enthält und wobei der Heterocyclus unsubstituiert oder bis zu zweifach substituiert sein kann durch Fluor, Chlor, Methyl, Ethyl, Trifluormethyl, Methoxy, Ethoxy, Hydroxymethyl, Acetyloxymethyl oder Isobutyryloxymethyl;
- R⁴: steht insbesondere ganz besonders bevorzugt für Wasserstoff,;
- M¹: steht ganz besonders bevorzugt für Na⁺, K⁺, ½ Ca²⁺, ½ Mg²⁺, NH₄⁺, NH₃CH₃⁺, NH₂(CH₃)₂⁺, NH(CH₃)₃⁺, NH(C₂H₅)₃⁺, NH₂(C₂H₅)₂⁺, NH₃C₂H₅⁺, NH₃i-C₃H₇⁺, NH₂(i-C₃H₇)₂⁺, NH₃-CH₂-C₆H₅⁺ oder N(CH₃)₃-H₂-C₆H₅⁺;
- R⁵: steht insbesondere ganz besonders bevorzugt für S(O)R⁷, S(O)₂R⁷,
- R⁷: steht insbesondere ganz besonders bevorzugt für Wasserstoff, für jeweils gegebenenfalls einfach bis dreifach durch Methoxy, Ethoxy, Methylthio, Ethylthio, Cyano, CO-O-Methyl oder CO-O-Ethyl substituiertes Methyl, Ethyl, n-Propyl, iso-Propyl oder Cyclopropyl, für Halogenmethyl, -propyl, -n-propyl, -i-propyl mit 1 bis 7 Fluor- und/oder Chloratomen,
- R⁸: steht insbesonders ganz bevorzugt für Wasserstoff, Methyl, Ethyl, Propyl, iso-Propyl, Allyl, Propargyl, C₃-C₄-Alkenyl, Methoxy oder Ethoxy; oder
- R⁷ und R⁸: stehen insbesonders ganz besonders bevorzugt gemeinsam mit dem Stickstoffatom, an das sie gebunden sind, für einen gesättigten 5- oder 6-Ring, der gegebenenfalls durch Sauerstoff oder Schwefel unterbrochen sein kann; und
- R⁹ und R¹⁰: stehen insbesonders ganz besonders bevorzugt unabhängig voneinander für jeweils gegebenenfalls einfach bis dreifach durch Fluor und/oder Chlor substituiertes Methyl, Ethyl, Methoxy, Ethoxy, Propoxy, i-Propoxy, Butoxy, Alkyloxy, Methylthio, Ethylthio, Propylthio, Butylthio, i-Propylthio, sek.-Butylthio, Allylthio.

Hervorgehoben sind Verbindungen der Formel (I), in welcher X für Halogen steht.

Hervorgehoben sind Verbindungen der Formel (I), in welcher Z für die Gruppe 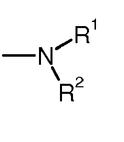 steht.

Hervorgehoben sind Verbindungen der Formel (I), in welcher Z für die Gruppe 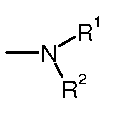 und R² für Wasserstoff steht.

Hervorgehoben sind Verbindungen der Formel (I), in welcher Z für die Gruppe 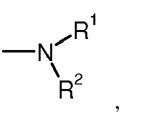
- R¹: für Wasserstoff, Alkyl mit 1 bis 10 Kohlenstoffatomen, das unsubstituiert oder einfach bis fünffach, gleichartig oder verschieden substituiert ist durch Cyano, Hydroxy, Alkoxy mit 1 bis 4 Kohlenstoffatomen, Cycloalkyl mit 3 bis 6 Kohlenstoffatomen, Mercapto, Alkylthio mit 1 bis 4 Kohlenstoffatomen, Amino, Mono- oder Dialkylamino mit jeweils 1 bis 4 Kohlenstoffatomen sowie Halogenalkyl mit 1 bis 10 Kohlenstoff- und 1 bis 21 Halogenatomen und R² für Wasserstoff steht.

Hervorgehoben sind Verbindungen der Formel (I), in welcher R⁴ für Wasserstoff steht.

Hervorgehoben sind Verbindungen der Formel (I), in denen R⁵ für S(O)R⁷ und R⁷ für Alkyl mit 1 bis 3 Kohlenstoffatomen und/ oder Halogenalkyl mit 1 bis 3 Kohlenstoff- und 1 bis 5 Fluor-und/oder Chloratomen und/oder Cyclopropyl steht.

Hervorgehoben sind Verbindungen der Formel (I), in denen R⁵ für S(O)₂R⁷ und R⁷ für Alkyl mit 1 bis 3 Kohlenstoffatomen und/ oder Halogenalkyl mit 1 bis 3 Kohlenstoff- und 1 bis 5 Fluor-und/oder Chloratomen und/oder Cyclopropyl steht.

Hervorgehoben sind Verbindungen der Formel (I), in denen
- R⁵: für S(O)R⁷ oder S(O)₂R⁷ und R⁷ für Wasserstoff, für jeweils gegebenenfalls einfach bis fünffach durch Fluor, Chlor, C₁-C₃-Alkoxy, C₁-C₃-Alkylthio, Cyano, CO₂H oder CO-O-C₁-C₃-Alkyl substituiertes C₁-C₈-Alkyl, C₃-C₈-Alkenyl oder C₃-C₈-Alkinyl, für C₁-C₈-Halogenalkyl mit 1 bis 17 Fluor- und/oder Chloratomen, für jeweils gegebenenfalls einfach bis dreifach durch Fluor, Chlor, C₁-C₄-Alkyl, C₁-C₂-Halogenalkyl, C₁-C₂-Alkoxy, CN, CO₂H oder CO-O-C₁-C₂-Alkyl substituiertes C₃-C₆-Cycloalkyl, C₃-C₆-Cycloalkylmethyl oder C₅-C₆-Cycloalkenyl, welches gegebenenfalls durch ein Sauerstoff- oder Schwefelatom unterbrochen sein kann, steht.

Verbindungen der Formel (I) werden bevorzugt, in denen A ein aromatischer Heterocyclus ist.

Außerdem werden Verbindungen der Formel (I) bevorzugt, in denen A ein drei, fünf- oder sechsgliedriger, insbesondere ein fünfgliedriger Heterocyclus ist.

Insbesondere sind Verbindungen der Formel (I) bevorzugt, in denen A ein stickstoffhaltiger Heterocyclus ist.

Daneben werden Verbindungen der Formel (I) bevorzugt, in denen A ein Heterocyclus ist, der über Stickstoff an den Pyrimidinring gebunden ist.

Gleichermaßen bevorzugt sind Verbindungen der Formel (I), in denen A für folgende Gruppen steht: Pyrrol, Pyrazol, Imidazol, 1,2,4-Triazol, 1,2,3-Triazol, Tetrazol, 1,2,3-Triazin, 1,2,4-Triazin, Oxazol, Isoxazol, 1,3,4-Oxadiazol, 1,3,4-Thiadiazol, Furan, Thiophen, Thiazol, Isothiazol, wobei der Heterocyclus über C oder N an den Pyrimidinring gebunden sein kann.

Gleichermaßen bevorzugt sind Verbindungen der Formel (I), in denen A für gegebenenfalls durch bis zu drei Gruppen R^{d} substituiertes Pyrazol, Pyrrol, Imidazol, 1,2,3-Triazol, 1,2,4-Triazol, 1,3,4-Oxadiazol, 1,3,4-Thiadiazol, Tetrazol, 2-Pyridin, 2-Pyrimidin, Pyrazin oder 3-Pyridazin steht.

Besonders bevorzugt werden Verbindungen der Formel (I), in denen A für Pyrazol, Pyrrol, Imidazol, 1,2,3-Triazol, 1,2,4-Triazol, oder Tetrazol steht.

Daneben werden Verbindungen der Formel (I) besonders bevorzugt, in denen der Cyclus A durch eine bis zwei gleiche oder verschiedene der folgenden Gruppen R^{d} substituiert ist:
Halogen, Hydroxy, Cyano, Nitro, Amino, Mercapto, C₁-C₆-Alkyl, C₁-C₆-Halogenalkyl, C₂-C₆-Alkenyl, C₂-C₆-Alkinyl, C₃-C₆-Cycloalkyl, C₁-C₆-Alkoxy, C₁-C₆-Halogenalkoxy, Oxo, C₁-C₇-Alkylcarbonylamino, C₁-C₆-Alkylamino, Di-(C₁-C₆-alkyl)amino, C₁-C₆-Alkylthio, C₁-C₆-Alkylsulfinyl, C₁-C₆-Alkylsulfonyl, C₁-C₆-Alkylaminosulfonyl oder Di-(C₁-C₆-alkyl)aminosulfonyl.

Insbesondere werden Verbindungen der Formel (I) besonders bevorzugt, in denen der Cyclus A durch eine bis zwei gleiche oder verschiedene der folgenden Gruppen R^{d} substituiert ist:
Halogen, Cyano, Nitro, Hydroxy, Amino, C₁-C₆-Alkyl, C₁-C₆-Halogenalkyl, C₁-C₆-Alkoxy, der C₁-C₇-Alkylcarbonylamino oder Oxo.

Besonders bevorzugt werden Verbindungen der Formel (I), in denen A unsubstituiert oder einfach substituiert ist durch Halogen, Cyano, Nitro, Methyl, Hydroxy, Oxo oder Methoxy.

Die oben aufgeführten allgemeinen oder in Vorzugsbereichen aufgeführten Restedefinitionen bzw. Erläuterungen können untereinander, also auch zwischen den jeweiligen Bereichen und Vorzugsbereichen beliebig kombiniert werden. Sie gelten für die Endprodukte sowie für die Vor-und Zwischenprodukte entsprechend.

Gesättigte oder ungesättigte Kohlenwasserstoffreste wie Alkyl oder Alkenyl können, auch in Verbindung mit Heteroatomen, wie z.B. in Alkoxy, soweit möglich, jeweils geradkettig oder verzweigt sein.

Gegebenenfalls substituierte Reste können, sofern nicht anderes angegeben ist, einfach oder mehrfach substituierte sein, wobei bei Mehrfachsubstitutionen die Substituenten gleich oder verschieden sein können.

Die vorstehend als gegebenenfalls substituiert bezeichneten Reste einschließlich Gruppen und Ringe bzw. Cyclen können unsubstituiert oder substituiert sein, insbesondere mit denselben Substituenten, wie sie für die entsprechenden Reste in den nachfolgend beschriebenen weiteren Ausführungsformen der vorliegenden Erfindung definiert sind. Hierbei können die Substituenten einer Ausführungsform mit denen einer anderen Ausführungsform beliebig kombiniert werden.

Erfindungsgemäße Heterocyclyl-Pyrimidine der Formel (I) sowie deren agrochemisch wirksame Salze eignen sich sehr gut als Schädlingsbekämpfungsmittel, insbesondere zur Bekämpfung von tierischen Schädlingen wie Insekten, Parasiten der Unterklasse der Acari (Acarina) (wie Milben, Spinnmilben und/oder Zecken) und/oder Nematoden. Sie eignen sich auch zur Bekämpfung pflanzenpathogener Schadpilze. Die vorgenannten erfindungsgemäßen Verbindungen zeigen vor allem eine starke insektizide und/oder akarizide und/oder nematizide und/oder fungizide Wirksamkeit und lassen sich sowohl im Pflanzenschutz, im Bereich Haushalt und Hygiene als auch im Materialschutz verwenden.

Die Verbindungen der Formel (I) können sowohl in reiner Form als auch als Mischungen verschiedener möglicher isomerer Formen, insbesondere von Stereoisomeren, wie E- und Z-, threo- und erythro-, sowie optischen Isomeren, wie R- und S-Isomeren oder Atropisomeren, gegebenenfalls aber auch von Tautomeren vorliegen. Die Erfindung umfasst sowohl die reinen Isomeren als auch deren Gemische.

Die Verbindungen der Formel (I) können gegebenenfalls in verschiedenen polymorphen Formen oder als Mischung verschiedener polymorpher Formen vorliegen. Sowohl die reinen Polymorphe als auch die Polymorphgemische sind Gegenstand der Erfindung und können erfindungsgemäß verwendet werden.

Je nach Art der oben definierten Substituenten weisen die Verbindungen der Formel (I) saure oder basische Eigenschaften auf und können Salze, gegebenenfalls auch innere Salze bilden. Tragen die Verbindungen der Formel (I) Hydroxy, Carboxy oder andere, saure Eigenschaften induzierende Gruppen, so können diese Verbindungen mit Basen zu Salzen umgesetzt werden. Geeignete Basen sind beispielsweise Hydroxide, Carbonate, Hydrogencarbonate der Alkali- und Erdalkalimetalle, insbesondere die von Natrium, Kalium, Magnesium und Calcium, weiterhin Ammoniak, primäre, sekundäre und tertiäre Amine mit (C₁-C₄-)-Alkylresten, Mono-, Di- und Trialkanolamine von (C₁-C₄)-Alkanolen, Cholin sowie Chlorcholin. Tragen die Verbindungen der Formel (I) Amino, Alkylamino oder andere, basische Eigenschaften induzierende Gruppen, so können diese Verbindungen mit Säuren zu Salzen umgesetzt werden. Geeignete Säuren sind beispielsweise Mineralsäuren, wie Salz-, Schwefel- und Phosphorsäure, organische Säuren, wie Essigsäure oder Oxalsäure, und saure Salze, wie NaHSO₄ und KHSO₄. Die so erhältlichen Salze weisen ebenfalls insektizide und/oder fungizide Eigenschaften auf.

Je nach Art der oben definierten Substituenten weisen die Verbindungen der Formel (X) saure oder basische Eigenschaften auf und können Salze, gegebenenfalls auch innere Salze bilden. Tragen die Verbindungen der Formel (X) Hydroxy, Carboxy oder andere, saure Eigenschaften induzierende Gruppen, so können diese Verbindungen mit Basen zu Salzen umgesetzt werden. Geeignete Basen sind beispielsweise Hydroxide, Carbonate, Hydrogencarbonate der Alkali- und Erdalkalimetalle, insbesondere die von Natrium, Kalium, Magnesium und Calcium, weiterhin Ammoniak, primäre, sekundäre und tertiäre Amine mit (C₁-C₄-)-Alkylresten, Mono-, Di- und Trialkanolamine von (C₁-C₄)-Alkanolen, Cholin sowie Chlorcholin. Tragen die Verbindungen der Formel (X) Amino, Alkylamino oder andere, basische Eigenschaften induzierende Gruppen, so können diese Verbindungen mit Säuren zu Salzen umgesetzt werden. Geeignete Säuren sind beispielsweise Mineralsäuren, wie Salz-, Schwefel- und Phosphorsäure, organische Säuren, wie Essigsäure oder Oxalsäure, und saure Salze, wie NaHSO₄ und KHSO₄. Die so erhältlichen Salze weisen ebenfalls insektizide und/oder fungizide Eigenschaften auf.

Die nachfolgenden Begriffe sind in dieser Anmeldung wie folgt definiert:
Gegebenenfalls substituierte Gruppen können einfach oder mehrfach substituiert sein, wobei bei Mehrfachsubstitutionen die Substituenten gleich oder verschieden sein können.
"Alkyl" ist definiert als verzweigtes oder unverzweigtes C₁₋₁₀ Alkyl, wie zum Beispiel Methyl, Ethyl, n- oder iso-Propyl, n-, iso-, sec- oder tert-Butyl, n-Pentyl, n-Hexyl, n-Heptyl, n-Octyl, n-Nonyl, n-Decyl und ähnliche, bevorzugt als C₁₋₈ Alkyl, besonders bevorzugt als C₁₋₄ Alkyl.
"Alkenyl" ist definiert als verzweigtes oder unverzweigtes C₃₋₁₀ Alkenyl enthaltend mindestens eine Doppelbindung, zum Beispiel Vinyl, Allyl, 1-Propenyl, Isopropenyl, 1-Butenyl, 2-Butenyl, 3-Butenyl, 1,3-Butanedienyl, 1-Pentenyl, 2-Pentenyl, 3-Pentenyl, 4-Pentenyl, 1,3 Pentanedienyl, 1-Hexenyl, 2-Hexenyl, 3-Hexenyl, 4-Hexenyl, 5-Hexenyl, 1,4-Hexanedienyl und ähnliche, bevorzugt als C₃₋₈ Alkenyl, besonders bevorzugt als C₃₋₆ Alkenyl.
"Alkinyl" ist definiert als C₃₋₁₀ Alkinyl enthaltend mindestens eine Dreifachbindung und gegebenenfalls eine oder mehrere Doppelbindungen wie zum Beispiel Ethinyl, 1-Propinyl, Propargyl und ähnliche, bevorzugt als C₃₋₈ Alkinyl, besonders bevorzugt als C₃₋₆ Alkinyl.
Jeder Alkylanteil in "Alkoxy", "Halogenalkyl", "Alkoxyalkyl", "Alkylaminocarbonyl", "Dialkylaminoalkyl", "Halogenalkoxy", "Alkoxycarbonyl", "Alkoxycarbonyl" und "Alkoxycarbonylalkyl" und ähnliche, beziehen auf die oben angeführte Beschreibung von "Alkyl" inklusive der genannten Beispiele.
"Cycloalkyl" ist definiert als C₃₋₁₀ Cycloalkyl, wie zum Beispiel Cyclopropyl, Cyclobutyl, Cyclopentyl, Cyclohexyl, Cycloheptyl, Cyclooctyl und ähnliche, bevorzugt als C₃₋₈ Cycloalkyl.
"Heterocycloalkyl" ist definiert als Cycloalkyl, enthaltend einen 3-10 gliedrigen Ring, wobei der Ring ein, zwei oder mehrere Heteroatome, wie Stickstoff, Sauerstoff und/oder Schwefel, enthält, wie zum Beispiel Tetrahydrofuran, Thiolan, Pyrrolidin, Oxathiolan, Oxazolidin, Tetrahydropurane, Piperidin, Dioxan, und ähnliche, bevorzugt ist Tetrahydrofuran.
"Aryl" ist definiert als ein ungesättigtes C₅-C₁₂ Cycloalkyl, wie zum Beispiel Phenyl, α- oder β-Naphthyl, bevorzugt Phenyl.
"Heteroaryl" ist definiert als ein Aryl, enthaltend einen 3-10 gliedrigen Ring, wobei der Ring ein, zwei oder mehrere Heteroatome, wie Stickstoff, Sauerstoff und/oder Schwefel, enthält, wie zum Beispiel Furan, Thiophen, Pyrrol, Oxazol, Thiazol, Pyrazol, Pyridin, Pyrimidin, Pyrazol und ähnliche.
"Arylalkyl" ist zum Beispiel definiert als Benzyl, Phenethyl or α-Methylbenzyl, bevorzugt ist Benzyl.
"Halogen" ist definiert als Fluor, Chlor, Brom oder Jod, bevorzugt sind Fluor oder Chlor.

Die Verbindungen der Formel (I) können auf verschiedenen Wegen erhalten werden.

Die vorliegende Erfindung betrifft daher auch ein Verfahren zur Herstellung von Heterocyclyl-Pyrimidinen der allgemeinen Formel (I) wie oben definiert **dadurch gekennzeichnet, dass** man in Verfahren (1)
(a) Verbindungen der Formel (IIa) 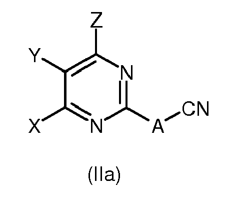 wobei X, Y, Z, A wie oben definiert sind, in Gegenwart von Säure oder Base hydrolysiert und
(b) die so erhaltenen Verbindungen der Formel (III) 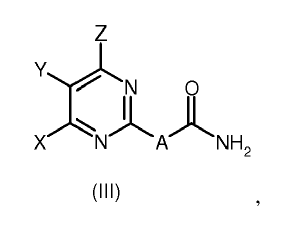 wobei X, Y, Z, A wie oben definiert sind,
   mit Verbindungen der Formel (IVa)

   LG-R⁵ (IVa),

   wobei R⁵ wie oben definiert ist und LG für eine nucleophil abspaltbare Abgangsgruppe steht, gegebenenfalls in Gegenwart einer Base umsetzt und
(c) die so erhaltenen Verbindungen der Formel (Ia) 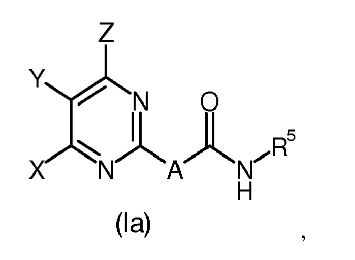 wobei X, Y, Z, A, und R⁵ wie oben definiert sind, gegebenenfalls mit Verbindungen der Formel (IVb)

   LG-R⁴ (IVb),

   wobei R⁴ wie oben definiert, aber nicht Wasserstoff ist und LG für eine nucleophil abspaltbare Abgangsgruppe steht, gegebenenfalls in Gegenwart einer Base umsetzt, wobei die Schritte (b) und (c) in ihrer Reihenfolge vertauscht werden können;
   oder dass man in Verfahren (2)
(d) Verbindungen der Formel (IIb) 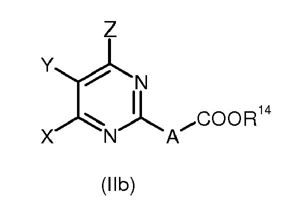 wobei X, Y, Z, A wie oben definiert sind und R¹⁴ für Wasserstoff oder Alkyl steht,
   im Falle, dass R¹⁴ Alkyl ist, in Gegenwart von Säure oder Base verseift, und
(e) die so erhaltenen Verbindungen der Formel (V) 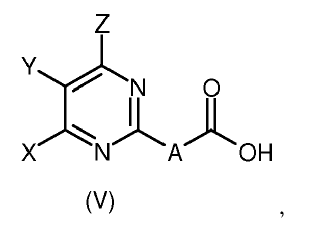 wobei X, Y, Z, A wie oben definiert sind, mit Verbindungen der Formel (VI) 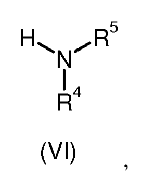 wobei R⁴ und R⁵ wie oben definiert sind, umsetzt. Methoden zur Bildung von Amidbindungen sind literaturbekannt (z. B. METHODS OF ORGANIC CHEMISTRY (Houben Weyl): 'Synthesis of Peptides and Peptidomimetics', Volume E22a, Goodman et al. (Eds.), Georg Thieme Verlag Stuttgart, 2002.)
   Die Verbindungen der Formel (Ia) können mit Basen, die M¹ als Kation enthalten, zu Verbindungen der Formel (I) umgesetzt werden, wobei X, Y, Z, A, R⁵ und M¹ wie oben definiert sind und W für 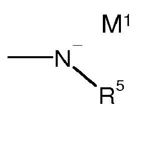 steht.
   Die Verbindungen der Formel (I), wobei X, Y, Z, A, R⁵ wie oben definiert sind und R⁴ Wasserstoff ist, können mit Basen, die M¹ als Kation enthalten, zu Verbindungen der Formel (I) umgesetzt werden, wobei X, Y, Z, A, R⁵ und M¹ wie oben definiert sind und W für 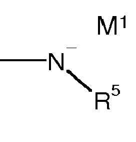 steht.
   Die Verbindungen der Formel (V) sowie deren agrochemisch wirksame Salze eignen sich sehr gut als Schädlingsbekämpfungsmittel, insbesondere zur Bekämpfung von tierischen Schädlingen wie Insekten, Parasiten der Unterklasse der Acari (Acarina) (wie Milben, Spinnmilben und/oder Zecken) und/oder Nematoden.
   Die vorliegende Erfindung betrifft auch ein Verfahren zur Herstellung von Verbindungen der allgemeinen Formel (IIa) sowie (IIb) wie oben definiert **dadurch gekennzeichnet,**
   **dass** man in Verfahren (3)
(f) Verbindungen der Formel (VII) 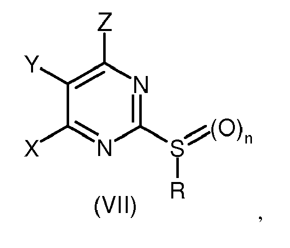 wobei X, Y, Z wie oben definiert sind, n aus 1 oder 2 ausgewählt und R Alkyl ist,
   mit Heterocyclen der Formel (VIII)

   H-A¹-E (VIII),

   wobei E für Cyano oder COOR¹⁴ und A¹ für einen über Stickstoff an den Wasserstoff gebundenen, drei- bis zehngliedrigen gesättigten, partiell ungesättigten oder aromatischen mono- oder bicyclischen Heterocyclus steht, enthaltend ein bis vier Heteroatome aus der Gruppe O, N oder S,
   wobei A¹ durch eine bis drei gleiche oder verschiedene Gruppen R^{d} wie oben definiert, substituiert sein kann, gegebenenfalls in Gegenwart einer Base zu Verbindungen der Formel (IIc) 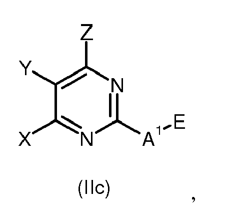 wobei X, Y, Z wie oben definiert sind, E für Cyano oder COOR¹⁴ und R¹⁴ für Wasserstoff oder Alkyl steht,
   umsetzt;
   oder dass man in Verfahren (4)
(g) Verbindungen der Formel (IXa) 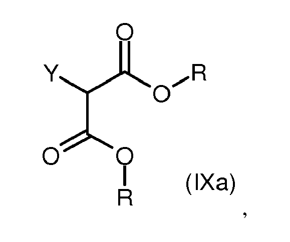 wobei Y wie oben definiert ist und R Alkyl ist,
   mit Heterocyclen der Formel (X) 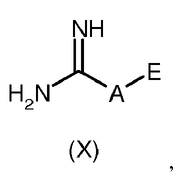 wobei A wie oben definiert ist und E für Cyano oder COOR¹⁴ und R¹⁴ für Wasserstoff oder Alkyl steht, umsetzt, und
(h) die so erhaltenen Verbindungen der Formel (XIa) 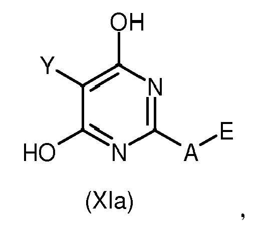 wobei Y und A wie oben definiert sind und E für Cyano oder COOR¹⁴ und R¹⁴ für Wasserstoff oder Alkyl steht, halogeniert und
(i) die so erhaltenen Verbindungen der Formel (XIIa) 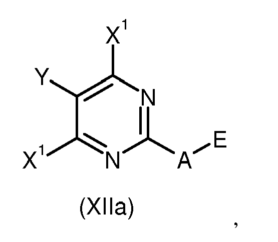 wobei Y und A wie oben definiert sind, E für Cyano oder COOR¹⁴ und R¹⁴ für Wasserstoff oder Alkyl und X¹ für Halogen steht,
   mit Verbindungen der Formel (XIII) 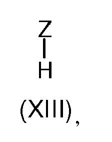 wobei Z wie oben definiert ist, gegebenenfalls in Gegenwart einer Base zu Verbindungen der Formel (IId) 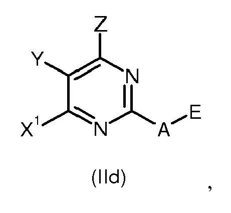 wobei Y, Z, A wie oben definiert sind, E für Cyano oder COOR¹⁴, R¹⁴ für Wasserstoff oder Alkyl und X¹ für Halogen steht, umsetzt;
   oder dass man in Verfahren (5)
(k) Verbindungen der Formel (IXb) 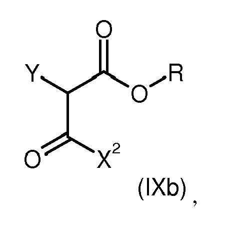 wobei Y wie oben definiert ist, X² für Wasserstoff, gegebenenfalls substituiertes Alkyl oder gegebenenfalls substituiertes Phenyl steht und R Alkyl ist,
   mit Heterocyclen der Formel (X) 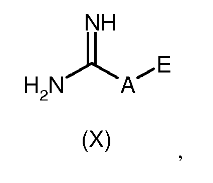 wobei A wie oben definiert ist und E für Cyano oder COOR¹⁴ und R¹⁴ für Wasserstoff oder Alkyl steht, umsetzt, und
(l) die so erhaltenen Verbindungen der Formel (XIb) 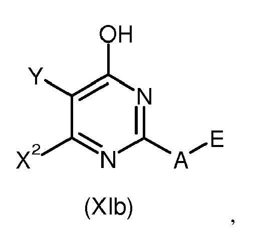 wobei Y und A wie oben definiert sind, E für Cyano oder COOR¹⁴ und R¹⁴ für Wasserstoff oder Alkyl steht und X² für Wasserstoff, gegebenenfalls substituiertes Alkyl oder gegebenenfalls substituiertes Phenyl steht, halogeniert und
(m) die so erhaltenen Verbindungen der Formel (XIIb) 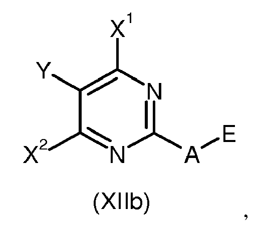 wobei A und Y wie oben definiert sind, E für Cyano oder COOR¹⁴, X¹ für Halogen und X² für Wasserstoff, gegebenenfalls substituiertes Alkyl oder gegebenenfalls substituiertes Phenyl steht, mit Verbindungen der Formel (XIII) 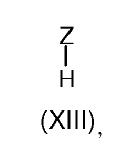 wobei Z wie oben definiert ist, gegebenenfalls in Gegenwart einer Base zu Verbindungen der Formel (IIe) 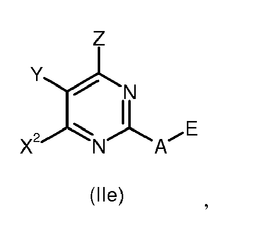 wobei Y, Z, A wie oben definiert sind, X² für Wasserstoff, gegebenenfalls substituiertes Alkyl oder gegebenenfalls substituiertes Phenyl steht, E für Cyano oder COOR¹⁴ und R¹⁴ für Wasserstoff oder Alkyl steht, umsetzt.

Verbindungen der Formel (XIIa), in der X¹ für Fluor oder Iod steht, werden aus den entsprechenden Verbindungen der Formel (XIIa), in der X¹ für Chlor oder Brom steht, durch Umsetzung mit den entsprechenden Alkali- oder Erdalkalimetallhalogeniden erhalten.

Verbindungen der Formel (IIb), in der X für Cyano steht, werden aus den entsprechenden Verbindungen der Formel (IId) wie oben definiert, durch Umsetzung mit den entsprechenden Alkali- oder Erdalkalimetallcyaniden erhalten.

R steht für Alkyl, bevorzugt für C₁-C₆-Alkyl. R¹⁴ steht für Wasserstoff und Alkyl, bevorzugt für Wasserstoff und C₁-C₆-Alkyl. X¹ steht für Halogen, bevorzugt für Cl und Br.

Die Substituenten X, X¹, X², Y, Z, A, E, W, R¹, R², R⁴ und R⁵ auf die in den Formeln (Ia), (IIa), (IIb), (IIc), (IId), (IIe), (III), (IVa), (IVb), (V), (VI), (VII), (XIII), (IXa), (IXb), (X), (XIa), (XIb), (XIIa), (XIIb) und (XIII) Bezug genommen wird, haben vorzugsweise diejenigen Bedeutungen, die bereits im Zusammenhang mit der Beschreibung der erfindungsgemäßen Verbindungen der Formel (I) für diese Reste als bevorzugt genannt wurden.

Die Verbindungen der Formel (IVa), (IVb), (VI), (VIII), (IXa), (IXb), (X) und (XIII) sind bekannt. Sie sind kommerziell erhältlich oder lassen sich nach bekannten, dem Fachmann geläufigen Verfahren herstellen, wie sie beispielsweise in Houben-Weyl, Methoden der Organischen Chemie beschrieben sind. Die Verbindungen der Formel (VII) sind teilweise bekannt und lassen sich nach literaturbekannten Verfahren herstellen (z.B. WO 02/074753, WO 04/029204, WO 05/085212, WO 06/029867).

Die erfindungsgemäßen Verfahren zur Herstellung der Verbindungen der Formel (I) werden vorzugsweise unter Verwendung eines oder mehrerer Reaktionshilfsmittel durchgeführt.

Als Reaktionshilfsmittel kommen gegebenenfalls die üblichen anorganischen oder organischen Basen oder Säureakzeptoren in Betracht. Hierzu gehören vorzugsweise Alkalimetall- oder Erdalkalimetall- -acetate, -amide, -carbonate, -hydrogencarbonate, -hydride, -hydroxide oder - alkanolate, wie beispielsweise Natrium-, Kalium- oder Calcium-acetat, Lithium-, Natrium-, Kalium- oder Calcium-amid, Natrium-, Kalium- oder Calcium-carbonat, Natrium-, Kalium- oder Calcium-hydrogencarbonat, Lithium-, Natrium-, Kalium- oder Calcium-hydrid, Lithium-, Natrium-Kalium- oder Calcium-hydroxid, Natrium- oder Kalium- -methanolat, -ethanolat, -n- oder -i-propanolat, -n-, -i-, -s- oder -t-butanolat; weiterhin auch basische organische Stickstoffverbindungen, wie beispielsweise Trimethylamin, Triethylamin, Tripropylamin, Tributylamin, Ethyldiisopropylamin, N,N-Dimethyl-cyclohexylamin, Dicyclohexylamin, Ethyl-dicyclohexylamin, N,N-Dimethyl-anilin, N,N-Dimethyl-benzylamin, Pyridin, 2-Methyl-, 3-Methyl-, 4-Methyl-, 2,4-Dimethyl-, 2,6-Dimethyl-, 3,4-Dimethyl- und 3,5-Dimethyl-pyridin, 5-Ethyl-2-methyl-pyridin, 4-Dimethylamino-pyridin, N-Methyl-piperidin, 1,4-Diazabicyclo[2,2,2]-octan (DABCO), 1,5-Diazabicyclo[4,3,0]-non-5-en (DBN), oder 1,8 Diazabicyclo[5,4,0]-undec-7-en (DBU).

Die erfindungsgemäßen Verfahren werden vorzugsweise unter Verwendung eines oder mehrerer Verdünnungsmittel durchgeführt. Als Verdünnungsmittel kommen praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methyl-isopropyl- oder Methyl-isobutyl-keton, Ester wie Essigsäuremethylester oder -ethylester, Nitrile wie z.B. Acetonitril oder Propionitril, Amide wie z.B. Dimethylformamid, Dimethylacetamid und N-Methyl-pyrrolidon, sowie Dimethylsulfoxid, Tetramethylensulfon und Hexamethylphosphorsäuretriamid.

Die Reaktionstemperaturen können bei den erfindungsgemäßen Verfahren in einem größeren Bereich variiert werden. Im Allgemeinen arbeitet man bei Temperaturen zwischen 0°C und 250°C, vorzugsweise bei Temperaturen zwischen 10°C und 185°C.

Die erfindungsgemäßen Verfahren werden im Allgemeinen unter Normaldruck durchgeführt. Es ist jedoch auch möglich, unter erhöhtem oder vermindertem Druck zu arbeiten.

Zur Durchführung der erfindungsgemäßen Verfahren werden die jeweils benötigten Ausgangsstoffe im Allgemeinen in angenähert äquimolaren Mengen eingesetzt. Es ist jedoch auch möglich, eine der jeweils eingesetzten Komponenten in einem größeren Überschuss zu verwenden. Die Aufarbeitung erfolgt bei den erfindungsgemäßen Verfahren jeweils nach üblichen Methoden (vgl. die Herstellungsbeispiele).

In der Literatur wurde bereits beschrieben, dass sich die Wirkung verschiedener Wirkstoffe durch Zugabe von Ammoniumsalzen steigern lässt. Dabei handelt es sich jedoch um als Detergens wirkende Salze (z.B. WO 95/017817) bzw. Salze mit längeren Alkyl- und / oder Arylsubstituenten, die permeabilisierend wirken oder die Löslichkeit des Wirkstoffs erhöhen (z.B. EP-A 0 453 086, EP-A 0 664 081, FR-A 2 600 494, US 4 844 734, US 5 462 912, US 5 538 937, US-A 03/0224939, US-A 05/0009880, US-A 05/0096386). Weiterhin beschreibt der Stand der Technik die Wirkung nur für bestimmte Wirkstoffe und / oder bestimmte Anwendungen der entsprechenden Mittel. In wieder anderen Fällen handelt es sich um Salze von Sulfonsäuren, bei denen die Säuren selber paralysierend auf Insekten wirken (US 2 842 476). Eine Wirkungssteigerung z.B. durch Ammoniumsulfat ist beispielsweise für die Herbizide Glyphosat und Phosphinothricin beschrieben (US 6 645 914, EP-A2 0 036 106). Eine entsprechende Wirkung bei Insektiziden wird durch diesen Stand der Technik weder offenbart noch nahegelegt.

Auch der Einsatz von Ammoniumsulfat als Formulierhilfsmittel ist für bestimmte Wirkstoffe und Anwendungen beschrieben (WO 92/16108), es dient dort aber zur Stabilisierung der Formulierung, nicht zur Wirkungssteigerung.

Es wurde nun völlig überraschend gefunden, dass sich die Wirkung von Insektiziden und/oder Akariziden aus der Klasse der Heterocyclyl-Pyrimidine der Formel (I) durch den Zusatz von Ammonium- oder Phosphoniumsalzen zur Anwendungslösung oder durch den Einbau dieser Salze in eine Formulierung enthaltend Heterocyclyl-Pyrimidine der Formel (I) deutlich steigern lässt. Gegenstand der vorliegenden Erfindung ist also die Verwendung von Ammonium- oder Phosphoniumsalzen zur Wirkungssteigerung von Pflanzenschutzmitteln, die insektizid und/oder akarizid wirksame Heterocyclyl-Pyrimidine der Formel (I) als Wirkstoff enthalten. Gegenstand der Erfindung sind ebenfalls Mittel, die insektizid und/oder akarizid wirksame Heterocyclyl-Pyrimidine der Formel (I) und die Wirkung steigernde Ammonium- oder Phosphoniumsalze enthalten und zwar sowohl formulierte Wirkstoffe als auch anwendungsfertige Mittel (Spritzbrühen). Gegenstand der Erfindung ist schließlich weiterhin die Verwendung dieser Mittel zur Bekämpfung von Schadinsekten und/oder Spinnmilben.

Ammonium- und Phosphoniumsalze, die erfindungsgemäß die Wirkung von Pflanzenschutzmitteln enthaltend Heterocyclyl-Pyrimidine der Formel (I) steigern, werden durch Formel (XV) definiert 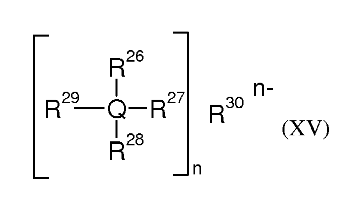 in welcher
- Q: für Stickstoff oder Phosphor steht,
- Q: bevorzugt für Stickstoff steht,

R²⁶, R²⁷, R²⁸ und R²⁹ unabhängig voneinander für Wasserstoff oder jeweils gegebenenfalls substituiertes C₁-C₈-Alkyl oder einfach oder mehrfach ungesättigtes, gegebenenfalls substituiertes C₁-C₈-Alkylen stehen, wobei die Substituenten aus Halogen, Nitro und Cyano ausgewählt sein können,
R²⁶, R²⁷, R²⁸ und R²⁹ bevorzugt unabhängig voneinander für Wasserstoff oder jeweils gegebenenfalls substituiertes C₁-C₄-Alkyl stehen, wobei die Substituenten aus Halogen, Nitro und Cyano ausgewählt sein können,
R²⁶, R²⁷, R²⁸ und R²⁹ besonders bevorzugt unabhängig voneinander für Wasserstoff, Methyl, Ethyl, n-Propyl, i-Propyl, n-Butyl, i-Butyl, s-Butyl oder t-Butyl stehen,
R²⁶, R²⁷, R²⁸ und R²⁹ ganz besonders bevorzugt für Wasserstoff stehen,
R²⁶, R²⁷, R²⁸ und R²⁹ weiterhin ganz besonders bevorzugt gleichzeitig für Methyl oder gleichzeitig für Ethyl stehen,

- n: für 1, 2, 3 oder 4 steht,
- n: bevorzugt für 1 oder 2 steht,
- R³⁰: für ein anorganisches oder organisches Anion steht,
- R³⁰: bevorzugt für Hydrogencarbonat, Tetraborat, Fluorid, Bromid, Jodid, Chlorid, Monohydrogenphosphat, Dihydrogenphosphat, Hydrogensulfat, Tartrat, Sulfat, Nitrat, Thiosulfat, Thiocyanat, Formiat, Laktat, Acetat, Propionat, Butyrat, Pentanoat, Citrat oder Oxalat steht,
- R³⁰: weiterhin bevorzugt für Carbonat, Pentaborat, Sulfit, Benzoat, Hydrogenoxalat, Hydrogencitrat, Methylsulfat oder Tetrafluoroborat steht,
- R³⁰: besonders bevorzugt für Laktat, Sulfat, Nitrat, Thiosulfat, Thiocyanat, Citrat, Oxalat, Acetat oder Formiat steht,
- R³⁰: außerdem besonders bevorzugt für Monohydrogenphosphat oder Dihydrogenphosphat steht und
- R³⁰: ganz besonders bevorzugt für Thiocyanat, Dihydrogenphosphat, Monohydrogenphosphat oder Sulfat steht.

Die Ammonium- und Phosphoniumsalze der Formel (XV) können in einem breiten Konzentrationsbereich zur Steigerung der Wirkung von Pflanzenschutzmitteln enthaltend Heterocyclyl-Pyrimidine der Formel (I) eingesetzt werden. Im Allgemeinen werden die Ammonium- oder Phosphoniumsalze im anwendungsfertigen Pflanzenschutzmittel in einer Konzentration von 0,5 bis 80 mmol/l, bevorzugt 0,75 bis 37,5 mmol/l, besonders bevorzugt 1,5 bis 25 mmol/l eingesetzt. Im Fall eines formulierten Produktes wird die Ammonium- und/oder Phosphoniumsalzkonzentration in der Formulierung so gewählt, dass sie nach Verdünnung der Formulierung auf die gewünschte Wirkstoffkonzentration in diesen angegebenen allgemeinen, bevorzugten oder besonders bevorzugten Bereichen liegt. Die Konzentration des Salzes in der Formulierung beträgt dabei üblicherweise 1 - 50 Gew.-%. Die Konzentration des Wirkstoffs in der Formulierung beträgt dabei üblicherweise 0,01 - 50 Gew.-%.

In einer bevorzugten Ausführungsform der Erfindung wird den Pflanzenschutzmitteln zur Wirkungssteigerung nicht nur ein Ammonium- und/oder Phosphoniumsalz, sondern zusätzlich ein Penetrationsförderer zugegeben. Es ist als völlig überraschend zu bezeichnen, dass selbst in diesen Fällen eine noch weiter gehende Wirkungssteigerung zu beobachten ist. Gegenstand der vorliegenden Erfindung ist also ebenfalls die Verwendung einer Kombination von Penetrationsförderer und Ammonium- und/oder Phosphoniumsalzen zur Wirkungssteigerung von Pflanzenschutzmitteln, die insektizid und/oder akarizid wirksame Heterocyclyl-Pyrimidine der Formel (I) als Wirkstoff enthalten. Gegenstand der Erfindung sind ebenfalls Mittel, die insektizid und/oder akarizid wirksame Heterocyclyl-Pyrimidine der Formel (I), Penetrationsförderer und Ammonium- und/oder Phosphoniumsalze enthalten und zwar sowohl formulierte Wirkstoffe als auch anwendungsfertige Mittel (Spritzbrühen). Gegenstand der Erfindung ist schließlich weiterhin die Verwendung dieser Mittel zur Bekämpfung von Schadinsekten.

Als Penetrationsförderer kommen im vorliegenden Zusammenhang alle diejenigen Substanzen in Betracht, die üblicherweise eingesetzt werden, um das Eindringen von agrochemischen Wirkstoffen in Pflanzen zu verbessern. Penetrationsförderer werden in diesem Zusammenhang dadurch definiert, dass sie aus der wässerigen Spritzbrühe und/oder aus dem Spritzbelag in die Kutikula der Pflanze eindringen und dadurch die Stoffbeweglichkeit (Mobilität) von Wirkstoffen in der Kutikula erhöhen können. Die in der Literatur (Baur et al., 1997, Pesticide Science 51, 131-152) beschriebene Methode kann zur Bestimmung dieser Eigenschaft eingesetzt werden.

Als Penetrationsförderer kommen beispielsweise Alkanol-alkoxylate in Betracht. Erfindungsgemäße Penetrationsförderer sind Alkanol-alkoxylate der Formel

R-O-(-AO)_{V}-R' (XVI)

in welcher
- R: für geradkettiges oder verzweigtes Alkyl mit 4 bis 20 Kohlenstoffatomen steht,
- R^{'}: für Wasserstoff, Methyl, Ethyl, n-Propyl, i-Propyl, n-Butyl, i-Butyl, t-Butyl, n-Pentyl oder n-Hexyl steht,
- AO: für einen Ethylenoxid-Rest, einen Propylenoxid-Rest, einen Butylenoxid-Rest oder für Gemische aus Ethylenoxid- und Propylenoxid-Resten oder Butylenoxid-Resten steht und
- V: für Zahlen von 2 bis 30 steht.

Eine bevorzugte Gruppe von Penetrationsförderern sind Alkanolalkoxylate der Formel

R-O-(-EO-)ₙ-R' (XVI-a)

in welcher
- R: die oben angegebene Bedeutung hat,
- R': die oben angegebene Bedeutung hat,
- EO: für -CH₂-CH₂-O- steht und
- n: für Zahlen von 2 bis 20 steht.

Eine weitere bevorzugte Gruppe von Penetrationsförderern sind Alkanol-alkoxylate der Formel

R-O-(-EO-)ₚ-(-PO-)_{q}-R' (XVI-b)

in welcher
- R: die oben angegebene Bedeutung hat,
- R': die oben angegebene Bedeutung hat,
- EO: für -CH₂-CH₂-O- steht,
- PO: für 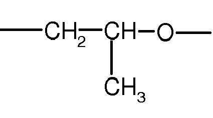 steht,
- p: für Zahlen von 1 bis 10 steht und
- q: für Zahlen von 1 bis 10 steht.

Eine weitere bevorzugte Gruppe von Penetrationsförderern sind Alkanol-Alkoxylate der Formel

R-O-(-PO-)ᵣ-(EO-)ₛ-R' (XVI-c)

in welcher
- R: die oben angegebene Bedeutung hat,
- R': die oben angegebene Bedeutung hat,
- EO: für -CH₂-CH₂-O- steht,
- PO: für 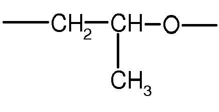
steht,
- r: für Zahlen von 1 bis 10 steht und
- s: für Zahlen von 1 bis 10 steht.

Eine weitere bevorzugte Gruppe von Penetrationsförderern sind Alkanol-alkoxylate der Formel

R-O-(-EO-)ₚ-(-BO-)_{q}-R' (XVI-d)

in welcher
- R und: R^{'} die oben angegebenen Bedeutungen haben,
- EO: für CH₂-CH₂-O- steht,

- BO: für 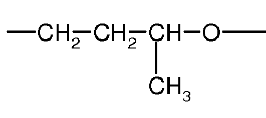
steht,
- p: für Zahlen von 1 bis 10 steht und
- q: für Zahlen von 1 bis 10 steht.

Eine weitere bevorzugte Gruppe von Penetrationsförderern sind Alkanol-alkoxylate der Formel

R-O-(-BO-)ᵣ-(-EO-)ₛ-R' (XVI-e)

in welcher
- R und R': die oben angegebenen Bedeutungen haben,

- BO: für 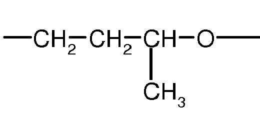
steht,
- EO: für CH₂-CH₂-O- steht,
- r: für Zahlen von 1 bis 10 steht und
- s: für Zahlen von 1 bis 10 steht.

Eine weitere bevorzugte Gruppe von Penetrationsförderern sind Alkanol-Alkoxylate der Formel

CH₃-(CH₂)ₜ-CH₂-O-(-CH₂-CH₂-O-)ᵤ-R' (XVI-f)

in welcher
- R': die oben angegebene Bedeutung hat,
- t: für Zahlen von 8 bis 13 steht
- u: für Zahlen von 6 bis 17 steht.
In den zuvor angegebenen Formeln steht

Rvorzugsweise für Butyl, i-Butyl, n-Pentyl, i-Pentyl, Neopentyl, n-Hexyl, i-Hexyl, n-Octyl, i-Octyl, 2-Ethyl-hexyl, Nonyl, i-Nonyl, Decyl, n-Dodecyl, i-Dodecyl, Lauryl, Myristyl, i-Tridecyl, Trimethyl-nonyl, Palmityl, Stearyl oder Eicosyl.

Als Beispiel für ein Alkanol-Alkoxylat der Formel (XVI-c) sei 2-Ethyl-hexyl-alkoxylat der Formel 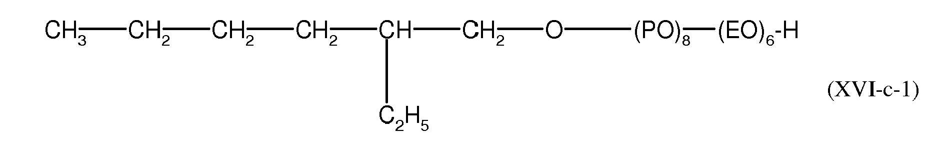 in welcher
- EO: für -CH₂-CH₂-O- steht,

- PO: für 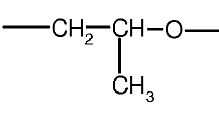 steht und
die Zahlen 8 und 6 Durchschnittswerte darstellen, genannt.

Als Beispiel für ein Alkanol-Alkoxylat der Formel (III-d) sei die Formel

CH₃-(CH₂)₁₀-O-(-EO-)₆-(-BO-)₂-CH₃ (XVI-d-1)

in welcher
- EO: für CH₂-CH₂-O- steht,
- BO: für 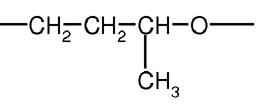 steht und
die Zahlen 10, 6 und 2 Durchschnittswerte darstellen, genannt.

Besonders bevorzugte Alkanol-Alkoxylate der Formel (XVI-f) sind Verbindungen dieser Formel, in denen
- t: für Zahlen von 9 bis 12 und
- u: für Zahlen von 7 bis 9
steht.

Ganz besonders bevorzugt genannt sei Alkanol-Alkoxylat der Formel (XVI-f-1)

CH₃-(CH₂)ₜ-CH₂-O-(-CH₂-CH₂-O-)ᵤ-H (XVI-f-1)

in welcher
- t: für den Durchschnittswert 10,5 steht und
- u: für den Durchschnittswert 8,4 steht.

Die Alkanol-Alkoxylate sind durch die obigen Formeln allgemein definiert. Bei diesen Substanzen handelt es sich um Gemische von Stoffen des angegebenen Typs mit unterschiedlichen Kettenlängen. Für die Indices errechnen sich deshalb Durchschnittswerte, die auch von ganzen Zahlen abweichen können.

Die Alkanol-Alkoxylate der angegebenen Formeln sind bekannt und sind teilweise kommerziell erhältlich oder lassen sich nach bekannten Methoden herstellen (vgl. WO 98/35 553, WO 00/35 278 und EP-A 0 681 865).

Als Penetrationsförderer kommen beispielsweise auch Substanzen in Betracht, die die Verfügbarkeit der Verbindungen der Formel (I) im Spritzbelag fördern. Dazu gehören beispielsweise mineralische oder vegetabile Öle. Als Öle kommen alle üblicherweise in agrochemischen Mitteln einsetzbaren mineralischen oder vegetabilen - gegebenenfalls modifizierte - Öle in Frage. Beispielhaft genannt seien Sonnenblumenöl, Rapsöl, Olivenöl, Rizinusöl, Rüböl, Maiskernöl, Baumwollsaatöl und Sojabohnenöl oder die Ester der genannten Öle. Bevorzugt sind Rapsöl, Sonnenblumenöl und deren Methyl- oder Ethylester.

Die Konzentration an Penetrationsförderer kann in den erfindungsgemäßen Mitteln in einem weiten Bereich variiert werden. Bei einem formulierten Pflanzenschutzmittel liegt sie im allgemeinen bei 1 bis 95 Gew.-%, bevorzugt bei 1 bis 55 Gew.-%, besonders bevorzugt bei 15 - 40 Gew.-%. In den anwendungsfertigen Mitteln (Spritzbrühen) liegen die Konzentration im Allgemeinen zwischen 0,1 und 10 g/l, bevorzugt zwischen 0,5 und 5 g/l.

Erfindungsgemäße Pflanzenschutzmittel können auch weitere Komponenten, beispielsweise Tenside bzw. Dispergierhilfsmittel oder Emulgatoren enthalten.

Als nicht-ionische Tenside bzw. Dispergierhilfsmittel kommen alle üblicherweise in agrochemischen Mitteln einsetzbaren Stoffe dieses Typs in Betracht. Vorzugsweise genannt seien Polyethylenoxid-polypropylenoxid-Blockcopolymere, Polyethylenglykolether von linearen Alkoholen, Umsetzungsprodukte von Fettsäuren mit Ethylenoxid und/oder Propylenoxid, ferner Polyvinylalkohol, Polyvinylpyrrolidon, Mischpolymerisate aus Polyvinylalkohol und Polyvinylpyrrolidon sowie Copolymerisate aus (Meth)acrylsäure und (Meth)acrylsäureestern, weiterhin Alkylethoxylate und Alkylarylethoxylate, die gegebenenfalls phosphatiert und gegebenenfalls mit Basen neutralisiert sein können, wobei Sorbitolethoxylate beispielhaft genannt seien, sowie Polyoxyalkylenamin-Derivate.

Als anionische Tenside kommen alle üblicherweise in agrochemischen Mitteln einsetzbaren Substanzen dieses Typs in Frage. Bevorzugt sind Alkalimetall- und Erdalkalimetall-Salze von Alkylsulfonsäuren oder Alkylarylsulfonsäuren.

Eine weitere bevorzugte Gruppe von anionischen Tensiden bzw. Dispergierhilfsmitteln sind in Pflanzenöl wenig lösliche Salze von Polystyrolsulfonsäuren, Salze von Polyvinylsulfonsäuren, Salze von Naphthalinsulfonsäure-Formaldehyd-Kondensationsprodukten, Salze von Kondensationsprodukten aus Naphthalinsulfonsäure, Phenolsulfonsäure und Formaldehyd sowie Salze von Ligninsulfonsäure.

Als Zusatzstoffe, die in den erfindungsgemäßen Formulierungen enthalten sein können, kommen Emulgatoren, schaumhemmende Mittel, Konservierungsmittel, Antioxydantien, Farbstoffe und inerte Füllmaterialien in Betracht.

Bevorzugte Emulgatoren sind ethoxylierte Nonylphenole, Umsetzungsprodukte von Alkylphenolen mit Ethylenoxid und/oder Propylenoxid, ethoxylierte Arylalkylphenole, weiterhin ethoxylierte und propoxylierte Arylalkylphenole, sowie sulfatierte oder phosphatierte Arylalkylethoxylate bzw. -ethoxy-propoxylate, wobei Sorbitan-Derivate, wie Polyethylenoxid-Sorbitan-Fettsäureester und Sorbitan-Fettsäureester, beispielhaft genannt seien.

Die erfindungsgemäßen Wirkstoffe eignen sich bei guter Pflanzenverträglichkeit, günstiger Warmblütertoxizität und guter Umweltverträglichkeit zum Schutz von Pflanzen und Pflanzenorganen, zur Steigerung der Ernteerträge, Verbesserung der Qualität des Erntegutes und zur Bekämpfung von tierischen Schädlingen, insbesondere Insekten, Spinnentieren, Helminthen, Nematoden und Mollusken, die in der Landwirtschaft, im Gartenbau, bei der Tierzucht, in Forsten, in Gärten und Freizeiteinrichtungen, im Vorrats- und Materialschutz sowie auf dem Hygienesektor vorkommen. Sie können vorzugsweise als Pflanzenschutzmittel eingesetzt werden. Sie sind gegen normal sensible und resistente Arten sowie gegen alle oder einzelne Entwicklungsstadien wirksam. Zu den oben erwähnten Schädlingen gehören:
Aus der Ordnung der Anoplura (Phthiraptera) z.B. Damalinia spp., Haematopinus spp., Linognathus spp., Pediculus spp., Trichodectes spp..
Aus der Klasse der Arachnida z.B. Acarus siro, Aceria sheldoni, Aculops spp., Aculus spp., Amblyomma spp., Argas spp., Boophilus spp., Brevipalpus spp., Bryobia praetiosa, Chorioptes spp., Dermanyssus gallinae, Eotetranychus spp., Epitrimerus pyri, Eutetranychus spp., Eriophyes spp., Hemitarsonemus spp., Hyalomma spp., Ixodes spp., Latrodectus mactans, Metatetranychus spp., Oligonychus spp., Ornithodoros spp., Panonychus spp., Phyllocoptruta oleivora, Polyphagotarsonemus latus, Psoroptes spp., Rhipicephalus spp., Rhizoglyphus spp., Sarcoptes spp., Scorpio maurus, Stenotarsonemus spp., Tarsonemus spp., Tetranychus spp., Vasates lycopersici.
Aus der Klasse der Bivalva z.B. Dreissena spp..
Aus der Ordnung der Chilopoda z.B. Geophilus spp., Scutigera spp..
Aus der Ordnung der Coleoptera z.B. Acanthoscelides obtectus, Adoretus spp., Agelastica alni, Agriotes spp., Amphimallon solstitialis, Anobium punctatum, Anoplophora spp., Anthonomus spp., Anthrenus spp., Apogonia spp., Atomaria spp., Attagenus spp., Bruchidius obtectus, Bruchus spp., Ceuthorhynchus spp., Cleonus mendicus, Conoderus spp., Cosmopolites spp., Costelytra zealandica, Curculio spp., Cryptorhynchus lapathi, Dermestes spp., Diabrotica spp., Epilachna spp., Faustinus cubae, Gibbium psylloides, Heteronychus arator, Hylamorpha elegans, Hylotrupes bajulus, Hypera postica, Hypothenemus spp., Lachnosterna consanguinea, Leptinotarsa decemlineata, Lissorhoptrus oryzophilus, Lixus spp., Lyctus spp., Meligethes aeneus, Melolontha melolontha, Migdolus spp., Monochamus spp., Naupactus xanthographus, Niptus hololeucus, Oryctes rhinoceros, Oryzaephilus surinamensis, Otiorrhynchus sulcatus, Oxycetonia jucunda, Phaedon cochleariae, Phyllophaga spp., Popillia japonica, Premnotrypes spp., Psylliodes chrysocephala, Ptinus spp., Rhizobius ventralis, Rhizopertha dominica, Sitophilus spp., Sphenophorus spp., Sternechus spp., Symphyletes spp., Tenebrio molitor, Tribolium spp., Trogoderma spp., Tychius spp., Xylotrechus spp., Zabrus spp..
Aus der Ordnung der Collembola z.B. Onychiurus armatus.
Aus der Ordnung der Dermaptera z.B. Forficula auricularia.
Aus der Ordnung der Diplopoda z.B. Blaniulus guttulatus.
Aus der Ordnung der Diptera z.B. Aedes spp., Anopheles spp., Bibio hortulanus, Calliphora erythrocephala, Ceratitis capitata, Chrysomyia spp., Cochliomyia spp., Cordylobia anthropophaga, Culex spp., Cuterebra spp., Dacus oleae, Dermatobia hominis, Drosophila spp., Fannia spp., Gastrophilus spp., Hylemyia spp., Hyppobosca spp., Hypoderma spp., Liriomyza spp.. Lucilia spp., Musca spp., Nezara spp., Oestrus spp., Oscinella frit, Pegomyia hyoscyami, Phorbia spp., Stomoxys spp., Tabanus spp., Tannia spp., Tipula paludosa, Wohlfahrtia spp.
Aus der Klasse der Gastropoda z.B. Arion spp., Biomphalaria spp., Bulinus spp., Deroceras spp., Galba spp., Lymnaea spp., Oncomelania spp., Succinea spp..
Aus der Klasse der Helminthen z.B. Ancylostoma duodenale, Ancylostoma ceylanicum, Acylostoma braziliensis, Ancylostoma spp., Ascaris lubricoides, Ascaris spp., Brugia malayi, Brugia timori, Bunostomum spp., Chabertia spp., Clonorchis spp., Cooperia spp., Dicrocoelium spp, Dictyocaulus filaria, Diphyllobothrium latum, Dracunculus medinensis, Echinococcus granulosus, Echinococcus multilocularis, Enterobius vermicularis, Faciola spp., Haemonchus spp., Heterakis spp., Hymenolepis nana, Hyostrongulus spp., Loa Loa, Nematodirus spp., Oesophagostomum spp., Opisthorchis spp., Onchocerca volvulus, Ostertagia spp., Paragonimus spp., Schistosomen spp, Strongyloides fuelleborni, Strongyloides stercoralis, Stronyloides spp., Taenia saginata, Taenia solium, Trichinella spiralis, Trichinella nativa, Trichinella britovi, Trichinella nelsoni, Trichinella pseudopsiralis, Trichostrongulus spp., Trichuris trichuria, Wuchereria bancrofti.

Weiterhin lassen sich Protozoen, wie Eimeria, bekämpfen.

Aus der Ordnung der Heteroptera z.B. Anasa tristis, Antestiopsis spp., Blissus spp., Calocoris spp., Campylomma livida, Cavelerius spp., Cimex spp., Creontiades dilutus, Dasynus piperis, Dichelops furcatus, Diconocoris hewetti, Dysdercus spp., Euschistus spp., Eurygaster spp., Heliopeltis spp., Horcias nobilellus, Leptocorisa spp., Leptoglossus phyllopus, Lygus spp., Macropes excavatus, Miridae, Nezara spp., Oebalus spp., Pentomidae, Piesma quadrata, Piezodorus spp., Psallus seriatus, Pseudacysta persea, Rhodnius spp., Sahlbergella singularis, Scotinophora spp., Stephanitis nashi, Tibraca spp., Triatoma spp.

Aus der Ordnung der Homoptera z.B. Acyrthosipon spp., Aeneolamia spp., Agonoscena spp., Aleurodes spp., Aleurolobus barodensis, Aleurothrixus spp., Amrasca spp., Anuraphis cardui, Aonidiella spp., Aphanostigma piri, Aphis spp., Arboridia apicalis, Aspidiella spp., Aspidiotus spp., Atanus spp., Aulacorthum solani, Bemisia spp., Brachycaudus helichrysii, Brachycolus spp., Brevicoryne brassicae, Calligypona marginata, Carneocephala fulgida, Ceratovacuna lanigera, Cercopidae, Ceroplastes spp., Chaetosiphon fragaefolii, Chionaspis tegalensis, Chlorita onukii, Chromaphis juglandicola, Chrysomphalus ficus, Cicadulina mbila, Coccomytilus halli, Coccus spp., Cryptomyzus ribis, Dalbulus spp., Dialeurodes spp., Diaphorina spp., Diaspis spp., Doralis spp., Drosicha spp., Dysaphis spp., Dysmicoccus spp., Empoasca spp., Eriosoma spp., Erythroneura spp., Euscelis bilobatus, Geococcus coffeae, Homalodisca coagulata, Hyalopterus arundinis, Icerya spp., Idiocerus spp., Idioscopus spp., Laodelphax striatellus, Lecanium spp., Lepidosaphes spp., Lipaphis erysimi, Macrosiphum spp., Mahanarva fimbriolata, Melanaphis sacchari, Metcalfiella spp., Metopolophium dirhodum, Monellia costalis, Monelliopsis pecanis, Myzus spp., Nasonovia ribisnigri, Nephotettix spp., Nilaparvata lugens, Oncometopia spp., Orthezia praelonga, Parabemisia myricae, Paratrioza spp., Parlatoria spp., Pemphigus spp., Peregrinus maidis, Phenacoccus spp., Phloeomyzus passerinii, Phorodon humuli, Phylloxera spp., Pinnaspis aspidistrae, Planococcus spp., Protopulvinaria pyriformis, Pseudaulacaspis pentagona, Pseudococcus spp., Psylla spp., Pteromalus spp., Pyrilla spp., Quadraspidiotus spp., Quesada gigas, Rastrococcus spp., Rhopalosiphum spp., Saissetia spp., Scaphoides titanus, Schizaphis graminum, Selenaspidus articulatus, Sogata spp., Sogatella furcifera, Sogatodes spp., Stictocephala festina, Tenalaphara malayensis, Tinocallis caryaefoliae, Tomaspis spp., Toxoptera spp., Trialeurodes vaporariorum, Trioza spp., Typhlocyba spp., Unaspis spp., Viteus vitifolii.

Aus der Ordnung der Hymenoptera z.B. Diprion spp., Hoplocampa spp., Lasius spp., Monomorium pharaonis, Vespa spp..

Aus der Ordnung der Isopoda z.B. Armadillidium vulgare, Oniscus asellus, Porcellio scaber.

Aus der Ordnung der Isoptera z.B. Reticulitermes spp., Odontotermes spp..

Aus der Ordnung der Lepidoptera z.B. Acronicta major, Aedia leucomelas, Agrotis spp., Alabama argillacea, Anticarsia spp., Barathra brassicae, Bucculatrix thurberiella, Bupalus piniarius, Cacoecia podana, Capua reticulana, Carpocapsa pomonella, Cheimatobia brumata, Chilo spp., Choristoneura fumiferana, Clysia ambiguella, Cnaphalocerus spp., Earias insulana, Ephestia kuehniella, Euproctis chrysorrhoea, Euxoa spp., Feltia spp., Galleria mellonella, Helicoverpa spp., Heliothis spp., Hofmannophila pseudospretella, Homona magnanima, Hyponomeuta padella, Laphygma spp., Lithocolletis blancardella, Lithophane antennata, Loxagrotis albicosta, Lymantria spp., Malacosoma neustria, Mamestra brassicae, Mocis repanda, Mythimna separata, Oria spp., Oulema oryzae, Panolis flammea, Pectinophora gossypiella, Phyllocnistis citrella, Pieris spp., Plutella xylostella, Prodenia spp., Pseudaletia spp., Pseudoplusia includens, Pyrausta nubilalis, Spodoptera spp., Thermesia gemmatalis, Tinea pellionella, Tineola bisselliella, Tortrix viridana, Trichoplusia spp..

Aus der Ordnung der Orthoptera z.B. Acheta domesticus, Blatta orientalis, Blattella germanica, Gryllotalpa spp., Leucophaea maderae, Locusta spp., Melanoplus spp., Periplaneta americana, Schistocerca gregaria.

Aus der Ordnung der Siphonaptera z.B. Ceratophyllus spp., Xenopsylla cheopis.

Aus der Ordnung der Symphyla z.B. Scutigerella immaculata.

Aus der Ordnung der Thysanoptera z.B. Baliothrips biformis, Enneothrips flavens, Frankliniella spp., Heliothrips spp., Hercinothrips femoralis, Kakothrips spp., Rhipiphorothrips cruentatus, Scirtothrips spp., Taeniothrips cardamoni, Thrips spp..

Aus der Ordnung der Thysanura z.B. Lepisma saccharina.

Zu den pflanzenparasitären Nematoden gehören z.B. Anguina spp., Aphelenchoides spp., Belonoaimus spp., Bursaphelenchus spp., Ditylenchus dipsaci, Globodera spp., Heliocotylenchus spp., Heterodera spp., Longidorus spp., Meloidogyne spp., Pratylenchus spp., Radopholus similis, Rotylenchus spp., Trichodorus spp., Tylenchorhynchus spp., Tylenchulus spp., Tylenchulus semipenetrans, Xiphinema spp..

Die erfindungsgemäßen Verbindungen der Formel (I) zeichnen sich insbesondere aus durch starke Wirkung gegen Insekten, Parasiten der Unterklasse der Acari (Acarina) (wie Milben, Spinnmilben und/oder Zecken) und/oder Nematoden.

Die erfindungsgemäßen Verbindungen weisen eine starke fungizide Wirkung auf und können zur Bekämpfung von pflanzenpathogenen Schadpilzen im Pflanzen- und Materialschutz eingesetzt werden.

Fungizide lassen sich im Pflanzenschutz zur Bekämpfung von Plasmodiophoromycetes, Oomycetes, Chytridiomycetes, Zygomycetes, Ascomycetes, Basidiomycetes und Deuteromycetes einsetzen.

Beispielhaft aber nicht begrenzend seien einige Erreger von pilzlichen und bakteriellen Erkrankungen, die unter die oben aufgezählten Oberbegriffe fallen, genannt:
Erkrankungen, hervorgerufen durch Erreger des Echten Mehltaus wie z.B.
Blumeria-Arten, wie beispielsweise Blumeria graminis;
Podosphaera-Arten, wie beispielsweise Podosphaera leucotricha;
Sphaerotheca-Arten, wie beispielsweise Sphaerotheca fuliginea;
Uncinula-Arten, wie beispielsweise Uncinula necator;
Erkrankungen, hervorgerufen durch Erreger von Rostkrankheiten wie z.B.
Gymnosporangium-Arten, wie beispielsweise Gymnosporangium sabinae
Hemileia-Arten, wie beispielsweise Hemileia vastatrix;
Phakopsora-Arten, wie beispielsweise Phakopsora pachyrhizi und Phakopsora meibomiae;
Puccinia-Arten, wie beispielsweise Puccinia recondita;
Uromyces-Arten, wie beispielsweise Uromyces appendiculatus;
Erkrankungen, hervorgerufen durch Erreger der Gruppe der Oomyceten wie z.B.
Bremia-Arten, wie beispielsweise Bremia lactucae;
Peronospora-Arten, wie beispielsweise Peronospora pisi oder P. brassicae;
Phytophthora-Arten, wie beispielsweise Phytophthora infestans;
Plasmopara-Arten, wie beispielsweise Plasmopara viticola;
Pseudoperonospora-Arten, wie beispielsweise Pseudoperonospora humuli oder
Pseudoperonospora cubensis;
Pythium-Arten, wie beispielsweise Pythium ultimum;
Blattfleckenkrankheiten und Blattwelken, hervorgerufen durch z.B.
Alternaria-Arten, wie beispielsweise Alternaria solani;
Cercospora-Arten, wie beispielsweise Cercospora beticola;
Cladosporium-Arten, wie beispielsweise Cladosporium cucumerinum;
Cochliobolus-Arten, wie beispielsweise Cochliobolus sativus
(Konidienform: Drechslera, Syn: Helminthosporium);
Colletotrichum-Arten, wie beispielsweise Colletotrichum lindemuthanium;
Cycloconium-Arten, wie beispielsweise Cycloconium oleaginum;
Diaporthe-Arten, wie beispielsweise Diaporthe citri;
Elsinoe-Arten, wie beispielsweise Elsinoe fawcettii;
Gloeosporium-Arten, wie beispielsweise Gloeosporium laeticolor;
Glomerella-Arten, wie beispielsweise Glomerella cingulata;
Guignardia-Arten, wie beispielsweise Guignardia bidwelli;
Leptosphaeria-Arten, wie beispielsweise Leptosphaeria maculans;
Magnaporthe-Arten, wie beispielsweise Magnaporthe grisea;
Mycosphaerella-Arten, wie beispielsweise Mycosphaerella graminicola und Mycosphaerella fijiensis;
Phaeosphaeria-Arten, wie beispielsweise Phaeosphaeria nodorum;
Pyrenophora-Arten, wie beispielsweise Pyrenophora teres;
Ramularia-Arten, wie beispielsweise Ramularia collo-cygni;
Rhynchosporium-Arten, wie beispielsweise Rhynchosporium secalis;
Septoria-Arten, wie beispielsweise Septoria apii;
Typhula-Arten, wie beispielsweise Typhula incarnata;
Venturia-Arten, wie beispielsweise Venturia inaequalis;
Wurzel- und Stengelkrankheiten, hervorgerufen durch z.B.
Corticium-Arten, wie beispielsweise Corticium graminearum;
Fusarium-Arten, wie beispielsweise Fusarium oxysporum;
Gaeumannomyces-Arten, wie beispielsweise Gaeumannomyces graminis;
Rhizoctonia-Arten, wie beispielsweise Rhizoctonia solani;
Tapesia-Arten, wie beispielsweise Tapesia acuformis;
Thielaviopsis-Arten, wie beispielsweise Thielaviopsis basicola;
Ähren- und Rispenerkrankungen (inklusive Maiskolben), hervorgerufen durch z.B.
Alternaria-Arten, wie beispielsweise Alternaria spp.;
Aspergillus-Arten, wie beispielsweise Aspergillus flavus;
Cladosporium-Arten, wie beispielsweise Cladosporium cladosporioides;
Claviceps-Arten, wie beispielsweise Claviceps purpurea;
Fusarium-Arten, wie beispielsweise Fusarium culmorum;
Gibberella-Arten, wie beispielsweise Gibberella zeae;
Monographella-Arten, wie beispielsweise Monographella nivalis;
Erkrankungen, hervorgerufen durch Brandpilze wie z.B.
Sphacelotheca-Arten, wie beispielsweise Sphacelotheca reiliana;
Tilletia-Arten, wie beispielsweise Tilletia caries;
Urocystis-Arten, wie beispielsweise Urocystis occulta;
Ustilago-Arten, wie beispielsweise Ustilago nuda;
Fruchtfäule hervorgerufen durch z.B.
Aspergillus-Arten, wie beispielsweise Aspergillus flavus;
Botrytis-Arten, wie beispielsweise Botrytis cinerea;
Penicillium-Arten, wie beispielsweise Penicillium expansum und Penicillium purpurogenum;
Sclerotinia-Arten, wie beispielsweise Sclerotinia sclerotiorum;
Verticilium-Arten, wie beispielsweise Verticilium alboatrum;
Samen- und bodenbürtige Fäulen und Welken, sowie Sämlingserkrankungen, hervorgerufen durch z.B.
Fusarium-Arten, wie beispielsweise Fusarium culmorum;
Phytophthora Arten, wie beispielsweise Phytophthora cactorum;
Pythium-Arten, wie beispielsweise Pythium ultimum;
Rhizoctonia-Arten, wie beispielsweise Rhizoctonia solani;
Sclerotium-Arten, wie beispielsweise Sclerotium rolfsii;
Krebserkrankungen, Gallen und Hexenbesen, hervorgerufen durch z.B.
Nectria-Arten, wie beispielsweise Nectria galligena;
Welkeerkrankungen hervorgerufen durch z.B.
Monilinia-Arten, wie beispielsweise Monilinia laxa;
Deformationen von Blättern, Blüten und Früchten, hervorgerufen durch z.B.
Taphrina-Arten, wie beispielsweise Taphrina deformans;
Degenerationserkrankungen holziger pflanzen, hervorgerufen durch z.B.
Esca-Arten, wie beispielsweise Phaeomoniella chlamydospora und Phaeoacremonium aleophilum und Fomitiporia mediterranea;
Blüten- und Samenerkrankungen, hervorgerufen durch z.B.
Botrytis-Arten, wie beispielsweise Botrytis cinerea;
Erkrankungen von Pflanzenknollen, hervorgerufen durch z.B.
Rhizoctonia-Arten, wie beispielsweise Rhizoctonia solani;
Helminthosporium-Arten, wie beispielsweise Helminthosporium solani;
Erkrankungen, hervorgerufen durch bakterielle Erreger wie z.B.
Xanthomonas-Arten, wie beispielsweise Xanthomonas campestris pv. oryzae;
Pseudomonas-Arten, wie beispielsweise Pseudomonas syringae pv. lachrymans;
Erwinia-Arten, wie beispielsweise Erwinia amylovora;
Bevorzugt können die folgenden Krankheiten von Soja-Bohnen bekämpft werden:
   Pilzkrankheiten an Blättern, Stängeln, Schoten und Samen verursacht durch z.B.

Alternaria leaf spot (Alternaria spec. atrans tenuissima), Anthracnose (Colletotrichum gloeosporoides dematium var. truncatum), Brown spot (Septoria glycines), Cercospora leaf spot and blight (Cercospora kikuchii), Choanephora leaf blight (Choanephora infundibulifera trispora (Syn.)), Dactuliophora leaf spot (Dactuliophora glycines), Downy Mildew (Peronospora manshurica), Drechslera blight (Drechslera glycini), Frogeye Leaf spot (Cercospora sojina), Leptosphaerulina Leaf Spot (Leptosphaerulina trifolii), Phyllostica Leaf Spot (Phyllosticta sojaecola), Pod and Stem Blight (Phomopsis sojae), Powdery Mildew (Microsphaera diffusa), Pyrenochaeta Leaf Spot (Pyrenochaeta glycines), Rhizoctonia Aerial, Foliage, and Web Blight (Rhizoctonia solani), Rust (Phakopsora pachyrhizi), Scab (Sphaceloma glycines), Stemphylium Leaf Blight (Stemphylium botryosum), Target Spot (Corynespora cassiicola)

Pilzkrankheiten an Wurzeln und der Stängelbasis verursacht durch z.B.

Black Root Rot (Calonectria crotalariae), Charcoal Rot (Macrophomina phaseolina), Fusarium Blight or Wilt, Root Rot, and Pod and Collar Rot (Fusarium oxysporum, Fusarium orthoceras, Fusarium semitectum, Fusarium equiseti), Mycoleptodiscus Root Rot (Mycoleptodiscus terrestris), Neocosmospora (Neocosmopspora vasinfecta), Pod and Stem Blight (Diaporthe phaseolorum), Stem Canker (Diaporthe phaseolorum var. caulivora), Phytophthora Rot (Phytophthora megasperma), Brown Stem Rot (Phialophora gregata), Pythium Rot (Pythium aphanidermatum, Pythium irregulare, Pythium debaryanum, Pythium myriotylum, Pythium ultimum), Rhizoctonia Root Rot, Stem Decay, and Damping-Off (Rhizoctonia solani), Sclerotinia Stem Decay (Sclerotinia sclerotiorum), Sclerotinia Southern Blight (Sclerotinia rolfsii), Thielaviopsis Root Rot (Thielaviopsis basicola).

Die erfindungsgemäßen Verbindungen weisen auch eine starke stärkende Wirkung in Pflanzen auf. Sie eignen sich daher zur Mobilisierung pflanzeneigener Abwehrkräfte gegen Befall durch unerwünschte pflanzenpathogene Schadpilze.

Unter pflanzenstärkenden (resistenzinduzierenden) Verbindungen sind im vorliegenden Zusammenhang solche Substanzen zu verstehen, die in der Lage sind, das Abwehrsystem von Pflanzen so zu stimulieren, dass die behandelten Pflanzen bei nachfolgender Inokulation mit unerwünschten pflanzenpathogene Schadpilze weitgehende Resistenz gegen diese pflanzenpathogene Schadpilze entfalten.

Die erfindungsgemäßen Verbindungen können also eingesetzt werden, um Pflanzen innerhalb eines gewissen Zeitraums nach der Behandlung gegen den Befall durch die genannten Schaderreger zu schützen. Der Zeitraum, innerhalb dessen Schutz herbeigeführt wird, erstreckt sich im Allgemeinen von 1 bis 10 Tage, vorzugsweise 1 bis 7 Tage nach der Behandlung der Pflanzen mit den Wirkstoffen.

Die gute Pflanzenverträglichkeit der Verbindungen in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei lassen sich die erfindungsgemäßen Verbindungen mit besonders gutem Erfolg zur Bekämpfung von Getreidekrankheiten, wie z.B. gegen Puccinia-Arten und von Krankheiten im Wein-, Obst- und Gemüseanbau, wie z.B. gegen Botrytis-, Venturia- oder Alternaria-Arten, einsetzen.

Die erfindungsgemäßen Verbindungen eignen sich auch zur Steigerung des Ernteertrages. Sie sind außerdem mindertoxisch und weisen eine gute Pflanzenverträglichkeit auf.

Die erfindungsgemäßen Verbindungen können gegebenenfalls in bestimmten Konzentrationen bzw. Aufwandmengen auch als Herbizide, Safener, Wachstumsregulatoren oder Mittel zur Verbesserung der Pflanzeneigenschaften, oder als Mikrobizide, beispielsweise als Fungizide, Antimykotika, Bakterizide, Virizide (einschließlich Mittel gegen Viroide) oder als Mittel gegen MLO (Mycoplasma-like-organism) und RLO (Rickettsia-like-organism) verwendet werden. Sie lassen sich gegebenenfalls auch als Zwischen- oder Vorprodukte für die Synthese weiterer Wirkstoffe einsetzen.

Die Wirkstoffe können in die üblichen Formulierungen überführt werden, wie Lösungen, Emulsionen, Spritzpulver, wasser- und ölbasierte Suspensionen, Pulver, Stäubemittel, Pasten, lösliche Pulver, lösliche Granulate, Streugranulate, Suspensions-Emulsions-Konzentrate, Wirkstoff-imprägnierte Naturstoffe, Wirkstoff-imprägnierte synthetische Stoffe, Düngemittel sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln. Die Herstellung der Formulierungen erfolgt entweder in geeigneten Anlagen oder auch vor oder während der Anwendung.

Als Hilfsstoffe können solche Stoffe Verwendung finden, die geeignet sind, dem Mittel selbst oder und/oder davon abgeleitete Zubereitungen (z.B. Spritzbrühen, Saatgutbeizen) besondere Eigenschaften zu verleihen, wie bestimmte technische Eigenschaften und/oder auch besondere biologische Eigenschaften. Als typische Hilfsmittel kommen in Frage: Streckmittel, Lösemittel und Trägerstoffe.

Als Streckmittel eignen sich z.B. Wasser, polare und unpolare organische chemische Flüssigkeiten z.B. aus den Klassen der aromatischen und nicht-aromatischen Kohlenwasserstoffe (wie Paraffine, Alkylbenzole, Alkylnaphthaline, Chlorbenzole), der Alkohole und Polyole (die ggf. auch substituiert, verethert und/oder verestert sein können), der Ketone (wie Aceton, Cyclohexanon), Ester (auch Fette und Öle) und (poly-)Ether, der einfachen und substituierten Amine, Amide, Lactame (wie N-Alkylpyrrolidone) und Lactone, der Sulfone und Sulfoxide (wie Dimethylsysulfoxid).

Im Falle der Benutzung von Wasser als Streckmittel können z.B. auch organische Lösemittel als Hilfslösungsmittel verwendet werden. Als flüssige Lösemittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Papier, Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage nicht-ionische und/oder ionische Stoffe, z.B. aus den Klassen der Alkohol-POE- und/oder POP-Ether, Säure- und/oder POP- POE-Ester, Alkyl-Aryl- und/oder POP- POE-Ether, Fett- und/oder POP- POE-Addukte, POE- und/oder POP-Polyol Derivate, POE- und/oder POP-Sorbitan- oder-Zucker-Addukte, Alky- oder Aryl-Sulfate, Sulfonate und Phosphate oder die entsprechenden PO-Ether-Addukte. Ferner geeignete Oligo- oder Polymere, z.B. ausgehend von vinylischen Monomeren, von Acrylsäure, aus EO und/oder PO allein oder in Verbindung mit z.B. (poly-) Alkoholen oder (poly-) Aminen. Ferner können Einsatz finden Lignin und seine Sulfonsäure-Derivate, einfache und modifizierte Cellulosen, aromatische und/oder aliphatische Sulfonsäuren sowie deren Addukte mit Formaldehyd.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Weitere Additive können Duftstoffe, mineralische oder vegetabile gegebenenfalls modifizierte Öle, Wachse und Nährstoffe (auch Spurennährstoffe), wie Salze von Eisen, Mangan, Bor, Kupfer, Kobalt, Molybdän und Zink sein.

Weiterhin enthalten sein können Stabilisatoren wie Kältestabilisatoren, Konservierungsmittel, Oxidationsschutzmittel, Lichtschutzmittel oder andere die chemische und / oder physikalische Stabilität verbessernde Mittel.

Die Formulierungen enthalten im Allgemeinen zwischen 0,01 und 98 Gew.-% Wirkstoff, vorzugsweise zwischen 0,5 und 90 %.

Der erfindungsgemäße Wirkstoff kann in seinen handelsüblichen Formulierungen sowie in den aus diesen Formulierungen bereiteten Anwendungsformen in Mischung mit anderen Wirkstoffen wie Insektiziden, Lockstoffen, Sterilantien, Bakteriziden, Akariziden, Nematiziden, Fungiziden, wachstumsregulierenden Stoffen, Herbiziden, Safenern, Düngemitteln oder Semiochemicals vorliegen.

Auch eine Mischung mit anderen bekannten Wirkstoffen, wie Herbiziden, Düngemitteln, Wachstumsregulatoren, Safenern, Semiochemicals, oder auch mit Mitteln zur Verbesserung der Pflanzeneigenschaften ist möglich.

Die erfindungsgemäßen Wirkstoffe können ferner beim Einsatz als Insektizide oder Fungizide in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Die erfindungsgemäßen Wirkstoffe können ferner beim Einsatz als Insektizide oder Fungizide in ihren handelsüblichen Formulierungen sowie in den aus diesen Formulierungen bereiteten Anwendungsformen in Mischungen mit Hemmstoffen vorliegen, die einen Abbau des Wirkstoffes nach Anwendung in der Umgebung der Pflanze, auf der Oberfläche von Pflanzenteilen oder in pflanzlichen Geweben vermindern.

Der Wirkstoffgehalt der aus den handelsüblichen Formulierungen bereiteten Anwendungsformen kann in weiten Bereichen variieren. Die Wirkstoffkonzentration der Anwendungsformen kann von 0,00000001 bis zu 95 Gew.-% Wirkstoff, vorzugsweise zwischen 0,00001 und 1 Gew.-% liegen.

Die Anwendung geschieht in einer den Anwendungsformen angepaßten üblichen Weise.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Sproß, Blatt, Blüte und Wurzel verstanden werden, wobei beispielhaft Blätter, Nadeln, Stengel, Stämme, Blüten, Fruchtkörper, Früchte und Saatgut sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Saatgut.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Gießen, Sprühen, Verdampfen, Vernebeln, Streuen, Aufstreichen, Injizieren und bei Vermehrungsmaterial, insbesondere bei Saatgut, weiterhin durch ein- oder mehrschichtiges Umhüllen.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Die Begriffe "Teile" bzw. "Teile von Pflanzen" oder "Pflanzenteile" wurden oben erläutert.

Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Bio- und Genotypen sein.

Je nach Pflanzenarten bzw. Pflanzensorten, deren Standort und Wachstumsbedingungen (Böden, Klima, Vegetationsperiode, Ernährung) können durch die erfindungsgemäße Behandlung auch überadditive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel, besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte möglich, die über die eigentlich zu erwartenden Effekte hinausgehen.

Das erfindungsgemäße Behandlungsverfahren kann für die Behandlung von genetisch modifizierten Organismen (GMOs), z. B. Pflanzen oder Samen, verwendet werden. Genetisch modifizierte Pflanzen (oder transgene Pflanzen) sind Pflanzen, bei denen ein heterologes Gen stabil in das Genom integriert worden ist. Der Begriff "heterologes Gen" bedeutet im wesentlichen ein Gen, das außerhalb der Pflanze bereitgestellt oder assembliert wird und das bei Einführung in das Zellkerngenom, das Chloroplastengenom oder das Hypochondriengenom der transformierten Pflanze dadurch neue oder verbesserte agronomische oder sonstige Eigenschaften verleiht, daß es ein interessierendes Protein oder Polypeptid exprimiert oder daß es ein anderes Gen, das in der Pflanze vorliegt bzw. andere Gene, die in der Pflanze vorliegen, herunterreguliert oder abschaltet (zum Beispiel mittels Antisense-Technologie, Cosuppressionstechnologie oder RNAi-Technologie [RNA Interference]). Ein heterologes Gen, das im Genom vorliegt, wird ebenfalls als Transgen bezeichnet. Ein Transgen, das durch sein spezifisches Vorliegen im Pflanzengenom definiert ist, wird als Transformations- bzw. transgenes Event bezeichnet.

In Abhängigkeit von den Pflanzenarten oder Pflanzensorten, ihrem Standort und ihren Wachstumsbedingungen (Böden, Klima, Vegetationsperiode, Ernährung) kann die erfindungsgemäße Behandlung auch zu überadditiven ("synergistischen") Effekten führen. So sind zum Beispiel die folgenden Effekte möglich, die über die eigentlich zu erwartenden Effekte hinausgehen: verringerte Aufwandmengen und/oder erweitertes Wirkungsspektrum und/oder erhöhte Wirksamkeit der Wirkstoffe und Zusammensetzungen, die erfindungsgemäß eingesetzt werden können, besseres Pflanzenwachstum, erhöhte Toleranz gegenüber hohen oder niedrigen Temperaturen, erhöhte Toleranz gegenüber Trockenheit oder Wasser- oder Bodensalzgehalt, erhöhte Blühleistung, Ernteerleichterung, Reifebeschleunigung, höhere Erträge, größere Früchte, größere Pflanzenhöhe, intensiver grüne Farbe des Blatts, frühere Blüte, höhere Qualität und/oder höherer Nährwert der Ernteprodukte, höhere Zuckerkonzentration in den Früchten, bessere Lagerfähigkeit und/oder Verarbeitbarkeit der Ernteprodukte.

In gewissen Aufwandmengen können die erfindungsgemäßen Wirkstoffkombinationen auch eine stärkende Wirkung auf Pflanzen ausüben. Sie eignen sich daher für die Mobilisierung des pflanzlichen Abwehrsystems gegen Angriff durch unerwünschte phytopathogene Pilze und/oder Mikroorganismen und/oder Viren. Dies kann gegebenenfalls einer der Gründe für die erhöhte Wirksamkeit der erfindungsgemäßen Kombinationen sein, zum Beispiel gegen Pilze. Pflanzenstärkende (resistenzinduzierende) Substanzen sollen im vorliegenden Zusammenhang auch solche Substanzen oder Substanzkombinationen bedeuten, die fähig sind, das pflanzliche Abwehrsystem so zu stimulieren, daß die behandelten Pflanzen, wenn sie im Anschluß daran mit unerwünschten phytopathogenen Pilzen und/oder Mikroorganismen und/oder Viren inokkuliert werde, einen beträchtlichen Resistenzgrad gegen diese unerwünschten phytopathogenen Pilze und/oder Mikroorganismen und/oder Viren aufweisen. Im vorliegenden Fall versteht man unter unerwünschten phytopathogenen Pilzen und/oder Mikroorganismen und/oder Viren phytopathogene Pilze, Bakterien und Viren. Die erfindungsgemäßen Substanzen lassen sich daher zum Schutz von Pflanzen gegen Angriff durch die erwähnten Pathogene innerhalb eines gewissen Zeitraums nach der Behandlung einsetzen. Der Zeitraum, über den eine Schutzwirkung erzielt wird, erstreckt sich im allgemeinen von 1 bis 10 Tagen, vorzugsweise 1 bis 7 Tagen, nach der Behandlung der Pflanzen mit den Wirkstoffen.

Zu Pflanzen und Pflanzensorten, die vorzugsweise erfindungsgemäß behandelt werden, zählen alle Pflanzen, die über Erbgut verfügen, das diesen Pflanzen besonders vorteilhafte, nützliche Merkmale verleiht (egal, ob dies durch Züchtung und/oder Biotechnologie erzielt wurde).

Pflanzen und Pflanzensorten, die ebenfalls vorzugsweise erfindungsgemäß behandelt werden, sind gegen einen oder mehrere biotische Streßfaktoren resistent, d. h. diese Pflanzen weisen eine verbesserte Abwehr gegen tierische und mikrobielle Schädlinge wie Nematoden, Insekten, Milben, phytopathogene Pilze, Bakterien, Viren und/oder Viroide auf.

Pflanzen und Pflanzensorten, die ebenfalls erfindungsgemäß behandelt werden können, sind solche Pflanzen, die gegen einen oder mehrere abiotische Streßfaktoren resistent sind. Zu den abiotischen Streßbedingungen können zum Beispiel Dürre, Kälte- und Hitzebedingungen, osmotischer Streß, Staunässe, erhöhter Bodensalzgehalt, erhöhtes Ausgesetztsein an Mineralien, Ozonbedingungen, Starklichtbedingungen, beschränkte Verfügbarkeit von Stickstoffnährstoffen, beschränkte Verfügbarkeit von Phosphornährstoffen oder Vermeidung von Schatten zählen.

Pflanzen und Pflanzensorten, die ebenfalls erfindungsgemäß behandelt werden können, sind solche Pflanzen, die durch erhöhte Ertragseigenschaften gekennzeichnet sind. Ein erhöhter Ertrag kann bei diesen Pflanzen z. B. auf verbesserter Pflanzenphysiologie, verbessertem Pflanzenwuchs und verbesserter Pflanzenentwicklung, wie Wasserverwertungseffizienz, Wasserhalteeffizienz, verbesserter Stickstoffverwertung, erhöhter Kohlenstoffassimilation, verbesserter Photosynthese, verstärkter Keimkraft und beschleunigter Abreife beruhen. Der Ertrag kann weiterhin durch eine verbesserte Pflanzenarchitektur (unter Streß- und nicht-Streß-Bedingungen) beeinflußt werden, darunter frühe Blüte, Kontrolle der Blüte für die Produktion von Hybridsaatgut, Keimpflanzenwüchsigkeit, Pflanzengröße, Internodienzahl und -abstand, Wurzelwachstum, Samengröße, Fruchtgröße, Schotengröße, Schoten- oder Ährenzahl, Anzahl der Samen pro Schote oder Ähre, Samenmasse, verstärkte Samenfüllung, verringerter Samenausfall, verringertes Schotenplatzen sowie Standfestigkeit. Zu weiteren Ertragsmerkmalen zählen Samenzusammensetzung wie Kohlenhydratgehalt, Proteingehalt, Ölgehalt und Ölzusammensetzung, Nährwert, Verringerung der nährwidrigen Verbindungen, verbesserte Verarbeitbarkeit und verbesserte Lagerfähigkeit.

Pflanzen, die erfindungsgemäß behandelt werden können, sind Hybridpflanzen, die bereits die Eigenschaften der Heterosis bzw. des Hybrideffekts exprimieren, was im allgemeinen zu höherem Ertrag, höherer Wüchsigkeit, besserer Gesundheit und besserer Resistenz gegen biotische und abiotische Streßfaktoren führt. Solche Pflanzen werden typischerweise dadurch erzeugt, daß man eine ingezüchtete pollensterile Elternlinie (den weiblichen Kreuzungspartner) mit einer anderen ingezüchteten pollenfertilen Elternlinie (dem männlichen Kreuzungspartner) kreuzt. Das Hybridsaatgut wird typischerweise von den pollensterilen Pflanzen geerntet und an Vermehrer verkauft. Pollensterile Pflanzen können manchmal (z. B. beim Mais) durch Entfahnen (d. h. mechanischem Entfernen der männlichen Geschlechtsorgane bzw. der männlichen Blüten), produziert werden; es ist jedoch üblicher, daß die Pollensterilität auf genetischen Determinanten im Pflanzengenom beruht. In diesem Fall, insbesondere dann, wenn es sich bei dem gewünschten Produkt, da man von den Hybridpflanzen ernten will, um die Samen handelt, ist es üblicherweise günstig, sicherzustellen, daß die Pollenfertilität in Hybridpflanzen, die die für die Pollensterilität verantwortlichen genetischen Determinanten enthalten, völlig restoriert wird. Dies kann erreicht werden, indem sichergestellt wird, daß die männlichen Kreuzungspartner entsprechende Fertilitätsrestorergene besitzen, die in der Lage sind, die Pollenfertilität in Hybridpflanzen, die die genetischen Determinanten, die für die Pollensterilität verantwortlich sind, enthalten, zu restorieren. Genetische Determinanten für Pollensterilität können im Cytoplasma lokalisiert sein. Beispiele für cytoplasmatische Pollensterilität (CMS) wurden zum Beispiel für Brassica-Arten beschrieben. Genetische Determinanten für Pollensterilität können jedoch auch im Zellkerngenom lokalisiert sein. Pollensterile Pflanzen können auch mit Methoden der pflanzlichen Biotechnologie, wie Gentechnik, erhalten werden. Ein besonders günstiges Mittel zur Erzeugung von pollensterilen Pflanzen ist in WO 89/10396 beschrieben, wobei zum Beispiel eine Ribonuklease wie eine Barnase selektiv in den Tapetumzellen in den Staubblättern exprimiert wird. Die Fertilität kann dann durch Expression eines Ribonukleasehemmers wie Barstar in den Tapetumzellen restoriert werden.

Pflanzen oder Pflanzensorten (die mit Methoden der Pflanzenbiotechnologie, wie der Gentechnik, erhalten werden), die erfindungsgemäß behandelt werden können, sind herbizidtolerante Pflanzen, d. h. Pflanzen, die gegenüber einem oder mehreren vorgegebenen Herbiziden tolerant gemacht worden sind. Solche Pflanzen können entweder durch genetische Transformation oder durch Selektion von Pflanzen, die eine Mutation enthalten, die solch eine Herbizidtoleranz verleiht, erhalten werden.

Herbizidtolerante Pflanzen sind zum Beispiel glyphosatetolerante Pflanzen, d. h. Pflanzen, die gegenüber dem Herbizid Glyphosate oder dessen Salzen tolerant gemacht worden sind. So können zum Beispiel glyphosatetolerante Pflanzen durch Transformation der Pflanze mit einem Gen, das für das Enzym 5-Enolpyruvylshikimat-3-phosphatsynthase (EPSPS) kodiert, erhalten werden. Beispiele für solche EPSPS-Gene sind das AroA-Gen (Mutante CT7) des Bakterium *Salmonella typhimurium*, das CP4-Gen des Bakteriums *Agrobacterium sp*., die Gene, die für eine EPSPS aus der Petunie, für eine EPSPS aus der Tomate oder für eine EPSPS aus Eleusine kodieren. Es kann sich auch um eine mutierte EPSPS handeln. Glyphosatetolerante Pflanzen können auch dadurch erhalten werden, daß man ein Gen exprimiert, das für ein Glyphosate-Oxidoreduktase-Enzym kodiert. Glyphosatetolerante Pflanzen können auch dadurch erhalten werden, daß man ein Gen exprimiert, das für ein Glyphosate-acetyltransferase-Enzym kodiert. Glyphosatetolerante Pflanzen können auch dadurch erhalten werden, daß man Pflanzen, die natürlich vorkommende Mutationen der oben erwähnten Gene selektiert.

Sonstige herbizidresistente Pflanzen sind zum Beispiel Pflanzen, die gegenüber Herbiziden, die das Enzym Glutaminsynthase hemmen, wie Bialaphos, Phosphinotricin oder Glufosinate, tolerant gemacht worden sind. Solche Pflanzen können dadurch erhalten werden, daß man ein Enzym exprimiert, das das Herbizid oder eine Mutante des Enzyms Glutaminsynthase, das gegenüber Hemmung resistent ist, entgiftet. Solch ein wirksames entgiftendes Enzym ist zum Beispiel ein Enzym, das für ein Phosphinotricin-acetyltransferase kodiert (wie zum Beispiel das bar- oder pat-Protein aus Streptomyces-Arten). Pflanzen, die eine exogene Phosphinotricin-acetyltransferase exprimieren, sind beschrieben.

Weitere herbizidtolerante Pflanzen sind auch Pflanzen, die gegenüber den Herbiziden, die das Enzym Hydroxyphenylpyruvatdioxygenase (HPPD) hemmen, tolerant gemacht worden sind. Bei den Hydroxyphenylpyruvatdioxygenasen handelt es sich um Enzyme, die die Reaktion, in der para-Hydroxyphenylpyruvat (HPP) zu Homogentisat umgesetzt wird, katalysieren. Pflanzen, die gegenüber HPPD-Hemmern tolerant sind, können mit einem Gen, das für ein natürlich vorkommendes resistentes HPPD-Enzym kodiert, oder einem Gen, das für ein mutiertes HPPD-Enzym kodiert, transformiert werden. Eine Toleranz gegenüber HPPD-Hemmern kann auch dadurch erzielt werden, daß man Pflanzen mit Genen transformiert, die für gewisse Enzyme kodieren, die die Bildung von Homogentisat trotz Hemmung des nativen HPPD-Enzyms durch den HPPD-Hemmer ermöglichen. Die Toleranz von Pflanzen gegenüber HPPD-Hemmern kann auch dadurch verbessert werden, daß man Pflanzen zusätzlich zu einem Gen, das für ein HPPDtolerantes Enzym kodiert, mit einem Gen transformiert, das für ein Prephenatdehydrogenase-Enzym kodiert.

Weitere herbizidresistente Pflanzen sind Pflanzen, die gegenüber Acetolactatsynthase (ALS)-Hemmern tolerant gemacht worden sind. Zu bekannten ALS-Hemmern zählen zum Beispiel Sulfonylharnstoff, Imidazolinon, Triazolopyrimidine, Pyrimidinyloxy(thio)benzoate und/oder Sulfonylaminocarbonyltriazolinon-Herbizide. Es ist bekannt, daß verschiedene Mutationen im Enzym ALS (auch als Acetohydroxysäure-Synthase, AHAS, bekannt) eine Toleranz gegenüber unterschiedlichen Herbiziden bzw. Gruppen von Herbiziden verleihen. Die Herstellung von sulfonylharnstofftoleranten Pflanzen und imidazolinontoleranten Pflanzen ist in der internationalen Veröffentlichung WO 1996/033270 beschrieben. Weitere sulfonylharnstoff- und imidazolinontolerante Pflanzen sind auch in z.B. WO 2007/024782 beschrieben.

Weitere Pflanzen, die gegenüber Imidazolinon und/oder Sulfonylharnstoff tolerant sind, können durch induzierte Mutagenese, Selektion in Zellkulturen in Gegenwart des Herbizids oder durch Mutationszüchtung erhalten werden.

Pflanzen oder Pflanzensorten (die nach Methoden der pflanzlichen Biotechnologie, wie der Gentechnik, erhalten wurden), die ebenfalls erfindungsgemäß behandelt werden können, sind insektenresistente transgene Pflanzen, d.h. Pflanzen, die gegen Befall mit gewissen Zielinsekten resistent gemacht wurden. Solche Pflanzen können durch genetische Transformation oder durch Selektion von Pflanzen, die eine Mutation enthalten, die solch eine Insektenresistenz verleiht, erhalten werden.

Der Begriff "insektenresistente transgene Pflanze" umfaßt im vorliegenden Zusammenhang jegliche Pflanze, die mindestens ein Transgen enthält, das eine Kodiersequenz umfaßt, die für folgendes kodiert:
1) ein insektizides Kristallprotein aus *Bacillus thuringiensis* oder einen insektiziden Teil davon, wie die insektiziden Kristallproteine, die online bei: http://www.lifesci.sussex.ac.uk/Home/Neil Crickmore/Bt/ beschrieben sind, zusammengestellt wurden, oder insektizide Teile davon, z.B. Proteine der Cry-Proteinklassen Cry1Ab, Cry1Ac, Cry1F, Cry2Ab, Cry3Ae oder Cry3Bb oder insektizide Teile davon; oder
2) ein Kristallprotein aus *Bacillus thuringiensis* oder einen Teil davon, der in Gegenwart eines zweiten, anderen Kristallproteins als *Bacillus thuringiensis* oder eines Teils davon insektizid wirkt, wie das binäre Toxin, das aus den Kristallproteinen Cy34 und Cy35 besteht; oder
3) ein insektizides Hybridprotein, das Teile von zwei unterschiedlichen insektiziden Kristallproteinen aus *Bacillus thuringiensis* umfaßt, wie zum Beispiel ein Hybrid aus den Proteinen von 1) oben oder ein Hybrid aus den Proteinen von 2) oben, z. B. das Protein Cry1A.105, das von dem Mais-Event MON98034 produziert wird (WO 2007/027777); oder
4) ein Protein gemäß einem der Punkte 1) bis 3) oben, in dem einige, insbesondere 1 bis 10, Aminosäuren durch eine andere Aminosäure ersetzt wurden, um eine höhere insektizide Wirksamkeit gegenüber einer Zielinsektenart zu erzielen und/oder um das Spektrum der entsprechenden Zielinsektenarten zu erweitern und/oder wegen Veränderungen, die in die Kodier- DNA während der Klonierung oder Transformation induziert wurden, wie das Protein Cry3Bb1 in Mais-Events MON863 oder MON88017 oder das Protein Cry3A im Mais-Event MIR 604;
5) ein insektizides sezerniertes Protein aus *Bacillus thuringiensis* oder *Bacillus cereus* oder einen insektiziden Teil davon, wie die vegetativ wirkenden insektentoxischen Proteine (vegetative insekticidal proteins, VIP), die unter http://www.lifesci.sussex.ac.uk/Home/Neil_Crickmore/Bt/vip.html angeführt sind, z. B. Proteine der Proteinklasse VIP3Aa; oder
6) ein sezerniertes Protein aus *Bacillus thuringiensis* oder *Bacillus cereus*, das in Gegenwart eines zweiten sezernierten Proteins aus *Bacillus thuringiensis* oder *B. cereus* insektizid wirkt, wie das binäre Toxin, das aus den Proteinen VIP1A und VIP2A besteht.
7) ein insektizides Hybridprotein, das Teile von verschiedenen sezernierten Proteinen von *Bacillus thuringiensis* oder *Bacillus cereus* umfaßt, wie ein Hybrid der Proteine von 1) oder ein Hybrid der Proteine von 2) oben; oder
8) ein Protein gemäß einem der Punkte 1) bis 3) oben, in dem einige, insbesondere 1 bis 10, Aminosäuren durch eine andere Aminosäure ersetzt wurden, um eine höhere insektizide Wirksamkeit gegenüber einer Zielinsektenart zu erzielen und/oder um das Spektrum der entsprechenden Zielinsektenarten zu erweitern und/oder wegen Veränderungen, die in die Kodier- DNA während der Klonierung oder Transformation induziert wurden (wobei die Kodierung für ein insektizides Protein erhalten bleibt), wie das Protein VIP3Aa im Baumwoll-Event COT 102.

Natürlich zählt zu den insektenresistenten transgenen Pflanzen im vorliegenden Zusammenhang auch jegliche Pflanze, die eine Kombination von Genen umfaßt, die für die Proteine von einer der oben genannten Klassen 1 bis 8 kodieren. In einer Ausführungsform enthält eine insektenresistente Pflanze mehr als ein Transgen, das für ein Protein nach einer der oben genannten 1 bis 8 kodiert, um das Spektrum der entsprechenden Zielinsektenarten zu erweitern oder um die Entwicklung einer Resistenz der Insekten gegen die Pflanzen dadurch hinauszuzögern, daß man verschiedene Proteine einsetzt, die für dieselbe Zielinsektenart insektizid sind, jedoch eine unterschiedliche Wirkungsweise, wie Bindung an unterschiedliche Rezeptorbindungsstellen im Insekt, aufweisen.

Pflanzen oder Pflanzensorten (die nach Methoden der pflanzlichen Biotechnologie, wie der Gentechnik, erhalten wurden), die ebenfalls erfindungsgemäß behandelt werden können, sind gegenüber abiotischen Streßfaktoren tolerant. Solche Pflanzen können durch genetische Transformation oder durch Selektion von Pflanzen, die eine Mutation enthalten, die solch eine Streßresistenz verleiht, erhalten werden. Zu besonders nützlichen Pflanzen mit Streßtoleranz zählen folgende:
a. Pflanzen, die ein Transgen enthalten, das die Expression und/oder Aktivität des Gens für die Poly(ADP-ribose)polymerase (PARP) in den Pflanzenzellen oder Pflanzen zu reduzieren vermag.
b. Pflanzen, die ein streßtoleranzförderndes Transgen enthalten, das die Expression und/oder Aktivität der für PARG kodierenden Gene der Pflanzen oder Pflanzenzellen zu reduzieren vermag;
c. Pflanzen, die ein streßtoleranzförderndes Transgen enthalten, das für ein in Pflanzen funktionelles Enzym des Nicotinamidadenindinukleotid-Salvage-Biosynthesewegs kodiert, darunter Nicotinamidase, Nicotinatphosphoribosyltransferase, Nicotinsäuremononukleotidadenyltransferase, Nicotinamidadenindinukleotidsynthetase oder Nicotinamidphosphoribosyltransferase.

Pflanzen oder Pflanzensorten (die nach Methoden der pflanzlichen Biotechnologie, wie der Gentechnik, erhalten wurden), die ebenfalls erfindungsgemäß behandelt werden können, weisen eine veränderte Menge, Qualität und/oder Lagerfähigkeit des Ernteprodukts und/oder veränderte Eigenschaften von bestimmten Bestandteilen des Ernteprodukts auf, wie zum Beispiel:
1) Transgene Pflanzen, die eine modifizierte Stärke synthetisieren, die bezüglich ihrer chemisch-physikalischen Eigenschaften, insbesondere des Amylosegehalts oder des Amylose/Amylopektin-Verhältnisses, des Verzweigungsgrads, der durchschnittlichen Kettenlänge, der Verteilung der Seitenketten, des Viskositätsverhaltens, der Gelfestigkeit, der Stärkekorngröße und/oder Stärkekornmorphologie im Vergleich mit der synthetisierten Stärke in Wildtyppflanzenzellen oder -pflanzen verändert ist, so daß sich diese modifizierte Stärke besser für bestimmte Anwendungen eignet.
2) Transgene Pflanzen, die Nichtstärkekohlenhydratpolymere synthetisieren, oder Nichtstärkekohlenhydratpolymere, deren Eigenschaften im Vergleich zu Wildtyppflanzen ohne genetische Modifikation verändert sind. Beispiele sind Pflanzen, die Polyfructose, insbesondere des Inulin- und Levantyps, produzieren, Pflanzen, die alpha-1,4-Glucane produzieren, Pflanzen, die alpha-1,6-verzweigte alpha-1,4-Glucane produzieren und Pflanzen, die Alternan produzieren.
3) Transgene Pflanzen, die Hyaluronan produzieren.

Pflanzen oder Pflanzensorten (die nach Methoden der pflanzlichen Biotechnologie, wie der Gentechnik, erhalten wurden), die ebenfalls erfindungsgemäß behandelt werden können, sind Pflanzen wie Baumwollpflanzen mit veränderten Fasereigenschaften. Solche Pflanzen können durch genetische Transformation oder durch Selektion von Pflanzen, die eine Mutation enthalten, die solche veränderten Fasereigenschaften verleiht, erhalten werden; dazu zählen:
a) Pflanzen wie Baumwollpflanzen, die eine veränderte Form von Cellulosesynthasegenen enthalten,
b) Pflanzen wie Baumwollpflanzen, die eine veränderte Form von rsw2- oder rsw3-homologen Nukleinsäuren enthalten;
c) Pflanzen wie Baumwollpflanzen mit einer erhöhten Expression der Saccharosephosphatsynthase;
d) Pflanzen wie Baumwollpflanzen mit einer erhöhten Expression der Saccharosesynthase;
e) Pflanzen wie Baumwollpflanzen bei denen der Zeitpunkt der Durchlaßsteuerung der Plasmodesmen an der Basis der Faserzelle verändert ist, z. B. durch Herunterregulieren der faserselektiven β-1,3-Glucanase;
f) Pflanzen wie Baumwollpflanzen mit Fasern mit veränderter Reaktivität, z. B. durch Expression des N-Acetylglucosamintransferasegens, darunter auch nodC, und von Chitinsynthasegenen.

Pflanzen oder Pflanzensorten (die nach Methoden der pflanzlichen Biotechnologie, wie der Gentechnik, erhalten wurden), die ebenfalls erfindungsgemäß behandelt werden können, sind Pflanzen wie Raps oder verwandte Brassica-Pflanzen mit veränderten Eigenschaften der Ölzusammensetzung. Solche Pflanzen können durch genetische Transformation oder durch Selektion von Pflanzen, die eine Mutation enthalten, die solche veränderten Öleigenschaften verleiht, erhalten werden; dazu zählen:
a) Pflanzen wie Rapspflanzen, die Öl mit einem hohen Ölsäuregehalt produziere;
b) Pflanzen wie Rapspflanzen, die Öl mit einem niedrigen Linolensäuregehalt produzieren.
c) Pflanzen wie Rapspflanzen, die Öl mit einem niedrigen gesättigten Fettsäuregehalt produzieren.

Besonders nützliche transgene Pflanzen, die erfindungsgemäß behandelt werden können, sind Pflanzen mit einem oder mehreren Genen, die für ein oder mehrere Toxine kodieren, sind die transgenen Pflanzen, die unter den folgenden Handelsbezeichnungen angeboten werden: YIELD GARD® (zum Beispiel Mais, Baumwolle, Sojabohnen), KnockOut® (zum Beispiel Mais), BiteGard® (zum Beispiel Mais), BT-Xtra® (zum Beispiel Mais), StarLink® (zum Beispiel Mais), Bollgard® (Baumwolle), Nucotn® (Baumwolle), Nucotn 33B® (Baumwolle), NatureGard® (zum Beispiel Mais), Protecta® und NewLeaf® (Kartoffel). Herbizidtolerante Pflanzen, die zu erwähnen sind, sind zum Beispiel Maissorten, Baumwollsorten und Sojabohnensorten, die unter den folgenden Handelsbezeichnungen angeboten werden: Roundup Ready® (Glyphosatetoleranz, zum Beispiel Mais, Baumwolle, Sojabohne), Liberty Link® (Phosphinotricintoleranz, zum Beispiel Raps), IMI® (Imidazolinontoleranz) und SCS® (Sylfonylharnstofftoleranz), zum Beispiel Mais. Zu den herbizidresistenten Pflanzen (traditionell auf Herbizidtoleranz gezüchtete Pflanzen), die zu erwähnen sind, zählen die unter der Bezeichnung Clearfield® angebotenen Sorten (zum Beispiel Mais).

Besonders nützliche transgene Pflanzen, die erfindungsgemäß behandelt werden können, sind Pflanzen, die Transformations-Events, oder eine Kombination von Transformations-Events, enthalten und die zum Beispiel in den Dateien von verschiedenen nationalen oder regionalen Behörden angeführt sind (siehe zum Beispiel http://gmoinfo.jrc.it/gmp_browse.aspx und http://www.agbios.com/dbase.php).

Die aufgeführten Pflanzen können besonders vorteilhaft erfindungsgemäß mit den Verbindungen der allgemeinen Formel I bzw. den erfindungsgemäßen Wirkstoffmischungen behandelt werden. Die bei den Wirkstoffen bzw. Mischungen oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mischungen.

Die Bekämpfung von von tierischen Schädlingen und/oder pflanzenpathogenen Schadpilzen durch die Behandlung des Saatguts von Pflanzen ist seit langem bekannt und ist Gegenstand ständiger Verbesserungen. Dennoch ergeben sich bei der Behandlung von Saatgut eine Reihe von Problemen, die nicht immer zufrieden stellend gelöst werden können. So ist es erstrebenswert, Verfahren zum Schutz des Saatguts und der keimenden Pflanze zu entwickeln, die das zusätzliche Ausbringen von Pflanzenschutzmitteln nach der Saat oder nach dem Auflaufen der Pflanzen überflüssig machen oder zumindest deutlich verringern. Es ist weiterhin erstrebenswert, die Menge des eingesetzten Wirkstoffs dahingehend zu optimieren, dass das Saatgut und die keimende Pflanze vor dem Befall durch tierische Schädlinge und/oder pflanzenpathogene Schadpilze bestmöglich geschützt werden, ohne jedoch die Pflanze selbst durch den eingesetzten Wirkstoff zu schädigen. Insbesondere sollten Verfahren zur Behandlung von Saatgut auch die intrinsischen fungiziden Eigenschaften transgener Pflanzen einbeziehen, um einen optimalen Schutz des Saatguts und der keimenden Pflanze bei einem minimalen Aufwand an Pflanzenschutzmitteln zu erreichen.

Die vorliegende Erfindung bezieht sich daher insbesondere auch auf ein Verfahren zum Schutz von Saatgut und keimenden Pflanzen vor dem Befall von tierischen Schädlingen und/oder pflanzenpathogenen Schadpilzen, indem das Saatgut mit einem erfindungsgemäßen Mittel behandelt wird.

Die Erfindung bezieht sich ebenfalls auf die Verwendung der erfindungsgemäßen Mittel zur Behandlung von Saatgut zum Schutz des Saatguts und der keimenden Pflanze vor tierischen Schädlingen und/oder pflanzenpathogenen Schadpilzen.

Weiterhin bezieht sich die Erfindung auf Saatgut, welches zum Schutz vor tierischen Schädlingen und/oder pflanzenpathogenen Schadpilzen mit einem erfindungsgemäßen Mittel behandelt wurde.

Einer der Vorteile der vorliegenden Erfindung ist es, dass aufgrund der besonderen systemischen Eigenschaften der erfindungsgemäßen Mittel die Behandlung des Saatguts mit diesen Mitteln nicht nur das Saatgut selbst, sondern auch die daraus hervorgehenden Pflanzen nach dem Auflaufen vor tierischen Schädlingen und/oder pflanzenpathogenen Schadpilzen schützt. Auf diese Weise kann die unmittelbare Behandlung der Kultur zum Zeitpunkt der Aussaat oder kurz danach entfallen.

Ebenso ist es als vorteilhaft anzusehen, dass die erfindungsgemäßen Mischungen insbesondere auch bei transgenem Saatgut eingesetzt werden können.

Die erfindungsgemäßen Mittel eignen sich zum Schutz von Saatgut jeglicher Pflanzensorte, die in der Landwirtschaft, im Gewächshaus, in Forsten oder im Gartenbau eingesetzt wird. Insbesondere handelt es sich dabei um Saatgut von Getreide (wie Weizen, Gerste, Roggen, Hirse und Hafer), Mais, Baumwolle, Soja, Reis, Kartoffeln, Sonnenblume, Bohne, Kaffee, Rübe (z.B. Zuckerrübe und Futterrübe), Erdnuss, Gemüse (wie Tomate, Gurke, Zwiebeln und Salat), Rasen und Zierpflanzen. Besondere Bedeutung kommt der Behandlung des Saatguts von Getreide (wie Weizen, Gerste, Roggen und Hafer), Mais und Reis zu.

Im Rahmen der vorliegenden Erfindung wird das erfindungsgemäße Mittel alleine oder in einer geeigneten Formulierung auf das Saatgut aufgebracht. Vorzugsweise wird das Saatgut in einem Zustand behandelt, in dem so stabil ist, dass keine Schäden bei der Behandlung auftreten. Im Allgemeinen kann die Behandlung des Saatguts zu jedem Zeitpunkt zwischen der Ernte und der Aussaat erfolgen. Üblicherweise wird Saatgut verwendet, das von der Pflanze getrennt und von Kolben, Schalen, Stängeln, Hülle, Wolle oder Fruchtfleisch befreit wurde. So kann zum Beispiel Saatgut verwendet werden, das geerntet, gereinigt und bis zu einem Feuchtigkeitsgehalt von unter 15 Gew.-% getrocknet wurde. Alternativ kann auch Saatgut verwendet werden, das nach dem Trocknen z.B. mit Wasser behandelt und dann erneut getrocknet wurde.

Im Allgemeinen muss bei der Behandlung des Saatguts darauf geachtet werden, dass die Menge des auf das Saatgut aufgebrachten erfindungsgemäßen Mittels und/oder weiterer Zusatzstoffe so gewählt wird, dass die Keimung des Saatguts nicht beeinträchtigt bzw. die daraus hervorgehende Pflanze nicht geschädigt wird. Dies ist vor allem bei Wirkstoffen zu beachten, die in bestimmten Aufwandmengen phytotoxische Effekte zeigen können.

Die erfindungsgemäßen Mittel können unmittelbar aufgebracht werden, also ohne weitere Komponenten zu enthalten und ohne verdünnt worden zu sein. In der Regel ist es vorzuziehen, die Mittel in Form einer geeigneten Formulierung auf das Saatgut aufzubringen. Geeignete Formulierungen und Verfahren für die Saatgutbehandlung sind dem Fachmann bekannt und werden z.B. in den folgenden Dokumenten beschrieben: US 4,272,417 A, US 4,245,432 A, US 4,808,430 A, US 5,876,739 A, US 2003/0176428 A1, WO 2002/080675 A1, WO 2002/028186 A2.

Die erfindungsgemäß verwendbaren Wirkstoffkombinationen können in die üblichen Beizmittel-Formulierungen überführt werden, wie Lösungen, Emulsionen, Suspensionen, Pulver, Schäume, Slurries oder andere Hüllmassen für Saatgut, sowie ULV-Formulierungen.

Diese Formulierungen werden in bekannter Weise hergestellt, indem man die Wirkstoffe oder Wirkstoffkombinationen mit üblichen Zusatzstoffen vermischt, wie zum Beispiel übliche Streckmittel sowie Lösungs- oder Verdünnungsmittel, Farbstoffe, Netzmittel, Dispergiermittel, Emulgatoren, Entschäumer, Konservierungsmittel, sekundäre Verdickungsmittel, Kleber, Gibberelline und auch Wasser.

Als Farbstoffe, die in den erfindungsgemäß verwendbaren Beizmittel-Formulierungen enthalten sein können, kommen alle für derartige Zwecke üblichen Farbstoffe in Betracht. Dabei sind sowohl in Wasser wenig lösliche Pigmente als auch in Wasser lösliche Farbstoffe verwendbar. Als Beispiele genannt seien die unter den Bezeichnungen Rhodamin B, C.I. Pigment Red 112 und C.I. Solvent Red 1 bekannten Farbstoffe.

Als Netzmittel, die in den erfindungsgemäß verwendbaren Beizmittel-Formulierungen enthalten sein können, kommen alle zur Formulierung von agrochemischen Wirkstoffen üblichen, die Benetzung fördernden Stoffe in Frage. Vorzugsweise verwendbar sind Alkylnaphthalin-Sulfonate, wie Diisopropyl- oder Diisobutyl-naphthalin-Sulfonate.

Als Dispergiermittel und/oder Emulgatoren, die in den erfindungsgemäß verwendbaren Beizmittel-Formulierungen enthalten sein können, kommen alle zur Formulierung von agrochemischen Wirkstoffen üblichen nichtionischen, anionischen und kationischen Dispergiermittel in Betracht. Vorzugsweise verwendbar sind nichtionische oder anionische Dispergiermittel oder Gemische von nichtionischen oder anionischen Dispergiermitteln. Als geeignete nichtionische Dispergiermittel sind insbesondere Ethylenoxid-Propylenoxid Blockpolymere, Alkylphenolpolyglykolether sowie Tristryrylphenolpolyglykolether und deren phosphatierte oder sulfatierte Derivate zu nennen. Geeignete anionische Dispergiermittel sind insbesondere Ligninsulfonate, Polyacrylsäuresalze und Arylsulfonat-Formaldehydkondensate.

Als Entschäumer können in den erfindungsgemäß verwendbaren Beizmittel-Formulierungen alle zur Formulierung von agrochemischen Wirkstoffen üblichen schaumhemmenden Stoffe enthalten sein. Vorzugsweise verwendbar sind Silikonentschäumer und Magnesiumstearat.

Als Konservierungsmittel können in den erfindungsgemäß verwendbaren Beizmittel-Formulierungen alle für derartige Zwecke in agrochemischen Mitteln einsetzbaren Stoffe vorhanden sein. Beispielhaft genannt seien Dichlorophen und Benzylalkoholhemiformal.

Als sekundäre Verdickungsmittel, die in den erfindungsgemäß verwendbaren Beizmittel-Formulierungen enthalten sein können, kommen alle für derartige Zwecke in agrochemischen Mitteln einsetzbaren Stoffe in Frage. Vorzugsweise in Betracht kommen Cellulosederivate, Acrylsäurederivate, Xanthan, modifizierte Tone und hochdisperse Kieselsäure.

Als Kleber, die in den erfindungsgemäß verwendbaren Beizmittel-Formulierungen enthalten sein können, kommen alle üblichen in Beizmitteln einsetzbaren Bindemittel in Frage. Vorzugsweise genannt seien Polyvinylpyrrolidon, Polyvinylacetat, Polyvinylalkohol und Tylose.

Als Gibberelline, die in den erfindungsgemäß verwendbaren Beizmittel-Formulierungen enthalten sein können, kommen vorzugsweise die Gibberelline A1, A3 (= Gibberellinsäure), A4 und A7 infrage, be-sonders bevorzugt verwendet man die Gibberellinsäure. Die Gibberelline sind bekannt (vgl. R. Wegler "Chemie der Pflanzenschutz- und Schädlingsbekämpfungsmittel", Bd. 2, Springer Verlag, 1970, S. 401-412).

Die erfindungsgemäß verwendbaren Beizmittel-Formulierungen können entweder direkt oder nach vorherigem Verdünnen mit Wasser zur Behandlung von Saatgut der verschiedensten Art eingesetzt werden. So lassen sich die Konzentrate oder die daraus durch Verdünnen mit Wasser erhältlichen Zubereitungen einsetzen zur Beizung des Saatgutes von Getreide, wie Weizen, Gerste, Roggen, Hafer und Triticale, sowie des Saatgutes von Mais, Reis, Raps, Erbsen, Bohnen, Baumwolle, Sonnenblumen und Rüben oder auch von Gemüsesaatgut der verschiedensten Natur. Die erfindungsgemäß verwendbaren Beizmittel-Formulierungen oder deren verdünnte Zubereitungen können auch zum Beizen von Saatgut transgener Pflanzen eingesetzt werden. Dabei können im Zusammenwirken mit den durch Expression gebildeten Substanzen auch zusätzliche synergistische Effekte auftreten.

Zur Behandlung von Saatgut mit den erfindungsgemäß verwendbaren Beizmittel-Formulierungen oder den daraus durch Zugabe von Wasser hergestellten Zubereitungen kommen alle üblicherweise für die Beizung einsetzbaren Mischgeräte in Betracht. Im einzelnen geht man bei der Beizung so vor, dass man das Saatgut in einen Mischer gibt, die jeweils gewünschte Menge an Beizmittel-Formulierungen entweder als solche oder nach vorherigem Verdünnen mit Wasser hinzufügt und bis zur gleichmäßigen Verteilung der Formulierung auf dem Saatgut mischt. Gegebenenfalls schließt sich ein Trocknungsvorgang an.

Die Aufwandmenge an den erfindungsgemäß verwendbaren Beizmittel-Formulierungen kann innerhalb eines größeren Bereiches variiert werden. Sie richtet sich nach dem jeweiligen Gehalt der Wirkstoffe in den Formulierungen und nach dem Saatgut. Die Aufwandmengen an Wirkstoffkombination liegen im Allgemeinen zwischen 0,001 und 50 g pro Kilogramm Saatgut, vorzugsweise zwischen 0,01 und 15 g pro Kilogramm Saatgut.

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ekto- und Endoparasiten) wie Schildzecken, Lederzecken, Räudemilben, Laufmilben, Fliegen (stechend und leckend), parasitierende Fliegenlarven, Läuse, Haarlinge, Federlinge und Flöhe. Zu diesen Parasiten gehören:
Aus der Ordnung der Anoplurida z.B. Haematopinus spp., Linognathus spp., Pediculus spp., Phtirus spp., Solenopotes spp..
Aus der Ordnung der Mallophagida und den Unterordnungen Amblycerina sowie Ischnocerina z.B. Trimenopon spp., Menopon spp., Trinoton spp., Bovicola spp., Werneckiella spp., Lepikentron spp., Damalina spp., Trichodectes spp., Felicola spp..
Aus der Ordnung Diptera und den Unterordnungen Nematocerina sowie Brachycerina z.B. Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp..
Aus der Ordnung der Siphonapterida z.B. Pulex spp., Ctenocephalides spp., Xenopsylla spp., Ceratophyllus spp..
Aus der Ordnung der Heteropterida z.B. Cimex spp., Triatoma spp., Rhodnius spp., Panstrongylus spp..
Aus der Ordnung der Blattarida z.B. Blatta orientalis, Periplaneta americana, Blattela germanica, Supella spp..
Aus der Unterklasse der Acari (Acarina) und den Ordnungen der Meta- sowie Mesostigmata z.B. Argas spp., Ornithodorus spp., Otobius spp., Ixodes spp., Amblyomma spp., Boophilus spp., Dermacentor spp., Haemophysalis spp., Hyalomma spp., Rhipicephalus spp., Dermanyssus spp., Raillietia spp., Pneumonyssus spp., Sternostoma spp., Varroa spp..
Aus der Ordnung der Actinedida (Prostigmata) und Acaridida (Astigmata) z.B. Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp..

Die erfindungsgemäßen Wirkstoffe der Formel (I) eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so daß durch den Einsatz der erfindungsgemäßen Wirkstoffe eine wirtschaftlichere und einfachere Tierhaltung möglich ist.

Die Anwendung der erfindungsgemäßen Wirkstoffe geschieht im Veterinärsektor und bei der Tierhaltung in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw.

Bei der Anwendung für Vieh, Geflügel, Haustiere etc. kann man die Wirkstoffe der Formel (I) als Formulierungen (beispielsweise Pulver, Emulsionen, fließfähige Mittel), die die Wirkstoffe in einer Menge von 1 bis 80 Gew.-% enthalten, direkt oder nach 100 bis 10 000-facher Verdünnung anwenden oder sie als chemisches Bad verwenden.

Außerdem wurde gefunden, daß die erfindungsgemäßen Verbindungen eine hohe insektizide Wirkung gegen Insekten zeigen, die technische Materialien zerstören.

Beispielhaft und vorzugsweise - ohne jedoch zu limitieren - seien die folgenden Insekten genannt:
Käfer wie Hylotrupes bajulus, Chlorophorus pilosis, Anobium punctatum, Xestobium rufovillosum, Ptilinus pecticornis, Dendrobium pertinex, Ernobius mollis, Priobium carpini, Lyctus brunneus, Lyctus africanus, Lyctus planicollis, Lyctus linearis, Lyctus pubescens, Trogoxylon aequale, Minthes rugicollis, Xyleborus spec. Tryptodendron spec. Apate monachus, Bostrychus capucins, Heterobostrychus brunneus, Sinoxylon spec. Dinoderus minutus;
Hautflügler wie Sirex juvencus, Urocerus gigas, Urocerus gigas taignus, Urocerus augur;
Termiten wie Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis, Coptotermes formosanus;
Borstenschwänze wie Lepisma saccharina.

Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz, Holzverarbeitungsprodukte und Anstrichmittel.

Die anwendungsfertigen Mittel können gegebenenfalls noch weitere Insektizide und gegebenenfalls noch ein oder mehrere Fungizide enthalten.

Hinsichtlich möglicher zusätzlicher Zumischpartner sei auf die oben genannten Insektizide und Fungizide verwiesen.

Zugleich können die erfindungsgemäßen Verbindungen zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, eingesetzt werden.

Weiter können die erfindungsgemäßen Verbindungen allein oder in Kombinationen mit anderen Wirkstoffen als Antifouling-Mittel eingesetzt werden.

Die Wirkstoffe eignen sich auch zur Bekämpfung von tierischen Schädlingen im Haushalts-, Hygiene- und Vorratsschutz, insbesondere von Insekten, Spinnentieren und Milben, die in geschlossenen Räumen, wie beispielsweise Wohnungen, Fabrikhallen, Büros, Fahrzeugkabinen u.ä. vorkommen. Sie können zur Bekämpfung dieser Schädlinge allein oder in Kombination mit anderen Wirk- und Hilfsstoffen in Haushaltsinsektizid-Produkten verwendet werden. Sie sind gegen sensible und resistente Arten sowie gegen alle Entwicklungsstadien wirksam. Zu diesen Schädlingen gehören:
Aus der Ordnung der Scorpionidea z.B. Buthus occitanus.
Aus der Ordnung der Acarina z.B. Argas persicus, Argas reflexus, Bryobia ssp., Dermanyssus gallinae, Glyciphagus domesticus, Ornithodorus moubat, Rhipicephalus sanguineus, Trombicula alfreddugesi, Neutrombicula autumnalis, Dermatophagoides pteronissimus, Dermatophagoides forinae.
Aus der Ordnung der Araneae z.B. Aviculariidae, Araneidae.
Aus der Ordnung der Opiliones z.B. Pseudoscorpiones chelifer, Pseudoscorpiones cheiridium, Opiliones phalangium.
Aus der Ordnung der Isopoda z.B. Oniscus asellus, Porcellio scaber.
Aus der Ordnung der Diplopoda z.B. Blaniulus guttulatus, Polydesmus spp..
Aus der Ordnung der Chilopoda z.B. Geophilus spp..
Aus der Ordnung der Zygentoma z.B. Ctenolepisma spp., Lepisma saccharina, Lepismodes inquilinus.
Aus der Ordnung der Blattaria z.B. Blatta orientalies, Blattella germanica, Blattella asahinai, Leucophaea maderae, Panchlora spp., Parcoblatta spp., Periplaneta australasiae, Periplaneta americana, Periplaneta brunnea, Periplaneta fuliginosa, Supella longipalpa.
Aus der Ordnung der Saltatoria z.B. Acheta domesticus.
Aus der Ordnung der Dermaptera z.B. Forficula auricularia.
Aus der Ordnung der Isoptera z.B. Kalotermes spp., Reticulitermes spp.
Aus der Ordnung der Psocoptera z.B. Lepinatus spp., Liposcelis spp.
Aus der Ordnung der Coleoptera z.B. Anthrenus spp., Attagenus spp., Dermestes spp., Latheticus oryzae, Necrobia spp., Ptinus spp., Rhizopertha dominica, Sitophilus granarius, Sitophilus oryzae, Sitophilus zeamais, Stegobium paniceum.
Aus der Ordnung der Diptera z.B. Aedes aegypti, Aedes albopictus, Aedes taeniorhynchus, Anopheles spp., Calliphora erythrocephala, Chrysozona pluvialis, Culex quinquefasciatus, Culex pipiens, Culex tarsalis, Drosophila spp., Fannia canicularis, Musca domestica, Phlebotomus spp., Sarcophaga carnaria, Simulium spp., Stomoxys calcitrans, Tipula paludosa.
Aus der Ordnung der Lepidoptera z.B. Achroia grisella, Galleria mellonella, Plodia interpunctella, Tinea cloacella, Tinea pellionella, Tineola bisselliella.
Aus der Ordnung der Siphonaptera z.B. Ctenocephalides canis, Ctenocephalides felis, Pulex irritans, Tunga penetrans, Xenopsylla cheopis.
Aus der Ordnung der Hymenoptera z.B. Camponotus herculeanus, Lasius fuliginosus, Lasius niger, Lasius umbratus, Monomorium pharaonis, Paravespula spp., Tetramorium caespitum.
Aus der Ordnung der Anoplura z.B. Pediculus humanus capitis, Pediculus humanus corporis, Pemphigus spp., Phylloera vastatrix, Phthirus pubis.
Aus der Ordnung der Heteroptera z.B. Cimex hemipterus, Cimex lectularius, Rhodinus prolixus, Triatoma infestans.

Die Anwendung im Bereich der Haushaltsinsektizide erfolgt allein oder in Kombination mit anderen geeigneten Wirkstoffen wie Phosphorsäureestern, Carbamaten, Pyrethroiden, Neonicotinoiden, Wachstumsregulatoren oder Wirkstoffen aus anderen bekannten Insektizidklassen.

Die Anwendung erfolgt in Aerosolen, drucklosen Sprühmitteln, z.B. Pump- und Zerstäubersprays, Nebelautomaten, Foggern, Schäumen, Gelen, Verdampferprodukten mit Verdampferplättchen aus Cellulose oder Kunststoff, Flüssigverdampfern, Gel- und Membranverdampfern, propellergetriebenen Verdampfern, energielosen bzw. passiven Verdampfungssystemen, Mottenpapieren, Mottensäckchen und Mottengelen, als Granulate oder Stäube, in Streuködern oder Köderstationen.

Die erfindungsgemäßen Wirkstoffe der Formel (I) können gegebenenfalls in bestimmten Konzentrationen und Aufwandmengen auch als Herbizide, zur Beeinflussung des Pflanzenwachstums, sowie zur Bekämpfung von unerwünschten Mikroorganismen, wie Fungi und Bakterien, im Pflanzenschutz und im Materialschutz eingesetzt werden. Sie lassen sich gegebenenfalls auch als Zwischen- und Vorprodukte für die Synthese weiterer Wirkstoffe einsetzen.

Darüber hinaus kann durch die erfindungsgemäße Behandlung mit den erfindungsgemäßen Verbindungen der Mykotoxingehalt im Erntegut und den daraus hergestellten Nahrungs- und Futtermitteln verringert werden. Besonders, aber nicht ausschließlich sind hierbei folgende Mykotoxine zu nennen: Deoxynivalenol (DON), Nivalenol, 15-Ac-DON, 3-Ac-DON, T2- und HT2- Toxin, Fumonisine, Zearalenon, Moniliformin, Fusarin, Diaceotoxyscirpenol (DAS), Beauvericin, Enniatin, Fusaroproliferin, Fusarenol, Ochratoxine, Patulin, Mutterkornalkaloide und Aflatoxine, die beispielsweise von den folgenden Pilzen verursacht werden können: Fusarium spec., wie Fusarium acuminatum, F. avenaceum, F. crookwellense, F. culmorum, F. graminearum (Gibberella zeae), F. equiseti, F. fujikoroi, F. musarum, F. oxysporum, F. proliferatum, F. poae, F. pseudograminearum, F. sambucinum, F. scirpi, F. semitectum, F. solani, F. sporotrichoides, F. langsethiae, F. subglutinans, F. tricinctum, F. verticillioides u.a. sowie auch von Aspergillus spec., Penicillium spec., Claviceps purpurea, Stachybotrys spec. u.a.

Weiterhin eignen sich die erfindungsgemäßen Verbindungen der Formel (I) zur Unterdrückung des Wachstums von Tumorzellen in Menschen und Säugetieren. Dies basiert auf einer Wechselwirkung der erfindungsgemäßen Verbindungen mit Tubulin.

Zu diesem Zweck kann man eine wirksame Menge an einer oder mehreren Verbindungen der Formel (I) oder pharmazeutisch verträglicher Salze davon verabreichen.

Die Herstellung und die Verwendung der erfindungsgemäßen Wirkstoffe gehen aus den folgenden Beispielen hervor.

### Herstellungsbeispiele

### Beispiel I-1

### 1-[4-Chlor-5-(2-chlorphenyl)-6-(isopropylamino)pyrimidin-2-yl]-N-(methylsulfonyl)-1H-pyrazol-4-carboxamid

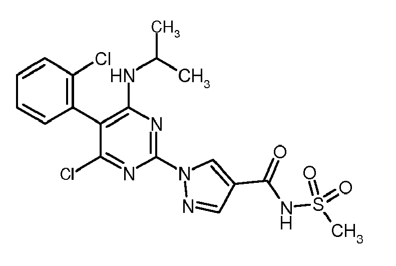

a) 35 mg Natriumhydrid (95%-ig) wurden in 15 mL THF vorgelegt. Bei 0 °C wurden 129 mg Pyrazol-4-carbonitril zugegeben und für 1 h bei Raumtemperatur gerührt. Es wurden 500 mg 4-Chlor-5-(2-chlorphenyl)-6-isopropylamino-2-methylsulfonyl-pyrimidin gelöst in 2 mL THF zugegeben. Das Gemisch wurde für 16 h bei Raumtemperatur gerührt. Die Reaktionsmischung wurde in Wasser eingerührt und mit Essigsäure-ethylester extrahiert. Von der organischen Phase wurde das Lösungsmittel im Vakuum entfernt. Es wurden 540 mg (Reinheit 95%) 1-[4-Chlor-5-(2-chlorphenyl)-6-(isopropylamino)pyrimidin-2-yl]-1H-pyrazol-4-carbonitril (IV-1) erhalten.
   ¹H-NMR (DMSO-d₆): δ = 9.45 (s, 1H), 8.38 (s, 1H), 7.65 (dd, 1H), 7.52 (m, 2 H), 7.40 (dd, 1H), 6.87 (bd, 1 H), 4.54 (m, 1H), 1.13 (m, 6H)
b) 1,19 g 1-[4-Chlor-5-(2-chlorphenyl)-6-(isopropylamino)pyrimidin-2-yl]-1H-pyrazol-4-carbonitril wurden in 30 mL Ethanol vorgelegt und mit 6 ml (35%-ig) WasserstoffperoxidLösung und 30 ml konzentrierter Ammoniaklösung versetzt. Das Gemisch wurde für 8 h bei 50 °C gerührt. Die Reaktionsmischung wurde in Wasser eingerührt und mit Essigsäure-ethylester extrahiert. Von der organischen Phase wurde das Lösungsmittel im Vakuum entfernt. Es wurden 1,1 g 1-[4-Chlor-5-(2-chlorphenyl)-6-(isopropylamino)pyrimidin-2-yl]-1H-pyrazol-4-carboxamid (V-1) erhalten.
   ¹H-NMR (DMSO-d₆): δ = 9.02 (s, 1H), 8.11 (s, 1H), 7.62 (dd, 1H), 7.50 (m, 2 H), 7.39 (dd, 1H), 6.40 (bd, 1 H), 4.48 (m, 1H), 1.16 (m, 6H)
c) 17 mg Natriumhydrid (95%-ig) wurden in 15 mL THF vorgelegt. 200 mg 1-[4-Chlor-5-(2-chlorphenyl)-6-(isopropylamino)pyrimidin-2-yl]-1H-pyrazol-4-carboxamid in 2 mL THF wurden zugegeben und 1 h bei Raumtemperatur gerührt. Es wurden 117 mg Methansulfonylchlorid zugegeben und für 16 h bei Raumtemperatur gerührt. Es wurden weitere 17 mg Natriumhydrid (95%-ig) zugegeben und für 24 h bei 50 °C gerührt. Die Reaktionsmischung wurde in Wasser eingerührt und mit Essigsäure-ethylester extrahiert. Von der organischen Phase wurde das Lösungsmittel im Vakuum entfernt. Der Rückstand wurde chromatografisch gereinigt (Kieselgel, Methylenchlorid/Methanol 5:1). Man erhielt 70 mg 1-[4-Chlor-5-(2-chlorphenyl)-6-(isopropylamino)pyrimidin-2-yl]-N-(methylsulfonyl)-1H-pyrazol-4-carboxamid.

### Beispiel I-2

### 1-[4-Chlor-5-(2-chlorphenyl)-6-(isopropylamino)pyrimidin-2-yl]-N-(methylsulfonyl)-1H-pyrazol-3-carboxamid

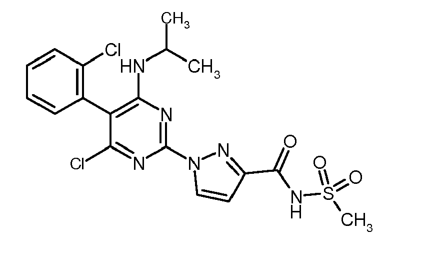

a) 250 mg 4-Chlor-5-(2-chlorphenyl)-6-isopropylamino-2-methylsulfinyl-pyrimidin und 78 mg Pyrazol-3-carbonsäure wurden in 10 mL Pyridin für 16 h bei 100 °C gerührt. Die Reaktionsmischung wurde in 1N Salzsäure eingerührt und mit Methylenchlorid extrahiert. Von der organischen Phase wurde das Lösungsmittel im Vakuum entfernt. Man erhielt 250 mg 1-[4-Chlor-5-(2-chlorphenyl)-6-(isopropylamino)pyrimidin-2-yl]-1H-pyrazol-3-carbonsäure (X-1).
   ¹H-NMR (DMSO-d₆): δ = 8.62 (d, 1H), 7.63 (dd, 1H), 7.51 (m, 2 H), 7.39 (dd, 1H), 6.91 (d, 1H), 6.43 (bd, 1 H), 4.48 (m, 1H), 1.16 (m, 6H)
b) 110 mg 1-[4-Chlor-5-(2-chlorphenyl)-6-(isopropylamino)pyrimidin-2-yl]-1H-pyrazol-3-carbonsäure und 80 mg Methansulfonamid wurden in 10 mL Pyridin vorgelegt. Es wurden 64 mg Phosphorylchlorid zugegeben. Die Mischung wurde für 16 h bei 80 °C gerührt. Die Reaktionsmischung wurde in Wasser eingerührt und mit Essigsäure-ethylester extrahiert. Von der organischen Phase wurde das Lösungsmittel im Vakuum entfernt. Der Rückstand wurde chromatografisch gereinigt (Kieselgel, Essigsäure-ethylester). Man erhielt 20 mg 1-[4-Chlor-5-(2-chlorphenyl)-6-(isopropylamino)pyrimidin-2-yl]-N-(methylsulfonyl)-1H-pyrazol-3-carboxamid.

### Beispiel I-3

### 1-[4-Chlor-5-(2-chlorphenyl)-6-(isopropylamino)pyrimidin-2-yl]-5-methyl-N-(methylsulfonyl)-1H-pyrazol-3-carboxamid

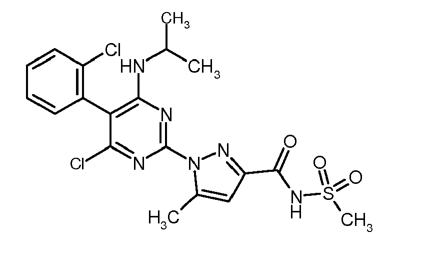

a) 500 mg 4-Chlor-5-(2-chlorphenyl)-6-isopropylamino-2-methylsulfonyl-pyrimidin, 214 mg 5-Methyl-1H-pyrazol-3-carbonsäure-ethylester (CAS 4027-57-0) und 384 mg Kaliumcarbonat wurden in 20 mL DMF für 16 h bei 80 °C gerührt. Die Reaktionsmischung wurde in Wasser eingerührt und mit Essigsäure-ethylester extrahiert. Von der organischen Phase wurde das Lösungsmittel im Vakuum entfernt. Man erhielt 740 mg (Reinheit 81%) 1-[4-Chlor-5-(2-chlorphenyl)-6-(isopropylamino)pyrimidin-2-yl]-5-methyl-1H-pyrazol-3-carbonsäure-ethylester (IX-1).
¹H-NMR (DMSO-d₆): δ = 7.64 (dd, 1H), 7.51 (m, 2 H), 7.40 (dd, 1H), 6.73 (s, 1H), 6.44 (bd, 1 H), 4.28-4.40 (m, 3H), 2.64 (s, 3H), 1.33 (t, 3H), 1.14 (m, 6H)
Es wurden z. B. die folgenden weiteren Zwischenprodukte der Formel (IX) hergestellt:

| | | | | | |
|---|---|---|---|---|---|
| 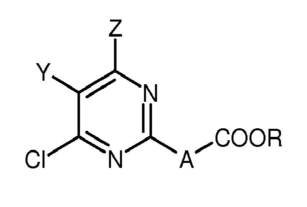 | | | | | |

| **Bsp.-Nr.** | **Y** | **Z** | **A** | **R** | **¹H-NMR (DMSO-d₆)** |
|---|---|---|---|---|---|
| IX-2 | Phenyl | NH-CH(CH₃)₂ | 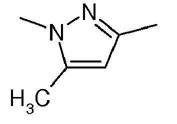 | C₂H₅ | 7.55 (m, 2H), 7.48 (m, 1 H), 7.34 (dd, 2H), 6.73 (s, 1H), 6.04 (bd, 1 H), 4.25-4.36 (m, 3H), 2.64 (s, 3H), 1.32 (t, 3H), 1.13 (d, 6H) |
| IX-3 | 2-C1-Phenyl | NH-CH₂CF₃ | 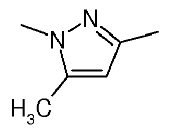 | C₂H₅ | 7.65 (dd, 1H), 7.52 (m, 2 H), 7.41 (dd, 1H), 7.18 (bt, 1H), 6.75 (s, 1H), 6.11 (tt, 1 H), 4.33 (q, 2H), 3.78 (m, 2H), 2.66 (s, 3H), 1.33 (t, 3H) |
| IX-4 | 2,4,6-F₃-Phenyl | NH-CH(CH₃)₂ | 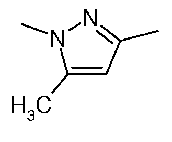 | C₂H₅ | 7.32 (dd, 2H), 7.15 (bd, 1 H), 6.74 (s, 1H), 4.30-4.41 (m, 3H), 2.65 (s, 3H), 1.32 (t, 3H), 1.16 (d, 6H) |

b) 230 mg 1-[4-Chlor-5-(2-chlorphenyl)-6-(isopropylamino)pyrimidin-2-yl]-5-methyl-1H-pyrazol-3-carbonsäure-ethylester wurden in 10 mL Dioxan vorgelegt und mit 10 mL 2N Kaliumhydroxid-Lösung versetzt. Es wurde für 16 h bei Raumtemperatur gerührt. Die Reaktionsmischung wurde mit 1N Salzsäure auf pH 1 eingestellt und mit Essigsäure-ethylester extrahiert. Von der organischen Phase wurde das Lösungsmittel im Vakuum entfernt. Man erhielt 230 mg (Reinheit 92%) 1-[4-Chlor-5-(2-chlorphenyl)-6-(isopropylamino)pyrimidin-2-yl]-5-methyl-1H-pyrazol-3-carbonsäure (X-2).
¹H-NMR (DMSO-d₆): δ = 7.63 (dd, 1H), 7.51 (m, 2 H), 7.40 (dd, 1H), 6.60 (s, 1H), 6.48 (bd, 1 H), 4.38 (m, 1H), 2.64 (s, 3 H), 1.14 (m, 6H)
Es wurden z. B. die folgenden weiteren Zwischenprodukte der Formel (X) hergestellt:

| | | | | |
|---|---|---|---|---|
| 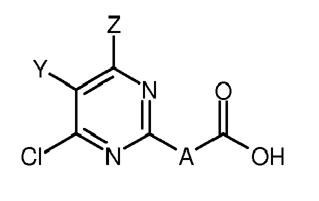 | | | | |

| **Bsp.-Nr.** | **Y** | **Z** | **A** | **¹H-NMR (DMSO-d₆)** |
|---|---|---|---|---|
| X-3 | Phenyl | NH-CH(CH₃)₂ | 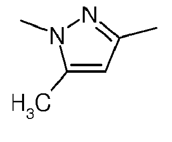 | 7.55 (m, 2H), 7.48 (m, 1 H), 7.34 (dd, 2H), 6.68 (s, 1H), 6.01 (bd, 1 H), 4.25-4.36 (m, 1H), 2.63 (s, 3H), 1.13 (d, 6H) |
| X-4 | 2-C1-Phenyl | NH-CH₂CF₃ | 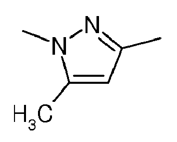 | 7.66 (dd, 1H), 7.52 (m, 2 H), 7.42 (dd, 1H), 7.15 (bt, 1H), 6.70 (s, 1H), 6.11 (tt, 1 H), 3.78 (m, 2H), 2.65 (s, 3H) |
| X-5 | 2,4,6-F₃-Phenyl | NH-CH(CH₃)₂ | 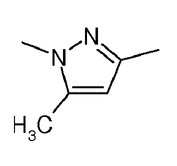 | 7.32 (dd, 2H), 7.12 (bd, 1 H), 6.69 (s, 1H), 4.33-4.41 (m, 1H), 2.65 (s, 3H), 1.16 (d, 6H) |

c) 260 mg 1-[4-Chlor-5-(2-chlorphenyl)-6-(isopropylamino)pyrimidin-2-yl]-5-methyl-1H-pyrazol-3-carbonsäure und 73 mg Methansulfonamid wurden in 10 mL Pyridin vorgelegt. Es wurden 147 mg Phosphorylchlorid zugegeben. Die Mischung wurde für 16 h bei 80 °C gerührt. Die Reaktionsmischung wurde in Wasser eingerührt und mit Essigsäure-ethylester extrahiert. Von der organischen Phase wurde das Lösungsmittel im Vakuum entfernt. Der Rückstand wurde chromatografisch gereinigt (Kieselgel, Dichlormethan/Methanol 10:1).Man erhielt 160 mg 1-[4-Chlor-5-(2-chlorphenyl)-6-(isopropylamino)pyrimidin-2-yl]-5-methyl-N-(methylsulfonyl)-1H-pyrazol-3-carboxamid.

Analog zu den zuvor angegebenen Methoden werden bzw. wurden auch die in der nachstehenden Tabelle 1 aufgeführten Verbindungen der Formel (I) erhalten.

**Tabelle 1**

| | | | | | | | |
|---|---|---|---|---|---|---|---|
| 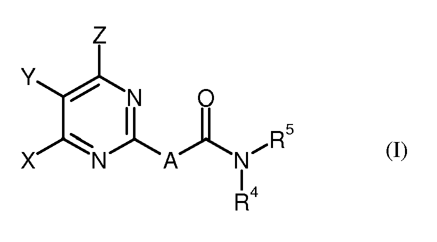 | | | | | | | |

| **Bsp.-Nr.** | **X** | **Y** | **Z** | **A** | **R⁵** | **R⁴** | **¹H-NMR (DMSO-d₆)** |
|---|---|---|---|---|---|---|---|
| I-1 | Cl | 2-Cl-Phenyl | NH-CH(CH₃)₂ | 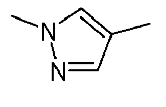 | SO₂-CH₃ | H | 9.14 (s, 1H), 8.17 (s, 1H), 7.63 (dd, 1H), 7.51 (m, 2 H), 7.39 (dd, 1H), 6.44 (bd, 1 H), 4.47 (m, 1H), 3.23 (s, 3 H), 1.17 (m, 6H) |
| I-2 | Cl | 2-Cl-Phenyl | NH-CH(CH₃)₂ | 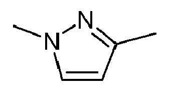 | SO₂-CH₃ | H | 8.69 (d, 1H), 7.63 (dd, 1H), 7.51 (m, 2 H), 7.39 (dd, 1H), 7.09 (d, 1H), 6.51 (bd, 1 H), 4.48 (m, 1H), 3.35 (s, 3 H), 1.16 (m, 6H) |
| I-3 | Cl | 2-Cl-Phenyl | NH-CH(CH₃)₂ | 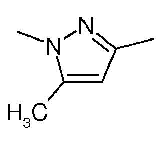 | SO₂-CH₃ | H | 7.63 (dd, 1H), 7.51 (m, 2 H), 7.40 (dd, 1H), 6.83 (s, 1H), 6.48 (bd, 1 H), 4.37 (m, 1H), 3.32 (s, 3 H), 2.66 (s, 3H), 1.14 (m, 6H) |
| I-4 | Cl | Phenyl | NH-CH(CH₃)₂ | 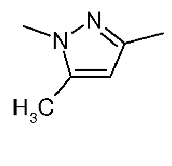 | SO₂-CH₃ | H | 7.54 (m, 2H), 7.48 (m, 1 H), 7.34 (dd, 2H), 6.84 (s, 1H), 6.08 (bd, 1 H), 4.25-4.35 (m, 1H), 3.33 (s, 3H), 2.66 (s, 3H), 1.14 (d, 6H) |
| I-5 | Cl | 2-Cl-Phenyl | NH-CH₂CF₃ | 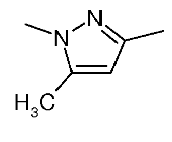 | SO₂-CH₃ | H | 7.66 (dd, 1H), 7.52 (m, 2 H), 7.42 (dd, 1H), 7.21 (bt, 1H), 6.86 (s, 1H), 6.12 (tt, 1 H), 3.78 (m, 2H), 3.34 (s, 3H), 2.67 (s, 3H) |
| I-6 | Cl | 2,4,6-F₃-Phenyl | NH-CH(CH₃)₂ | 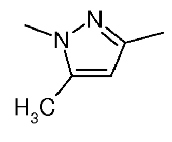 | SO₂-CH₃ | H | 8.56 (d, 1H), 7.35 (dd, 2H), 7.14 (bd, 1 H), 6.78 (s, 1H), 4.33-4.41 (m, 1H), 3.25 (s, 3H), 2.65 (s, 3H), 1.16 (d, 6H) |

| | | | | | | | |
|---|---|---|---|---|---|---|---|
| ¹H-NMR (400 MHz, DMSO-d₆): Verschiebung δ in ppm | | | | | | | |

### Herstellung der Zwischenprodukte:

### 4-Chlor-5-(2-chlorphenyl)-6-isopropylamino-2-methylsulfonyl-pyrimidin (II-1)

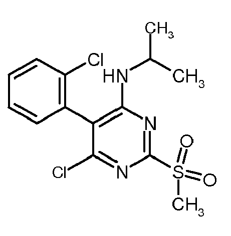

a) 12,4 g 4,6-Dihydroxy-5-(2-chlorphenyl)-2-(methylthio)pyrimidin wurden in 50 mL Phosphorylchlorid vorgelegt. Bei 5 °C wurden 11 mL N,N-Diethylanilin zugetropft. Es wurde für 16 h bei 85 °C gerührt. Die Reaktionsmischung wurde am Rotationsverdampfer auf ca. 40 mL eingeengt, auf Eiswasser gegeben und mit Methyl-tert.-butylether extrahiert. Die organische Phase wurde mit wäßriger Natriumhydrogencarbonat-Lösung gewaschen. Das Lösungsmittel wurde im Vakuum entfernt. Man erhielt 13,5 g 4,6-Dichlor-5-(2-chlorphenyl)-2-(methylthio)pyrimidin.
   MS (ES+): 305, logP: 4,52
b) 6,29 g 4,6-Dichlor-5-(2-chlorphenyl)-2-(methylthio)pyrimidin wurden in 60 mL Acetonitril gelöst und 3,41 g Kaliumcarbonat und 1,34 g Isopropylamin zugegeben. Es wurde für 16 h bei 50 °C gerührt. Die Reaktionsmischung wurde in 1N Salzsäure eingerührt und mit Essigsäure-ethylester extrahiert. Von der organischen Phase wurde das Lösungsmittel im Vakuum entfernt. Man erhielt 4,3 g 4-Chlor-5-(2-chlorphenyl)-6-(isopropylamino)-2-(methylthio)-pyrimidin.
   MS (ES+): 328, logP: 4,52
c) 1,5 g 4-Chlor-5-(2-chlorphenyl)-6-(isopropylamino)-2-(methylthio)-pyrimidin wurden in 30 mL Dichlormethan vorgelegt. Bei 0 °C wurden 0,63 g Ameisensäure, 179 mg Ammoniummolybdat(VI) und 466 mg Wasserstoffperoxid (35%-ig) zugegeben. Es wurde für drei Tage bei Raumtemperatur und für 16 h unter Rückfluß gerührt. Die Reaktionsmischung wurde in gesättigte Natriumhydrogencarbonat-Lösung eingerührt und mit Dichlormethan extrahiert. Von der organischen Phase wurde das Lösungsmittel im Vakuum entfernt. Der Rückstand wurde chromatografisch gereinigt (Kieselgel, Hexan/Essigsäure-ethylester 3:1). Man erhielt 0,8 g 4-Chlor-5-(2-chlorphenyl)-6-(isopropylamino)-2-(methylsulfonyl)-pyrimidin (II-1).
   MS (ES+): 360, logP: 2,96

### 4-Chlor-5-(2-chlorphenyl)-6-isopropylamino-2-methylsulfinyl-pyrimidin

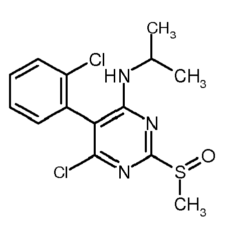

10,95 g 4-Chlor-5-(2-chlorphenyl)-6-(isopropylamino)-2-(methylthio)-pyrimidin wurden in 100 mL Dichlormethan vorgelegt. Bei 0 °C wurden 9,21 g Ameisensäure, 1,31 g Ammoniummolybdat(VI) und 6,81 g Wasserstoffperoxid (35%-ig) zugegeben. Es wurde für 16 h unter Rückfluß gerührt. Die Reaktionsmischung wurde in gesättigte Natriumhydrogencarbonat-Lösung eingerührt und mit Dichlormethan extrahiert. Von der organischen Phase wurde das Lösungsmittel im Vakuum entfernt. Man erhielt 9,53 g 4-Chlor-5-(2-chlorphenyl)-6-(isopropylamino)-2-(methylsulfinyl)-pyrimidin.
MS (ES+): 344, logP: 2,55
¹H-NMR (DMSO-d₆): δ = 7.62 (dd, 1H), 7.51 (m, 2 H), 7.37 (m, 1H), 6.54 (bd, 1 H), 4.32 - 4.38 (m, 1H), 2.87 (s, 3 H), 1.13 (m, 6H)

Die Bestimmung der in den Herstellungsbeispielen angegebenen logP-Werte erfolgte gemäß EEC-Directive 79/831 Annex V.A8 durch HPLC (High Performance Liquid Chromatography) an einer Phasenumkehrsäule (C 18). Temperatur: 43°C.

Die Bestimmung erfolgte mit der LC-MS bei pH 2,7 mit 0,1 % wässriger Ameisensäure und Acetonitril (enthält 0,1 % Ameisensäure) als Eluenten; linearer Gradient von 10 % Acetonitril bis 95 % Acetonitril.

Die Eichung erfolgte mit unverzweigten Alkan-2-onen (mit 3 bis 16 Kohlenstoffatomen), deren logP-Werte bekannt sind (Bestimmung der logP-Werte anhand der Retentionszeiten durch lineare Interpolation zwischen zwei aufeinanderfolgenden Alkanonen).

Die lambda-max-Werte wurden an Hand der UV-Spektren von 200 nm bis 400 nm in den Maxima der chromatographischen Signale ermittelt.

Aus den erhaltenen Massenspektren ist m/z des [M+H]⁺ Peaks angegeben.

### Anwendungsbeispiele

### Beispiel 1

### Myzus-Test (MYZUPE Spritzbehandlung)

| | | |
|---|---|---|
| Lösungsmittel: | 78,0 | Gewichtsteile Aceton |
| | 1,5 | Gewichtsteile Dimethylformamid |
| | | |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Chinakohlblattscheiben (*Brassica pekinensis*), die von allen Stadien der Grünen Pfirsichblattlaus (*Myzus persicae*) befallen sind, werden mit einer Wirkstoffzubereitung der gewünschten Konzentration gespritzt.

Nach der gewünschten Zeit wird die Wirkung in % bestimmt. Dabei bedeutet 100 %, dass alle Blattläuse abgetötet wurden; 0 % bedeutet, dass keine Blattläuse abgetötet wurden.

Bei diesem Test zeigen z. B. die folgenden Verbindungen der Herstellungsbeispiele eine Wirkung von ≥ 80 % bei einer Aufwandmenge von 500 g/ha:
Bsp. Nr. I-2, I-3, I-5, I-6, X-1, X-2, X-4, X-5

### Beispiel 2

### Meloidogyne-Test (MELGIN Spritzbehandlung)

| | | |
|---|---|---|
| Lösungsmittel: | 80,0 | Gewichtsteile Aceton |

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser auf die gewünschte Konzentration.

Gefäße werden mit Sand, Wirkstofflösung, *Meloidogyne incognita*-Ei-Larven-Suspension und Salatsamen gefüllt. Die Salatsamen keimen und die Pflänzchen entwickeln sich. An den Wurzeln entwickeln sich die Gallen.

Nach der gewünschten Zeit wird die nematizide Wirkung an Hand der Gallenbildung in % bestimmt. Dabei bedeutet 100 %, dass keine Gallen gefunden wurden; 0 % bedeutet, dass die Zahl der Gallen an den behandelten Pflanzen der der unbehandelten Kontrolle entspricht.

Bei diesem Test zeigen z. B. die folgenden Verbindungen der Herstellungsbeispiele eine Wirkung von ≥ 80 % bei einer Aufwandmenge von 4 ppm:
Bsp. Nr. I-3

### Beispiel 3

### Phaedon-Test (PHAECO Spritzbehandlung)

| | | |
|---|---|---|
| Lösungsmittel: | 78,0 | Gewichtsteile Aceton |
| | 1,5 | Gewichtsteile Dimethylformamid |
| | | |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Chinakohlblattscheiben *(Brassica pekinensis*) werden mit einer Wirkstoffzubereitung der gewünschten Konzentration gespritzt und nach dem Abtrocknen mit Larven des Meerrettichblattkäfers *(Phaedon cochleariae)* besetzt.

Nach der gewünschten Zeit wird die Wirkung in % bestimmt. Dabei bedeutet 100 %, dass alle Käferlarven abgetötet wurden; 0 % bedeutet, dass keine Käferlarven abgetötet wurden.

Bei diesem Test zeigen z. B. die folgenden Verbindungen der Herstellungsbeispiele eine Wirkung von ≥ 80 % bei einer Aufwandmenge von 500 g/ha:
Bsp. Nr. I-2, I-3, I-4, I-6, X-1, X-2, X-3, X-5

### Beispiel 4

### Sphaerotheca-Test (Gurke) / protektiv

| | | |
|---|---|---|
| Lösungsmittel: | 49 Gewichtsteile | N, N - Dimethylformamid |
| | | |
| Emulgator: | 1 Gewichtsteil | Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Gurkenpflanzen mit der Wirkstoffzubereitung in der angegebenen Aufwandmenge. 1 Tag nach der Behandlung werden die Pflanzen mit einer Sporensuspension von *Sphaerotheca fuliginea* inokuliert. Anschließend werden die Pflanzen in einem Gewächshaus bei 70 % relativer Luftfeuchtigkeit und einer Temperatur von 23□C aufgestellt.

7 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, daß kein Befall beobachtet wird.

In diesem Test zeigen die erfindungsgemäßen Verbindungen der nachfolgenden Formeln bei einer Konzentration an Wirkstoff von 500ppm einen Wirkungsgrad von 70% oder mehr:
Bsp. Nr. I-2, I-3

### Beispiel 5

### Puccinia-Test (Weizen) / protektiv

| | | |
|---|---|---|
| Lösungsmittel: | 49 Gewichtsteile | N, N - Dimethylformamid |
| | | |
| Emulgator: | 1 Gewichtsteil | Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Weizenpflanzen mit der Wirkstoffzubereitung in der angegebenen Aufwandmenge. 1 Tag nach der Behandlung werden die Pflanzen mit einer Sporensuspension von *Puccinia recondita* inokuliert und stehen dann 48h bei 100% rel. Feuchte und 22°C. Anschließend stehen die Pflanzen bei 80% rel. Luftfeuchtigkeit und einer Temperatur von 20□C.

7-9 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

In diesem Test zeigen die erfindungsgemäßen Verbindungen der nachfolgenden Formeln bei einer Konzentration an Wirkstoff von 500ppm einen Wirkungsgrad von 70% oder mehr:
Bsp. Nr. I-2, I-3

## Patentansprüche

1. Heterocyclyl-Pyrimidine der Formel (I), 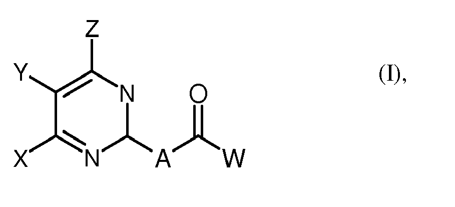 in welcher die Symbole folgende Bedeutung haben:
X für Wasserstoff, Halogen, Cyano, gegebenenfalls substituiertes Alkyl, gegebenenfalls substituiertes Phenyl steht;
Z steht für jeweils gegebenenfalls substituiertes Alkoxy, Alkylthio, Alkylsulfonyl oder die Gruppe 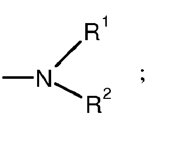
Y steht für gegebenenfalls substituiertes Aryl, gegebenenfalls substituiertes Heterocyclyl, gegebenenfalls substituiertes Alkyl, gegebenenfalls substituiertes Alkenyl, gegebenenfalls substituiertes Alkinyl, gegebenenfalls substituiertes Cycloalkyl, gegebenenfalls substituiertes Cycloalkenyl, gegebenenfalls substituiertes Arylalkyl, Halogen, eine gegebenenfalls substituierte Aminogruppe, gegebenenfalls substituiertes (C₁-C₈)-Alkoxy, gegebenenfalls substituiertes (C₁-C₈)-Alkylthio, gegebenenfalls substituiertes (C₆-C₁₀)-Aryloxy, gegebenenfalls substituiertes (C₆-C₁₀)-Arylthio, gegebenenfalls substituiertes Heterocyclyloxy, gegebenenfalls substituiertes (C₆-C₁₀)-Aryl-(C₁-C₄)-alkoxy, gegebenenfalls substituiertes (C₆-C₁₀)-Aryl-(C₁-C₄)-alkylthio, gegebenenfalls substituiertes Heterocyclyl-(C₁-C₄)-alkoxy, gegebenenfalls substituiertes Heterocyclyl-(C₁-C₄)-alkylthio;
A für einen drei- bis zehngliedrigen gesättigten, partiell ungesättigten oder aromatischen mono- oder bicyclischen Heterocyclus steht, enthaltend ein bis vier Heteroatome aus der Gruppe O, N oder S, wobei A durch eine bis drei gleiche oder verschiedene Gruppen R^{d} substituiert sein kann, und
R^{d} für Halogen, Hydroxy, Cyano, Oxo, Nitro, Amino, Mercapto, C₁-C₆-Alkyl, C₁-C₆-Halogenalkyl, C₂-C₆-Alkenyl, C₂-C₆-Alkinyl, C₃-C₆-Cycloalkyl, C₁-C₆-Alkoxy, C₁- C₆-Halogenalkoxy, C₁-C₇-Alkylcarbonylamino, C₁-C₆-Alkylamino, Di-(C₁-C₆-alkyl)amino, C₁-C₆-Alkylthio, C₁-C₆-Alkylsulfinyl, C₁-C₆-Alkylsulfonyl, Aminosulfonyl, C₁-C₆-Alkylaminosulfonyl, und/oder Di-(C₁-C₆-alkyl)aminosulfonyl steht;
W steht für 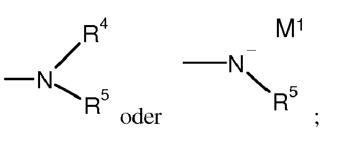
R¹ steht für Wasserstoff, Alkyl, Alkenyl, Alkinyl, Cycloalkyl, Halogenalkyl oder Heterocyclyl, Hydroxy, Alkoxy, Amin, Alkylamin, Dialkylamin, Hydroxyalkyl, Alkylcarbonyloxyalkyl oder Alkyloxycarbonyloxyalkyl;
R² steht für Wasserstoff oder Alkyl;
oder
R¹ und R² stehen gemeinsam mit dem Stickstoffatom, an das sie gebunden sind, für einen gegebenenfalls substituierten heterocyclischen Ring;
R⁴ steht für Wasserstoff, jeweils gegebenenfalls substituiertes Alkyl, Alkenyl, Alkinyl und Cycloalkyl, jeweils gegebenenfalls substituiertes gesättigtes oder ungesättigtes Heterocyclyl, jeweils gegebenenfalls substituiertes Phenyl, Heteroaryl, Arylalkyl, Heteroarylalkyl;
M¹ steht für ein Kation;
R⁵ steht für die Gruppen COR⁷, S(O)₁₋₂R⁷, Cyano, COOR⁷, 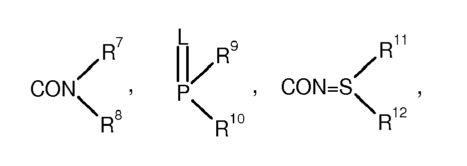 gesättigtes, teilweise oder vollständig ungesättigtes oder aromatisches, gegebenenfalls substituiertes 5- oder 6-Ring Heterocyclyl, das gegebenenfalls ein oder bis zu drei weitere Heteroatome, ausgewählt aus der Gruppe bestehend aus N, S und/oder O-Atomen enthält, wobei Sauerstoffatome nicht benachbart sein dürfen,
L steht für Sauerstoff oder Schwefel,
R⁷ steht für Wasserstoff, Alkyl, Alkenyl, Alkinyl, Halogenalkyl, Cycloalkyl-alkyl, Cycloalkyl oder Cycloalkenyl, Aryl, Heteroaryl, Arylalkyl oder Heteroarylalkyl,
R⁸ steht für Wasserstoff, jeweils gegebenenfalls substituiertes Alkyl, Alkenyl, Alkinyl, Alkoxy oder NH-R⁴,
R⁷ und R⁸ stehen gemeinsam mit dem Stickstoffatom, an das sie gebunden sind, für einen gegebenenfalls substituierten gesättigten oder ungesättigten Ring, der gegebenenfalls durch weitere Heteroatome unterbrochen sein kann;
R⁹ und R¹⁰ stehen unabhängig voneinander für jeweils gegebenenfalls substituiertes Alkyl, Alkenyl, Alkoxy, Alkenyloxy, Alkinyloxy, Cycloalkoxy, Cycloalkenyloxy, Cycloalkylalkoxy, Alkylthio, Alkenylthio, Phenoxy, Phenylthio, Benzyloxy, Benzylthio, Heteroaryloxy, Heteroarylthio, Heteroarylalkoxy oder Heteroarylalkylthio;
oder
R⁹ und R¹⁰ stehen gemeinsam mit dem Phosphoratom, an das sie gebunden sind, für einen gegebenenfalls substituierten 5- bis 7-gliedrigen Cyclus, der durch ein oder zwei Sauerstoff- und/oder Schwefelatome unterbrochen sein kann; und
R¹¹ und R¹² stehen unabhängig voneinander für jeweils gegebenenfalls substituiertes Alkyl, Alkenyl, Alkinyl, Phenyl oder Phenylalkyl;
sowie agrochemisch wirksame Salze davon.

2. Heterocyclyl-Pyrimidine der Formel (I) gemäß Anspruch 1, in welcher die Symbole folgende Bedeutung haben:
X steht bevorzugt für Wasserstoff, Fluor, Chlor, Brom, Cyano, C1-C6-Alkyl oder C1-C4-Halogenalkyl mit 1 bis 9 Halogenatomen;
Z steht für jeweils gegebenenfalls durch 1 bis 7 Halogenatome substituiertes geradkettiges oder verzweigtes C₁-C₆-Alkoxy, C₁-C₆-Alkylthio, C₁-C₆-Alkylsulfonyl oder die Gruppe 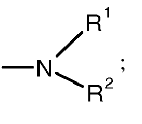
Y steht für Phenyl, das gegebenenfalls einfach bis vierfach, gleichartig oder verschieden substituiert sein kann durch
Halogen, Cyano, Nitro, Amino, Hydroxy, Formyl, Carboxy, Carboxyalkyl, Carbamoyl, Thiocarbamoyl;
jeweils geradkettiges oder verzweigtes Alkyl, Alkoxy, Alkylthio, Alkylsulfinyl oder Alkylsulfonyl mit jeweils 1 bis 6 Kohlenstoffatomen;
jeweils geradkettiges oder verzweigtes Alkenyl, Alkinyl, Alkinyloxy oder Alkenyloxy mit jeweils 2 bis 6 Kohlenstoffatomen;
jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen;
jeweils geradkettiges oder verzweigtes Halogenalkenyl oder Halogenalkenyloxy mit jeweils 2 bis 6 Kohlenstoffatomen und 1 bis 11 gleichen oder verschiedenen Halogenatomen;
jeweils geradkettiges oder verzweigtes Alkylamino, Dialkylamino, Alkylcarbonyl, Alkylcarbonyloxy, Alkoxycarbonyl, Alkylsulfonyloxy, Hydroximinoalkyl oder Alkoximinoalkyl mit jeweils 1 bis 6 Kohlenstoffatomen in den einzelnen Alkylteilen;
gegebenenfalls einfach bis zweifach, durch Fluor, Chlor, C₁-C₃-Alkyl oder C₁-C₃-Alkoxy substituiertes Cycloalkyl mit 3 bis 8 Kohlenstoffatomen;
in 2,3-Position oder 3,4-Position verknüpftes 1,3-Propandiyl, 1,4-Butandiyl, Methylendioxy (-O-CH₂-O-) oder 1,2-Ethylendioxy (-O-CH₂-CH₂-O-), wobei diese Reste einfach oder mehrfach, gleichartig oder verschieden substituiert sein können durch Halogen, Alkyl mit 1 bis 4 Kohlenstoffatomen und/oder Halogenalkyl mit 1 bis 4 Kohlenstoffatomen und 1 bis 9 gleichen oder verschiedenen Halogenatomen; oder
Y steht für gesättigtes oder ganz oder teilweise ungesättigtes oder aromatisches Heterocyclyl mit 3 bis 8 Ringgliedern und 1 bis 3 Heteroatomen aus der Gruppe Stickstoff, Sauerstoff und Schwefel, wobei das Heterocyclyl einfach oder zweifach substituiert sein kann durch Halogen, Alkyl mit 1 bis 4 Kohlenstoffatomen, Alkoxy mit 1 bis 4 Kohlenstoffatomen, Alkylthio mit 1 bis 4 Kohlenstoffatomen, Haloalkoxy mit 1 bis 4 Kohlenstoffatomen, Haloalkylthio mit 1 bis 4 Kohlenstoffatomen, Hydroxy, Mercapto, Cyano, Nitro und/oder Cycloalkyl mit 3 bis 6 Kohlenstoffatomen oder/und Carboxyalkyl;
W steht für 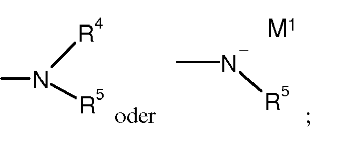
R¹ steht für Wasserstoff, Alkyl mit 1 bis 10 Kohlenstoffatomen, das unsubstituiert oder einfach bis fünffach, gleichartig oder verschieden substituiert ist durch Cyano, Hydroxy, Alkoxy mit 1 bis 4 Kohlenstoffatomen, Alkylcarbonyloxy mit 1 bis 6 Kohlenstoffatomen, Alkoxycarbonyloxy mit 1 bis 6 Kohlenstoffatomen, Cycloalkyl mit 3 bis 6 Kohlenstoffatomen, Mercapto, Alkylthio mit 1 bis 4 Kohlenstoffatomen, Amino, Mono- oder Dialkylamino mit jeweils 1 bis 4 Kohlenstoffatomen; oder
R¹ steht für Alkenyl mit 3 bis 10 Kohlenstoffatomen, das unsubstituiert oder einfach bis dreifach, gleichartig oder verschieden substituiert ist durch Halogen, Cyano, Hydroxy, Alkoxy mit 1 bis 4 Kohlenstoffatomen, Cycloalkyl mit 3 bis 6 Kohlenstoffatomen, Mercapto, Alkylthio mit 1 bis 4 Kohlenstoffatomen, Amino, Mono- oder Dialkylamino mit jeweils 1 bis 4 Kohlenstoffatomen; oder
R¹ steht für Alkinyl mit 3 bis 10 Kohlenstoffatomen, das unsubstituiert oder einfach bis dreifach, gleichartig oder verschieden substituiert ist durch Halogen, Cyano, Hydroxy, Alkoxy mit 1 bis 4 Kohlenstoffatomen, Cycloalkyl mit 3 bis 6 Kohlenstoffatomen, Mercapto, Alkylthio mit 1 bis 4 Kohlenstoffatomen, Amino, Mono- oder Dialkylamino mit jeweils 1 bis 4 Kohlenstoffatomen; oder
R¹ steht für Cycloalkyl mit 3 bis 10 Kohlenstoffatomen, das unsubstituiert oder einfach bis dreifach, gleichartig oder verschieden substituiert ist durch Halogen und/oder Alkyl, Halogenalkyl mit jeweils 1 bis 4 Kohlenstoffatomen; oder
R¹ steht für Halogenalkyl mit 1 bis 10 Kohlenstoff- und 1 bis 21 Halogenatomen; oder
R¹ steht für gesättigtes oder ungesättigtes Heterocyclyl mit 3 bis 10 Ringgliedern und 1 bis 3 Heteroatomen, wie Stickstoff, Sauerstoff und/oder Schwefel, wobei das Heterocyclyl unsubstituiert oder einfach oder mehrfach substituiert ist durch Halogen, Alkyl mit 1 bis 4 Kohlenstoffatomen, Alkoxy mit 1 bis 4 Kohlenstoffatomen, Cyano, Nitro, Cycloalkyl mit 3 bis 6 Kohlenstoffatomen, Hydroxy, Alkoxy, Alkenyloxy, Alkinyloxy mit 1 bis 6 Kohlenstoffatomen oder Mercapto;
R² steht für Wasserstoff oder Alkyl mit 1 bis 6 Kohlenstoffatomen; oder
R¹ und R² gemeinsam mit dem Stickstoffatom, an das sie gebunden sind, für einen gesättigten oder ungesättigten heterocyclischen Ring mit 3 bis 8 Ringgliedern, wobei der Heterocyclus gegebenenfalls ein weiteres Stickstoff-, Sauerstoff- oder Schwefelatom als Ringglied enthält und wobei der Heterocyclus unsubstituiert oder bis zu dreifach substituiert sein kann durch Fluor, Chlor, Brom, Alkyl mit 1 bis 4 Kohlenstoffatomen, Halogenalkyl mit 1 bis 4 Kohlenstoffatomen und 1 bis 9 Fluor- und/oder Chloratomen, Hydroxy, Alkoxy mit 1 bis 4 Kohlenstoffatomen, Hydroxyalkyl mit 1 bis 4 Kohlenstoffatomen, Alkylcarbonyloxyalkyl mit 1 bis 8 Kohlenstoffatomen, Alkyloxycarbonyloxyalkyl mit 1 bis 8 Kohlenstoffatomen, Haloalkoxy mit 1 bis 4 Kohlenstoffatomen und 1 bis 9 Fluor und/oder Chloratomen, Mercapto, Thioalkyl mit 1 bis 4 Kohlenstoffatomen und/oder Haloalkylthio mit 1 bis 4 Kohlenstoffatomen und 1 bis 9 Fluor und/oder Chloratomen;
R⁴ steht für Wasserstoff,
für jeweils gegebenenfalls einfach bis mehrfach durch Fluor, Chlor, C₁-C₄-Alkoxy, C₁-C₄-Alkylthio, Cyano, CO₂-H oder CO-O-C₁-C₄-Alkyl substituiertes C₁-C₁₀-Alkyl, C₃-C₁₀-Alkenyl oder C₃-C₁₀-Alkinyl, für jeweils gegebenenfalls einfach bis mehrfach durch Fluor, Chlor, C₁-C₄-Alkyl, C₁-C₄-Halogenalkyl, C₁-C₄-Alkoxy, CN, CO₂H oder CO-O-C₁-C₄-Alkyl substituiertes C₃-C₈-Cycloalkyl oder C₅-C₈-Cycloalkenyl, welches gegebenenfalls durch ein Sauerstoff- oder Schwefelatom unterbrochen sein kann,
für jeweils gegebenenfalls einfach bis dreifach durch Fluor, Chlor, Brom C₁-C₄-Alkyl, C₁-C₄-Halogenalkyl, C₁-C₄-Alkoxy, C₁-C₄-Halogenalkoxy, Nitro oder Cyano substituiertes Phenyl, Pyridyl, Thiazolyl, Pyrimidyl, Benzyl, Phenethyl, Pyridinylmethyl oder Thiazolylmethyl;
M¹ steht für Kationen aus der Reihe der Alkali- oder Erdalkalimetalle oder Ammoniumionen NH₄, mono-(C₁-C₁₀)-Alkylammonium, di-(C₁-C₁₀)-Alkylammonium, tri-(C₁-C₁₀)-Alkylammonium, tetra-(C₁-C₁₀)-Alkylammonium, wobei die Alkylreste der Ammoniumionen mit Aryl oder Hydroxy substituiert sein können, oder Cholinium,
R⁵ steht für die Gruppen COR⁷, S(O)₁₋₂R⁷, Cyano, COOR⁷, 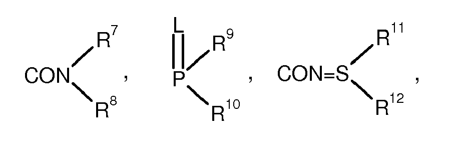 oder für jeweils gegebenenfalls einfach bis zweifach durch Fluor, Chlor, Brom, C₁-C₄-Alkyl, C₁-C₄-Alkoxy, Cyano oder Nitro substituiertes Pyridin, Pyrimidin, Triazin, Thiazol oder Pyrazol,
L steht für Sauerstoff oder Schwefel,
R⁷ steht für Wasserstoff, für jeweils gegebenenfalls einfach bis mehrfach durch C₁-C₄-Alkoxy, C₁-C₄-Alkylthio, Cyano, CO₂-H oder CO-O-C₁-C₄-Alkyl substituiertes C₁-C₁₀-Alkyl, C₃-C₁₀-Alkenyl oder C₃-C₁₀-Alkinyl, für C1-C10-Halogenalkyl mit 1 bis 21 Fluor- und/oder Chloratomen für jeweils gegebenenfalls einfach bis mehrfach durch Fluor, Chlor, C₁-C₄-Alkyl, C₁-C₄-Halogenalkyl, C₁-C₄-Alkoxy, CN, CO₂H oder CO-O-C₁-C₄-Alkyl substituiertes C₃-C₈-Cycloalkyl, C₃-C₆-Cycloalkyl-C₁-C₂-alkyl oder C₅-C₈-Cycloalkenyl, welches gegebenenfalls durch ein Sauerstoff- oder Schwefelatom unterbrochen sein kann,
für jeweils gegebenenfalls einfach bis dreifach durch Fluor, Chlor, Brom, C₁-C₄-Alkyl, C₁-C₄-Halogenalkyl, C₁-C₄-Alkoxy, C₁-C₄-Halogenalkoxy, Nitro oder Cyano substituiertes Phenyl, Pyridyl, Thiazolyl, Pyrimidyl, Benzyl, Phenethyl, Pyridinylmethyl oder Thiazolylmethyl,
R⁸ steht für Wasserstoff, jeweils gegebenenfalls einfach bis mehrfach durch Fluor und/oder Chlor substituiertes C₁-C₆-Alkyl, C₃-C₆-Alkenyl, C₃-C₆-Alkinyl oder C₁-C₆-Alkoxy;
oder
R⁷ und R⁸ stehen gemeinsam mit dem Stickstoffatom, an das sie gebunden sind, für einen gegebenenfalls einfach bis mehrfach durch C₁-C₄-Alkyl substituierten gesättigten oder ungesättigten 5- oder 6-Ring, der gegebenenfalls durch Sauerstoff oder Schwefel unterbrochen sein kann;
R⁹ und R¹⁰ stehen unabhängig voneinander für jeweils gegebenenfalls einfach bis mehrfach durch Fluor und/oder Chlor substituiertes C₁-C₈-Alkyl, C₃-C₈-Alkenyl, C₁-C₈-Alkoxy, C₃-C₈-Alkenyloxy, C₃-C₈-Alkinyloxy, C₁-C₆-Alkylthio, C₃-C₆-Alkenylthio, jeweils gegebenenfalls einfach bis zweifach durch Fluor, Chlor, C₁- C₄-Alkyl, C₁-C₄-Halogenalkyl, C₁-C₄-Alkoxy substituiertes C₃-C₈-Cycloalkoxy, C₄-C₈-Cycloalkenyloxy oder C₃-C₈-Cycloalkyl-C₁-C₂-alkoxy, für jeweils gegebenenfalls einfach bis dreifach durch Fluor, Chlor, Brom, C₁-C₆-Alkyl-C₁-C₄-Halogenalkyl, C₁-C₆-Alkoxy, C₁-C₄-Halogenalkoxy, C₁-C₆-Alkylthio, Cyano oder Nitro substituiertes Phenoxy, Phenylthio, Benzyloxy, Benzylthio, Pyridinyloxy, Pyrazolyloxy, Pyridinylthio, Pyrimidylthio, Thiazolylthio, Pyridyl-C₁-C₂-alkoxy, Pyrimidyl-C₁-C₂-alkyloxy, Thiazolyl-C₁-C₂-alkyloxy, Pyridyl-C₁-C₂-alkylthio, Thiazolyl-C₁-C₂-alkylthio oder Pyrimidyl-C₁-C₂-alkylthio;
oder
R⁹ und R¹⁰ stehen gemeinsam mit dem Phosphoratom an das sie gebunden sind, für einen gegebenenfalls einfach bis dreifach durch Fluor, Chlor, C₁-C₄-Alkyl, C₁-C₄-Alkoxy oder C₁-C₄-Halogenalkyl substituierten fünf- bis siebengliedrigen Cyclus der durch ein oder zwei Sauerstoff- und/oder Schwefelatome unterbrochen sein kann;
R¹¹ und R¹² stehen unabhängig voneinander für jeweils gegebenenfalls einfach bis dreifach durch Fluor oder Chlor substituiertes C₁-C₈-Alkyl, C₃-C₈-Alkenyl oder C₃-C₈-Alkinyl, für jeweils gegebenenfalls einfach bis zweifach durch Fluor, Chlor, Brom, C₁-C₄-Alkyl, C₁-C₄-Halogenalkyl, C₁-C₄-Alkoxy, C₁-C₄-Halogenalkoxy, Cyano oder Nitro substituiertes Phenyl oder Benzyl;

3. Heterocyclyl-Pyrimidine der Formel (I), gemäß Anspruch 1 oder 2, in welcher die Symbole folgende Bedeutung haben:
X steht für Wasserstoff, Fluor, Chlor, Brom, Cyano, C₁-C₄-Alkyl oder C₁-C₂-Halogenalkyl, welches mit ein bis fünf Fluor- und/oder Chloratome substituiert sein kann;
Z steht für jeweils gegebenenfalls durch 1 bis 3 Halogenatome substituiertes geradkettiges oder verzweigtes C₁-C₄-Alkoxy, C₁-C₄-Alkylthio oder die Gruppe 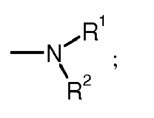
Y steht für Phenyl, das gegebenenfalls einfach bis dreifach, gleichartig oder verschieden substituiert sein kann durch Fluor, Chlor, Brom, Jod, Cyano, Nitro, Formyl, Methyl, Ethyl, n- oder i-Propyl, n-, i-, s- oder t-Butyl, Vinyl, 1-Propenyl, Ethinyl, 1-Propinyl, Allyl, Propargyl, Methoxy, Ethoxy, n- oder i-Propoxy, Methylthio, Ethylthio, n- oder i-Propylthio, Methylsulfinyl, Ethylsulfinyl, Methylsulfonyl, Ethylsulfonyl, Allyloxy, Propargyloxy, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl, Trifluormethylsulfonyl, Trichlorethinyloxy, Trifluorethinyloxy, Chlorallyloxy, Iodpropargyloxy, Methylamino, Ethylamino, n- oder i-Propylamino, Dimethylamino, Diethylamino, Acetyl, Propionyl, Acetyloxy, Methoxycarbonyl, Ethoxycarbonyl, Hydroximinomethyl, Hydroximinoethyl, Methoximinomethyl, Ethoximinomethyl, Methoximinoethyl, Ethoximinoethyl, Cyclopropyl, Cyclobutyl, Cyclopentyl oder Cyclohexyl, oder durch
in 2,3-Position oder 3,4-Position verknüpftes 1,3-Propandiyl, 1,4-Butandiyl, Methylendioxy (-O-CH₂-O-) oder 1,2-Ethylendioxy (-O-CH₂-CH₂-O-), wobei diese Reste einfach oder mehrfach, gleichartig oder verschieden substituiert sein können durch Fluor, Chlor, Methyl, Ethyl, n-Propyl, i-Propyl Trifluormethyl, Carboxy und/oder Carboxymethyl;
W steht für 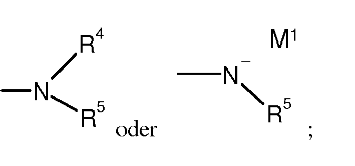
R¹ steht für für Wasserstoff, Alkyl mit 1 bis 8 Kohlenstoffatomen, das unsubstituiert oder einfach bis dreifach, gleichartig oder verschieden substituiert ist, durch Cyano, Alkoxy mit 1 bis 3 Kohlenstoffatomen, Hydroxy, Alkylcarbonyloxy mit 1 bis 5 Kohlenstoffatomen, Alkoxycarbonyloxy mit 1 bis 5 Kohlenstoffatomen, Cycloalkyl mit 3 bis 6 Kohlenstoffatomen, Alkylthio mit 1 bis 3 Kohlenstoffatomen; oder
R¹ steht für Alkenyl mit 3 bis 8 Kohlenstoffatomen, das unsubstituiert oder einfach bis dreifach, gleichartig oder verschieden substituiert ist durch Fluor, Chlor, Cyano, Alkoxy mit 1 bis 3 Kohlenstoffatomen, Cycloalkyl mit 3 bis 6 Kohlenstoffatomen, Alkylthio mit 1 bis 3 Kohlenstoffatomen; oder
R¹ steht für Alkinyl mit 3 bis 8 Kohlenstoffatomen, das unsubstituiert oder einfach bis dreifach, gleichartig oder verschieden substituiert ist durch Fluor, Chlor, Cyano, Alkoxy mit 1 bis 3 Kohlenstoffatomen, Cycloalkyl mit 3 bis 6 Kohlenstoffatomen, Alkylthio mit 1 bis 3 Kohlenstoffatomen; oder
R¹ steht für Cycloalkyl mit 3 bis 8 Kohlenstoffatomen, das unsubstituiert oder einfach bis dreifach, gleichartig oder verschieden substituiert ist durch Fluor, Chlor und/oder Alkyl, Halogenalkyl, Alkoxy mit jeweils 1 bis 2 Kohlenstoffatomen; oder
R1 steht für Halogenalkyl mit 1 bis 8 Kohlenstoff- und 1 bis 17 Fluor- und/oder Chloratomen; oder
R¹ steht für gesättigtes oder ungesättigtes Heterocyclyl mit 3 bis 8 Ringgliedern und 1 bis 2 Heteroatomen, wie Stickstoff, Sauerstoff und/oder Schwefel, steht, wobei das Heterocyclyl unsubstituiert oder einfach oder zweifach substituiert ist durch Hydroxy, Fluor, Chlor, Alkyl mit 1 bis 3 Kohlenstoffatomen, Alkoxy mit 1 bis 3 Kohlenstoffatomen, Cyano, Nitro oder Cycloalkyl mit 3 bis 6 Kohlenstoffatomen;
R² steht für Wasserstoff oder Alkyl mit 1 bis 6 Kohlenstoffatomen; oder
R¹ und R² stehen für gemeinsam mit dem Stickstoffatom, an das sie gebunden sind, für einen gesättigten oder ungesättigten heterocyclischen Ring mit 3 bis 6 Ringgliedern, wobei der Heterocyclus gegebenenfalls ein weiteres Stickstoff-, Sauerstoff- oder Schwefelatom als Ringglied enthält und wobei der Heterocyclus unsubstituiert oder bis zu dreifach substituiert sein kann durch Fluor, Chlor, Brom, Alkyl mit 1 bis 3 Kohlenstoffatomen, Halogenalkyl mit 1 bis 3 Kohlenstoffatomen und 1 bis 5 Fluor- und/oder Chloratomen, Alkoxy mit 1 bis 3 Kohlenstoffatomen, Hydroxyalkyl mit 1 bis 3 Kohlenstoffatomen, Alkylcarbonyloxyalkyl mit 1 bis 7 Kohlenstoffatomen, Alkyloxycarbonyloxyalkyl mit 1 bis 7 Kohlenstoffatomen, Halogenalkoxy mit 1 bis 3 Kohlenstoffatomen und 1 bis 5 Fluor und/oder Chloratomen, Thioalkyl mit 1 bis 3 Kohlenstoffatomen und/oder Halogenalkylthio mit 1 bis 3 Kohlenstoffatomen und 1 bis 5 Fluor und/oder Chloratomen;
R⁴ steht für Wasserstoff,
für jeweils gegebenenfalls einfach bis dreifach durch Fluor, Chlor, C₁-C₃-Alkoxy, C₁-C₃-Alkylthio, Cyano, CO₂H oder CO-O-C₁-C₃-Alkyl substituiertes C₁-C₈-Alkyl, C₃-C₈-Alkenyl oder C₃-C₈-Alkinyl, für jeweils gegebenenfalls einfach bis dreifach durch Fluor, Chlor, C₁-C₃-Alkyl, C₁-C₂-Halogenalkyl, C₁-C₃-Alkoxy, CN, CO₂H oder CO-O-C₁-C₃-Alkyl substituiertes C₃-C₆-Cycloalkyl, C₃-C₆-Cycloalkyl-methyl oder C₅-C₆-Cycloalkenyl, welches gegebenenfalls durch ein Sauerstoff- oder Schwefelatom unterbrochen sein kann,
für jeweils gegebenenfalls einfach bis zweifach durch Fluor, Chlor, Brom, C₁-C₃-Alkyl, C₁-C₃-Halogenalkyl, C₁-C₃-Alkoxy, C₁-C₃-Halogenalkoxy, Nitro oder Cyano substituiertes Phenyl, Pyridyl, Thiazolyl, Pyrimidyl, Benzyl, Phenethyl, Pyridinylmethyl oder Thiazolylmethyl;
M¹ steht für Na⁺, K⁺, ½ Ca²⁺, ½ Mg²⁺, NH₄⁺, NH₃CH₃⁺, NH₂(CH₃)₂⁺, NH(CH₃)₃⁺, NH(C₂H₅)₃⁺, NH₂(C₂H₅)₂⁺, NH₃C₂H₅⁺, NH₃i-C₃H₇⁺, NH₂(i-C₃H₇)₂⁺, NH₃-CH₂-C₆H₅⁺, N(CH₃)₃-H₂-C₆H₅⁺, 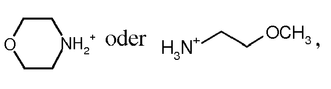
R⁵ steht für die Gruppen COR⁷, S(O)₁₋₂R⁷, Cyano, COOR⁷, 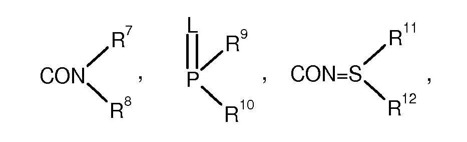 oder für jeweils gegebenenfalls einfach bis zweifach durch Fluor, Chlor, Brom, C₁-C₄-Alkyl, C₁-C₄-Alkoxy, Cyano oder Nitro substituiertes Pyridin, Pyrimidin, Triazin, Thiazol oder Pyrazol,
L steht für Sauerstoff oder Schwefel,
R⁷ steht für Wasserstoff, für jeweils gegebenenfalls einfach bis fünffach durch C₁-C₃-Alkoxy, C₁-C₃-Alkylthio, Cyano, CO₂H oder CO-O-C1-C₃-Alkyl substituiertes C₁-C₈-Alkyl, C₃-C₈-Alkenyl oder C₃-C₈-Alkinyl, für C₁-C₈-Halogenalkyl mit 1 bis 17 Fluor- und/oder Chloratomen, für jeweils gegebenenfalls einfach bis dreifach durch Fluor, Chlor, C₁-C₄-Alkyl, C₁-C₂-Halogenalkyl, C₁-C₂-Alkoxy, CN, CO2H oder CO-O-C1-C₂-Alkyl substituiertes C₃-C₆-Cycloalkyl, C₃-C₆-Cycloalkyl-methyl oder C₅-C₆-Cycloalkenyl, welches gegebenenfalls durch ein Sauerstoff- oder Schwefelatom unterbrochen sein kann,
für jeweils gegebenenfalls einfach bis zweifach durch Fluor, Chlor, Brom, C₁-C₃-Alkyl, C₁-C₃-Halogenalkyl, C₁-C₃-Alkoxy, C₁-C₃-Halogenalkoxy, Nitro oder Cyano substituiertes Phenyl, Pyridyl, Thiazolyl, Pyrimidyl, Benzyl, Phenethyl, Pyridinylmethyl oder Thiazolylmethyl;
R⁸ steht ganz für Wasserstoff, Methyl, Ethyl, Propyl, iso-Propyl, Allyl, Propargyl, C₃-C₄-Alkenyl, Methoxy oder Ethoxy; oder
R⁷ und R⁸ stehen gemeinsam mit dem Stickstoffatom, an das sie gebunden sind, für einen gesättigten 5- oder 6-Ring, der gegebenenfalls durch Sauerstoff oder Schwefel unterbrochen sein kann; und
R⁹ und R¹⁰ stehen unabhängig voneinander für jeweils gegebenenfalls einfach bis dreifach durch Fluor und/oder Chlor substituiertes Methyl, Ethyl, Methoxy, Ethoxy, Propoxy, i-Propoxy, Butoxy, Alkyloxy, Methylthio, Ethylthio, Propylthio, Butylthio, i-Propylthio, sek.-Butylthio, Allylthio.

4. Heterocyclyl-Pyrimidine der Formel (I), gemäß Anspruch 1 bis 3, in welcher die Symbole folgende Bedeutung haben:
X steht für Wasserstoff, Chlor, Brom, Methyl oder Trifluormethyl;
Z steht für Methoxy, Ethoxy, Methylthio, Ethythio oder die Gruppe 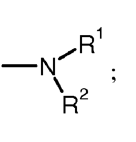
Y steht für einfach bis dreifach substituiertes Phenyl mit Substituenten aus der Gruppe Fluor, Chlor, Brom, Cyano, Nitro, Methyl, Ethyl, tert.-Butyl, Methoxy, Ethoxy, Trifluormethyl, Difluormethoxy oder Trifluormethoxy;
W steht für 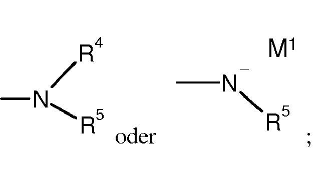
R¹ steht für Wasserstoff, Methyl, Ethyl, Propyl, Butyl, das unsubstituiert oder einfach bis vierfach, gleichartig oder verschieden substituiert ist, durch Methyl, Ethyl, Propyl, Butyl, Hydroxy, Acetyloxy, Isobutyryloxy, Methoxy, Ethoxy, Cyclopropyl, Cyclopentyl, Cyclohexyl, Methylthio oder Ethylthio; oder
R¹ steht für Alkenyl mit 3 bis 6 Kohlenstoffatomen, das unsubstituiert oder einfach bis dreifach, gleichartig oder verschieden substituiert ist durch Fluor, Chlor, Methoxy, Ethoxy, Methylthio oder Ethylthio; oder
R¹ steht für Alkinyl mit 3 bis 6 Kohlenstoffatomen, das unsubstituiert oder einfach bis dreifach, gleichartig oder verschieden substituiert ist durch Fluor, Chlor, Methoxy, Ethoxy, Methylthio oder Ethylthio; oder
R¹ steht für Cycloalkyl mit 3 bis 6 Kohlenstoffatomen, das unsubstituiert oder einfach bis dreifach, gleichartig oder verschieden substituiert ist durch Fluor, Chlor, Methyl, Ethyl, Methoxy oder Trifluormethyl;
R¹ steht für Methyl, Ethyl, Propyl oder Butyl, das mit 1 bis 9 Fluor- und /oder Chloratomen substituiert ist; oder
R² steht für Wasserstoff, Methyl oder Ethyl; oder
R¹ und R² stehen gemeinsam mit dem Stickstoffatom, an das sie gebunden sind, für einen gesättigten oder ungesättigten heterocyclischen Ring mit 3 bis 6 Ringgliedern, wobei der Heterocyclus gegebenenfalls ein weiteres Stickstoff-, Sauerstoff- oder Schwefelatom als Ringglied enthält und wobei der Heterocyclus unsubstituiert oder bis zu zweifach substituiert sein kann durch Fluor, Chlor, Methyl, Ethyl, Trifluormethyl, Methoxy, Ethoxy, Hydroxymethyl, Acetyloxymethyl oder Isobutyryloxymethyl;
R⁴ steht für Wasserstoff,;
M¹ steht für Na⁺, K⁺, ½ Ca²⁺, ½ Mg²⁺, NH₄⁺, NH₃CH₃⁺, NH₂(CH₃)₂⁺, NH(CH₃)₃⁺, NH(C₂H₅)₃⁺, NH₂(C₂H₅)₂⁺, NH₃C₂H₅⁺, NH₃i-C₃H₇⁺, NH₂(i-C₃H₇)₂⁺, NH₃-CH₂-C₆H₅⁺ oder N(CH₃)₃-H₂-C₆H₅⁺;
R⁵ steht für die Gruppen COR⁷, S(O)₁₋₂R⁷, Cyano, COOR⁷, 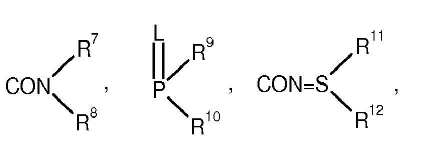
R⁷ steht für Wasserstoff, für jeweils gegebenenfalls einfach bis dreifach durch Methoxy, Ethoxy, Methylthio, Ethylthio, Cyano, CO-O-Methyl oder CO-O-Ethyl substituiertes Methyl, Ethyl, n-Propyl, iso-Propyl oder Cyclopropyl, für Halogenmethyl, -propyl, -n-propyl, -i-propyl mit 1 bis 7 Fluor- und/oder Chloratomen,
R⁸ steht für Wasserstoff, Methyl, Ethyl, Propyl, iso-Propyl, Allyl, Propargyl, C₃-C₄-Alkenyl, Methoxy oder Ethoxy; oder
R⁷ und R⁸ stehen gemeinsam mit dem Stickstoffatom, an das sie gebunden sind, für einen gesättigten 5- oder 6-Ring, der gegebenenfalls durch Sauerstoff oder Schwefel unterbrochen sein kann; und
R⁹ und R¹⁰ stehen unabhängig voneinander für jeweils gegebenenfalls einfach bis dreifach durch Fluor und/oder Chlor substituiertes Methyl, Ethyl, Methoxy, Ethoxy, Propoxy, i-Propoxy, Butoxy, Alkyloxy, Methylthio, Ethylthio, Propylthio, Butylthio, i-Propylthio, sek.-Butylthio, Allylthio;
R¹¹ und R¹² stehen besonders bevorzugt unabhängig voneinander für jeweils gegebenenfalls einfach bis dreifach durch Fluor oder Chlor substituiertes C₁-C₆-Alkyl, C₃-C₆-Alkenyl oder C₃-C₆-Alkinyl.

5. Heterocyclyl-Pyrimidine der Formel (I), gemäß Anspruch 1 bis 4, in welcher die Symbole folgende Bedeutung haben:
X steht für Wasserstoff, Chlor, Brom, Methyl oder Trifluormethyl;
Z steht für Methoxy, Ethoxy, Methylthio, Ethythio oder die Gruppe 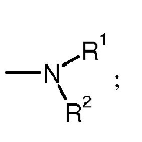
Y steht für einfach bis dreifach substituiertes Phenyl mit Substituenten aus der Gruppe Fluor, Chlor, Brom, Cyano, Nitro, Methyl, Ethyl, tert.-Butyl, Methoxy, Ethoxy, Trifluormethyl, Difluormethoxy oder Trifluormethoxy;
W steht für 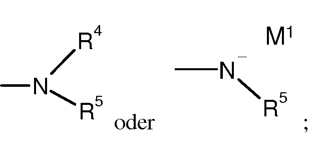
R¹ steht für für Wasserstoff, Methyl, Ethyl, Propyl, Butyl, das unsubstituiert oder einfach bis vierfach, gleichartig oder verschieden substituiert ist, durch Methyl, Ethyl, Propyl, Butyl, Hydroxy, Acetyloxy, Isobutyryloxy, Methoxy, Ethoxy, Cyclopropyl, Cyclopentyl, Cyclohexyl, Methylthio oder Ethylthio; oder
R¹ steht für Alkenyl mit 3 bis 6 Kohlenstoffatomen, das unsubstituiert oder einfach bis dreifach, gleichartig oder verschieden substituiert ist durch Fluor, Chlor, Methoxy, Ethoxy, Methylthio oder Ethylthio; oder
R¹ steht für Alkinyl mit 3 bis 6 Kohlenstoffatomen, das unsubstituiert oder einfach bis dreifach, gleichartig oder verschieden substituiert ist durch Fluor, Chlor, Methoxy, Ethoxy, Methylthio oder Ethylthio; oder
R¹ steht für Cycloalkyl mit 3 bis 6 Kohlenstoffatomen, das unsubstituiert oder einfach bis dreifach, gleichartig oder verschieden substituiert ist durch Fluor, Chlor, Methyl, Ethyl, Methoxy oder Trifluormethyl;
R¹ steht für Methyl, Ethyl, Propyl oder Butyl, das mit 1 bis 9 Fluor- und /oder Chloratomen substituiert ist; oder
R² steht für Wasserstoff, Methyl oder Ethyl; oder
R¹ und R² stehen gemeinsam mit dem Stickstoffatom, an das sie gebunden sind, für einen gesättigten oder ungesättigten heterocyclischen Ring mit 3 bis 6 Ringgliedern, wobei der Heterocyclus gegebenenfalls ein weiteres Stickstoff-, Sauerstoff- oder Schwefelatom als Ringglied enthält und wobei der Heterocyclus unsubstituiert oder bis zu zweifach substituiert sein kann durch Fluor, Chlor, Methyl, Ethyl, Trifluormethyl, Methoxy, Ethoxy, Hydroxymethyl, Acetyloxymethyl oder Isobutyryloxymethyl;
R⁴ steht für Wasserstoff,;
M¹ steht für Na⁺, K⁺, ½ Ca²⁺, ½ Mg²⁺, NH₄⁺, NH₃CH₃⁺, NH₂(CH₃)₂⁺, NH(CH₃)₃⁺, NH(C₂H₅)₃⁺, NH₂(C₂H₅)₂⁺, NH₃C₂H₅⁺, NH₃i-C₃H₇⁺, NH₂(i-C₃H₇)₂⁺, NH₃-CH₂-C₆H₅⁺ oder N(CH₃)₃-H₂-C₆H₅⁺;
R⁵ steht für S(O)R⁶, S(O)₂R⁶,
R⁷ steht Wasserstoff, für jeweils gegebenenfalls einfach bis dreifach durch Methoxy, Ethoxy, Methylthio, Ethylthio, Cyano, CO-O-Methyl oder CO-O-Ethyl substituiertes Methyl, Ethyl, n-Propyl, iso-Propyl oder Cyclopropyl, für Halogenmethyl, -propyl, -n-propyl, -i-propyl mit 1 bis 7 Fluor- und/oder Chloratomen,
R⁸ steht für Wasserstoff, Methyl, Ethyl, Propyl, iso-Propyl, Allyl, Propargyl, C₃-C₄-Alkenyl, Methoxy oder Ethoxy; oder
R⁷ und R⁸ stehen gemeinsam mit dem Stickstoffatom, an das sie gebunden sind, für einen gesättigten 5- oder 6-Ring, der gegebenenfalls durch Sauerstoff oder Schwefel unterbrochen sein kann; und
R⁹ und R¹⁰ stehen unabhängig voneinander für jeweils gegebenenfalls einfach bis dreifach durch Fluor und/oder Chlor substituiertes Methyl, Ethyl, Methoxy, Ethoxy, Propoxy, i-Propoxy, Butoxy, Alkyloxy, Methylthio, Ethylthio, Propylthio, Butylthio, i-Propylthio, sek.-Butylthio, Allylthio.

6. Verfahren zur Herstellung von Heterocyclyl-Pyrimidinen der allgemeinen Formel (I) gemäß Anspruch 1 **dadurch gekennzeichnet, dass** man
in Verfahren (1)
(a) Verbindungen der Formel (IIa) 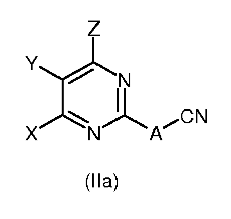 wobei X, Y, Z, A wie in Anspruch 1 definiert sind, in Gegenwart von Säure oder Base hydrolysiert und
(b) die so erhaltenen Verbindungen der Formel (III) 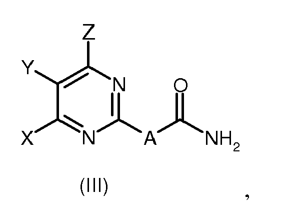 wobei X, Y, Z, A wie in Anspruch 1 definiert sind,
mit Verbindungen der Formel (IVa)
LG-R⁵ (IVa),
wobei R⁵ wie in Anspruch 1 definiert ist und LG für eine nucleophil abspaltbare Abgangsgruppe steht, gegebenenfalls in Gegenwart einer Base umsetzt und
(c) die so erhaltenen Verbindungen der Formel (Ia) 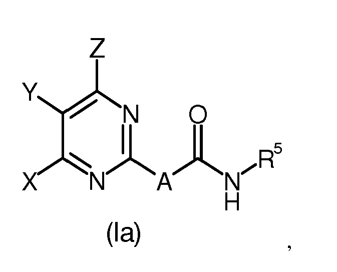 wobei X, Y, Z, A, und R⁵ wie in Anspruch 1 definiert sind, gegebenenfalls mit Verbindungen der Formel (IVb)
LG-R⁴ (IVb),
wobei R⁴ wie in Anspruch 1 definiert, aber nicht Wasserstoff ist und LG für eine nucleophil abspaltbare Abgangsgruppe steht, gegebenenfalls in Gegenwart einer Base umsetzt, wobei die Schritte (b) und (c) in ihrer Reihenfolge vertauscht werden können;
oder dass man in Verfahren (2)
(d) Verbindungen der Formel (IIb) 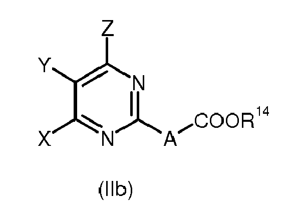 wobei X, Y, Z, A wie in Anspruch 1 definiert sind und R¹⁴ für Wasserstoff oder Alkyl steht,
im Falle, dass R¹⁴ Alkyl ist, in Gegenwart von Säure oder Base verseift, und
(e) die so erhaltenen Verbindungen der Formel (V) 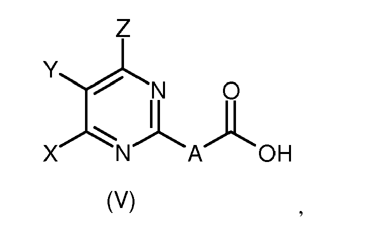
wobei X, Y, Z, A wie in Anspruch 1 definiert sind, mit Verbindungen der Formel (VI) 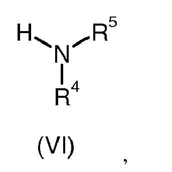 wobei R⁴ und R⁵ wie in Anspruch 1 definiert sind, umsetzt.

7. Verwendung der Verbindungen der Formel (V) 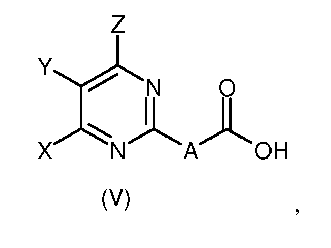 wobei X, Y, Z, A wie in Anspruch 1 definiert sind, zum Bekämpfen tierischer Schädlinge.

8. Verwendung gemäß irgendeinem der Ansprüche 1 bis 5 zum Bekämpfen tierischen Schädlinge und pflanzenpathogener Schadpilze.

9. Verwendung gemäß irgendeinem der Ansprüche 1 bis 5 sowie 7 und 8 zur Behandlung von Pflanzen oder zur Behandlung von Saatgut von Pflanzen.

10. Verwendung gemäß Anspruch 9, wobei die Pflanzen ausgewählt sind aus der Gruppe der transgenen Pflanzen.

11. Mittel zur Bekämpfung von tierischen Schädlingen und/oder pflanzenpathogenen Schadpilzen in und/oder auf Pflanzen oder in und/oder auf Saatgut von Pflanzen, wobei das genannte Mittel wenigstens eine wie in irgendeinem der Ansprüche 1 bis 5 sowie 7 definierte Verbindung und/oder wenigstens eines von deren agrochemisch wirksamen Salzen und agrochemisch übliche Hilfs- und/oder Zusatzstoffe umfaßt.

12. Mittel zur Bekämpfung von tierischen Schädlingen in und/oder auf Pflanzen oder in und/oder auf Saatgut von Pflanzen, wobei das genannte Mittel wenigstens eine wie in irgendeinem der Ansprüche 1 bis 5 sowie 7 definierte Verbindung und/oder wenigstens eines von deren agrochemisch wirksamen Salzen und mindestens ein Salz der Formel (XV) 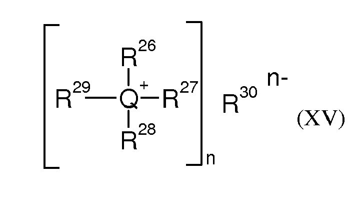 in welcher
Q für Stickstoff oder Phosphor steht,
R²⁶, R²⁷, R²⁸ und R²⁹ unabhängig voneinander für Wasserstoff oder jeweils gegebenenfalls substituiertes C₁-C₈-Alkyl oder einfach oder mehrfach ungesättigtes, gegebenenfalls substituiertes C₁-C₈-Alkylen stehen, wobei die Substituenten aus Halogen, Nitro und Cyano ausgewählt sein können,
n für 1, 2, 3 oder 4 steht,
R³⁰ für ein anorganisches oder organisches Anion steht,
umfaßt.

13. Verfahren zur Bekämpfung von tierischen Schädlingen in und/oder auf Pflanzen oder in und/oder auf Saatgut von Pflanzen umfassend das direkte oder indirekte Inkontaktbringen der genannten Schädlinge mit wenigstens einer wie in den Ansprüchen 1 bis 5 sowie 7 definierten Verbindung und/oder wenigstens einem von deren agrochemisch wirksamen Salzen oder einem wie in Ansprüchen 11 bis 12 definierten Mittel.

14. Saatgut, welches mit wenigstens einer wie in irgendeinem der Ansprüche 1 bis 5 sowie 7 definierten Verbindung und/oder wenigstens einem von deren agrochemisch wirksamen Salzen oder einem wie in den Ansprüchen 11 bis 12 definierten Mittel behandelt wurde.
